# EUROPEAN PATENT APPLICATION

(11) **EP 1 559 780 A1**
(43) Date of publication of application: **03.08.2005**
(21) Application number: 03748651.1
(22) Date of filing: 01.10.2003
(51) Int. Cl.: C12N 15/00, C12N 5/00, C07K 14/74, C07K 19/00, C12P 21/02, A61K 48/00, A61K 38/00, A61K 45/00, A61P 31/00, A61P 35/00, A61P 37/04, A61P 43/00

(54) **METHOD OF STRENGTHENING FOREIGN EPITOPE PRESENTATION BY MHC CLASS I BY INHIBITING TAP ACTIVITY**

(30) Priority: 01.10.2002 JP 2002288394
(71) Applicant: Dnavec Research Inc., Tsukuba-shi, Ibaraki 305-0856 (JP)
(72) Inventor: IWAMOTO, Aikichi, Bunkyo-ku, Tokyo 113-0022 (JP); TACHIKAWA, Ai, Setagaya-ku, Tokyo 154-0016 (JP)
(74) Representative: VOSSIUS & PARTNER
(86) International application number: PCT/JP2003/012595
(87) International publication number: WO 2004/031380

(57) **Abstract**

The present invention relates to methods for enhancing exogenous epitope display on an MHC class I molecule through inhibition of TAP activity. Mammalian cell-infecting virus vectors encoding both a TAP inhibitory factor and an epitope-linked-β2m were constructed and introduced into mammalian cells. The present inventors succeeded in displaying on the cell surface in high frequency the MHC class I/peptide complex containing an epitope-linked-β2m expressed from the vector by reducing endogenous MHC class I/peptide complexes by the action of the TAP inhibitory factor. The present invention finds utility in vaccine therapies for infectious diseases, cancers, and the like.

## Description

### Technical Field

This invention relates to methods for enhancing exogenous epitope display on an MHC class I molecule through inhibition of TAP activity.

### Background Art

The MHC (major histocompatibility complex) class I molecule is a heterodimer consisting of a heavy chain (HC) and β2-microglobulin (β2m), that is present on the cell membrane surface of almost every cell. The MHC class I molecule comprises a groove formed by the heavy chain that can stow a peptide of around 10 amino acids. The resulting MHC class I/peptide complex is recognized through T cell receptors (TCR) on cytotoxic T lymphocytes (CTL) that play a role in cellular immunity. Thus, a peptide displayed on an MHC class I molecule is called an "epitope" (antigenic determinant).

Peptide fragments derived from autoantigens produced in the cytoplasm, virus antigens, and tumor antigens are transported into the rough endoplasmic reticulum via a transporter called TAP (transporter associated with antigen processing) that exists on the surface of the rough endoplasmic reticulum. There they form stable MHC class I/peptide complexes with an MHC class I heavy chain and β2m. The β2-microglobulin molecule plays a role in retaining the stability of the 3-dimensional structure of the MHC class I molecule and in the transportation of the heavy chain to the cell surface (Tasuku Honjo and Takeshi Watanabe (ed.), "Immune System: Recognition, differentiation, and proliferation", The Molecular Biology Society of Japan (ed.), Maruzen Co., Ltd., Tokyo: p117-118). An empty MHC class I molecule unbound to a peptide is known to be very unstable under a culture condition of 37°C.

An epitope, *i*.*e*., a peptide displayed on an MHC class I molecule, is a very important molecule in inducing specific cellular immunity against a certain antigen. They are integral in the development of peptide vaccines as immunotherapeutic agents against infections with viruses, such as HIV, or against cancers. However, peptides of major epitopes derived from many virus antigens and tumor antigens have weak binding activity to MHC class I molecules. Therefore, generally, when these peptides are used alone as vaccines, no effect can be expected due to their low stability. To date, an artificial epitope having amino acid substitutions to enhance the binding ability to MHC class I molecule without losing its antigenicity has been effectively used in a clinical study with melanoma patients. However, identification and development of such epitopes with improved stability and antigenicity are troublesome and difficult and do not always result in an epitope of interest.

On the other hand, a system using the adjuvant effect of β2m is under development based on the finding that the stability of a peptide on an MHC class I molecule is enhanced by β2m.

Recently, the expression of MHC class I/peptide complexes wherein epitope peptides are fused with MHC class I molecules and MHC class II molecules or β2m has been attempted (Uger, R. A. and Barber, B. H., J. Immunol.160: 1598-1605 (1998); White, J. *et al*., J. Immunol. 162: 2671-2676 (1999); Kang, X. *et al*., Cancer Res. 57: 202-205 (1997); Uger, R. A. *et al*., J. Immunol. 162: 6024-6028 (1999); USP No. 5,869,270). These complexes are reported to exhibit enhanced stability and recognition by CTL as compared to the peptides alone, in particular, those constructed with epitopes having weak binding activity to MHC class I molecules, by the fusion of the epitope peptide sequence via a linker to the N-terminus of β2m (epitope-linked-β2m; e/β2m).

At present, the MHC class I/peptide complexes that comprise human MHC class I molecules are prepared by the independent expression and purification of heavy chains (HC) and β2m proteins in *E.coli*, followed by *in vitro* folding of these molecules with a synthetic peptide. In contrast, in relation with mouse MHC class I molecules, construction of an insect cell-derived MHC class I/peptide complex using a baculovirus vector has been reported (White, J. *et al*., J. Immunol. 162: 2671-2676 (1999)). In this system, the MHC class I/peptide complex is constructed in cells co-infected with baculoviruses expressing a heavy chain of MHC class I and an epitope-linked-β2m, respectively. Since the baculovirus vector system expresses exogenous genes located downstream of a promoter effective in insect cells (for example, Sf9 cells), it is difficult to apply this system to mammalian cells (Published Japanese Translation of International Publication No. Hei 6-502990). In addition, while Punnonen *et al*. have disclosed a genetic vaccine vector for use in DNA shuffling (Punnonen, J. *et al*., Genetic Vaccine Vector Engineering, USP Application Serial No. 20010006950), an epitope-fused MHC class I heavy chain or β2m has not yet been described.

### Disclosure of the Invention

An objective of the present invention is to provide methods for enhancing an exogenous epitope display on an MHC class I molecule through inhibition of TAP activity.

The present inventors constructed a gene encoding epitope-linked-β2m utilizing recombinant DNA technology, to express the epitope-linked-β2m molecule in mammalian cells using a mammalian cell-infecting virus vector, and to develop a system for producing MHC class I/peptide complexes in these cells. However, since there are many MHC class I molecules on the surface of mammalian cells and respective MHC class I molecules display a variety of peptides derived from proteins present intracellularly, available levels of MHC class I molecules necessary to form a complex with epitope-linked-β2m will decrease depending on the peptide levels, when the epitope-linked-β2m is expressed in mammalian cells.

The present inventors thought that to enhance frequency of MHC class I molecules on which an epitope of interest was displayed was very important for the induction of immunity. For example, for the quantification of frequency of antigen-specific CTL using an MHC class I/peptide complex, a method utilizing an "MHC class I/peptide tetrameric complex (tetramer)" has been developed (Altman J. D. *et al*., Science 274: 94-96 (1996)). This tetramer is prepared by tetramerization of a monomeric MHC class I/peptide complex using the biotin-avidin binding system followed by the addition of a fluorescence label and is used for FACS analysis. Since this method allows for direct analysis of peripheral blood mononuclear cell samples isolated from individuals, it is not only simple and reflects directly the frequency in each individual, but it also has high sensitivity and high specificity (Wilson, J.D.K. *et al*., J. Exp. Med. 188: 785-790 (1998); Kuroda, M.J. *et al*., J. Exp. Med. 187: 1373-1381 (1998)). The present inventors paid attention to the details that, in the analysis of above MHC class I/peptide complex, while the initially attempted FACS analysis failed due to a weak binding of the monomeric MHC class I/peptide complex to TCR at a ratio of 1:1, tetramerization of the monomeric MHC class I/peptide complex, that is, presence of multiple binding of MHC class I/peptide complex to TCR simultaneously, made it possible for the first time to analyze the complex.

Also, in the above-described epitope-linked-β2m expression system in mammalian cells, it is likely that probability of the presence of the MHC class I/peptide complex displaying an epitope of interest in the neighborhood will decline, when a variety of peptides derived from intracellular proteins are being displayed.

Taking the above two points into consideration, the present inventors speculated that if one kind of epitope could be displayed efficiently on MHC class I/peptide complex located on a cell membrane surface, it would also be recognized by TCR efficiently, that is, CTL could be activated efficiently. Until now, for example, it has been reported that CTL can be activated efficiently by adding a synthetic single peptide at a high concentration to a cell surface that was previously treated with an acid followed by washing out to remove the peptide displayed on the MHC class I/peptide complex (Langlade-Demoyen, P. *et al*., Int. Immunol. 6: 1759-1766 (1994)). However, while such manipulation can be done *in vitro*, practically, it is impossible in individuals. Moreover, it is difficult to apply such manipulation to immunotherapy, particularly when one takes the low stability of the peptide as described above into consideration.

Accordingly, the present inventors intended to present the epitope of interest alone fused with β2m as an antigen, by inhibiting the cell-derived antigen presentation using a molecule that inhibits the TAP-mediated peptide transportation. First of all, recombinant Sendai virus (SeV) vectors that express ICP47 (Hill, A. *et al*., Nature 375: 411-415 (1995); Fruh, K. *et al*., Nature 375: 415-418 (1995)) derived from type I herpes simplex virus (HSV-1) and US6 (Ahn, K. *et al*., Immunity 6: 613-621 (1997); Hengel, H. *et al*., Immunity 6: 623-632 (1997)) from human cytomegalovirus (CMV) which are known as TAP-inhibiting factors, were constructed. These vectors were introduced into a human CD4-positive T lymphocyte cell line H9 and MT-2 cells, and then the MHC class I/peptide complexes on the cell surface were analyzed by flow cytometry. As a result, while a significant change in the MHC class I/peptide complex levels was not detected in cells infected with wild type SeV as control cells, the MHC class I/peptide complex levels decreased with time in cells infected with the SeV vector expressing the TAP-inhibitory molecule US6, indicating that the expression of new (*de novo*) MHC class I/peptide complexes was suppressed.

Next, after the infection of the above cells with the SeV vector expressing either ICP47 or US6, epitopes expressed on the cell surface were dissociated by a further acid treatment of the cells. This treatment was capable of efficiently reducing the levels of the MHC class I/peptide complexes originally existing on the cell surface. Furthermore, the present inventors carried out an experiment to express in high levels the MHC class I/peptide complex having a single epitope on the cell surface by simultaneously expressing in the cells TAP-inhibitory molecule and an epitope-linked-β2m. For this purpose, the present inventors constructed a vector that co-expresses both a TAP-inhibitory molecule (US6) and an epitope-linked-β2m. The epitope expressed by the vector can exist irrelevant to TAP in the rough endoplasmic reticulum, since it is fused with β2m. Using this vector, by reducing MHC class I/peptide complexes displaying endogenous protein-derived peptides by the effect of the TAP-inhibitory factor, the present inventors succeeded in expressing at high frequency the MHC class I/peptide complex containing an exogenous epitope of interest bound to β2m. Thus, the present invention provides for the first time a novel concept that uses the TAP-inhibitory molecule for "an expression system for an MHC class I/peptide complex having a single peptide in high frequency on the cell surface", which is expected to be used as a vaccine for immunotherapy.

The present invention makes it possible to express an MHC class I/peptide complex containing an epitope-linked-β2m of interest in high purity in mammalian cells. For example, by introducing into cells a mammalian cell-infectious virus vector that expresses β2m or an MHC class I heavy chain bound to an epitope and expressing it in the presence of a TAP inhibitor as shown in Examples, it is possible to form preferentially on the cell surface an MHC class I/peptide complex containing the epitope expressed by the introduced gene.

As shown in Examples, cells co-expressing an epitope-linked-β2m (e.g., β2m bound with an epitope derived from the HIV-1 Nef protein) and the MHC class I heavy chain (HLA-A*2402) are recognized by antigen-specific CTL equivalently or more efficiently as compared to cells pulsed with a synthetic epitope peptide derived from the HIV-1 Nef protein. Also, the epitope-linked-β2m recovered from cells into which the SeV vector expressing the epitope-linked-β2m has been introduced induces an antigen specific CTL reaction against target cells by adding the epitope-linked-β2m to a culture solution of the target cells. These findings indicate that a vector encoding epitope-linked-β2m is useful for gene therapy *in vivo* or *ex vivo* to induce antigen-specific immune responses. Use of TAP inhibitors in this system makes it possible to display the epitope of interest in higher purity. It may be possible to realize an extremely effective immunotherapy by producing the MHC class I/peptide complex of interest in individuals in the presence of the TAP-inhibitory protein.

Thus, the present inventors established methods for the efficient display of an exogenous epitope by inhibiting the display of endogenous epitopes through the inhibition of TAP. *In vivo* or *ex vivo* introduction of a vector expressing an MHC class I heavy chain or β2m fused with an epitope in the presence of the TAP inhibitor makes it possible to induce antigen-specific cellular immunity. The methods of the present invention, for example, may be useful for the induction of protective immunity against infectious diseases caused by infection with viruses or bacteria, and for immunotherapy against cancers.

Namely, the present invention relates to methods for enhancing exogenous epitope display on MHC class I molecules through inhibition of TAP activity, more specifically to:
(1) a method for enhancing exogenous epitope display on an MHC class I molecule, wherein said method comprises the steps of:
   (a) inhibiting TAP activity in a cell; and
   (b) expressing an epitope-fused MHC class I heavy chain or an epitope-fused β2m in said cell;
(2) the method of (1), wherein said method further comprises the step of acid treatment;
(3) the method of (1) or (2), wherein said step of inhibiting TAP activity in a cell comprises the step of contacting said cell with a protein having TAP inhibitory activity, or the step of introducing a vector encoding said protein into said cell;
(4) the method of (3), wherein said protein having said TAP inhibitory activity is US6 or ICP47;
(5) the method of (3), wherein said vector is a mammalian cell-infecting virus vector;
(6) the method of (5), wherein said vector is a Sendai virus vector;
(7) a mammalian cell, wherein (i) expression of a TAP gene in said cell has been inhibited, said cell comprises a TAP inhibitor, or said cell bears a gene encoding said TAP inhibitor in an expressible way, and wherein (ii) said cell bears a gene encoding an epitope-fused MHC class I heavy chain or an epitope-fused-β2m in an expressible way;
(8) a kit for displaying an epitope comprising: (i) a TAP inhibitor or a vector capable of expressing said inhibitor; and (ii) an epitope-fused MHC class I heavy chain or an epitope-fused β2m, or a vector capable of expressing said epitope-fused MHC class I heavy chain or said epitope-fused β2m; and
(9) a vector capable of expressing both (i) and (ii):
   (i) a TAP inhibitor; and
   (ii) an epitope-fused MHC class I heavy chain or an epitope-fused β2m.

The present invention provides methods for enhancing exogenous epitope display on an MHC class I molecule through inhibition of TAP activity. Specifically, the methods of the present invention comprise the steps of: (a) inhibiting a cellular TAP activity, and (b) expressing an MHC class I heavy chain fused with an epitope or β2m fused with the epitope in the cells. There is no restriction on the order of the steps (a) and (b), and the above-described methods include the practical order of performing step (a) then step (b), step (b) then (a), and performing steps (a) and (b) simultaneously. TAP is a molecule that belongs to the ATP-binding cassette family, which includes two types, TAP-1 and TAP-2.

The human TAP-1 gene is described, for example, in Accession NM_000593 (SEQ ID NO: 47) (protein ID NP_000584 (SEQ ID NO: 48)) (Garbi, N. *et al*., Eur. J. Immunol. 33: 264-273 (2003); Heintke, S. *et al*., FEBS Lett. 533: 42-46 (2003); Ozbas-Gerceker, F. *et al*., Turk J Pediatr 44: 91-97 (2002); Seliger, B. *et al*., Int. J. Oncol. 20: 349-353 (2002); Saveanu, L. and Van Endert, P.M., Adv. Exp. Med. Biol. 495: 79-82 (2001); Chen, H.L. *et al*., Nat. Genet. 13: 210-213 (1996); Beck, S. *et al*., J. Mol. Biol. 255: 1-13 (1996); Jackson, D.G and Capra, J.D., Proc. Natl. Acad. Sci. U.S.A. 90: 11079-11083 (1993); Glynne, R. *et al*., Eur. J. Immunol. 23: 860-866 (1993); Beck, S. *et al*., J. Mol. Biol. 228: 433-441 (1992); Bodmer, J.G *et al*., Tissue Antigens 39: 161-173 (1992); Bahram, S. *et al*., Proc. Natl. Acad. Sci. U.S.A. 88: 10094-10098 (1991); Spies, T. *et al*., Nature 348: 744-747 (1990); Trowsdale, J. *et al*., Nature 348 (6303): 741-744 (1990)). TAP-1 is also called APT1, PSF1, ABC17, ABCB2, RING4, or D6S114E. In addition, the rat TAP-1 gene is described, for example, in Accession NM_032055 (protein ID NP_114444) (Rudolph, M.G. *et al*., J. Mol. Biol. 324: 975-990 (2002); Daumke, O. and Knittler, M.R., Eur. J. Biochem. 268: 4776-4786 (2001); Deverson, E.V. *et al*., Nature 348: 738-741 (1990)). The mouse TAP-1 gene is described in Accession NM_013683 (protein ID NP_038711) (Garbi, N. *et al*., Eur. J. Immunol. 33: 264-273 (2003); Ruedl, C. *et al*., Eur. J. Immunol. 32: 818-825 (2002); Neveu, R. *et al*., Mol. Immunol. 38: 661-667 (2002); Marusina, K. *et al*., J. Immunol. 158: 5251-5256 (1997); Van Kaer, L. *et al*., Cell 71: 1205-1214 (1992); Hanson, I.M. and Trowsdale, J., Immunogenetics 34: 5-11 (1991); Cho, S. *et al*., Proc. Natl. Acad. Sci. U.S.A. 88: 5197-5201 (1991); Monaco, J.J. *et al*., Science 250 (4988): 1723-1726 (1990)).

The human TAP-2 gene is described, for example, in Accession NM_000544 (SEQ ID NO: 49) (protein ID NP_000535 (SEQ ID NO: 50) (Garbi, N. *et al*., Eur. J. Immunol. 33: 264-273 (2003); Heintke, S. *et al*., FEBS Lett. 533: 42-46 (2003); Ozbas-Gerceker, F. *et al*., Turk J Pediatr 44: 91-97 (2002); Penfornis, A. *et al*., Hum. Immunol. 63: 61-70 (2002); Saveanu, L. and Van Endert, P.M., Adv. Exp. Med. Biol. 495: 79-82 (2001); Yan, G. *et al*., J. Immunol. 162: 852-859 (1999); Cesari, M.M. *et al*., Immunogenetics 45: 280-281 (1997); Pattanakitsakul, S. *et al*., Tissue Antigens 47: 353-355 (1996); Beck, S. *et al*., J. Mol. Biol. 255: 1-13 (1996); Cano, P. and Baxter-Lowe, L.A., Tissue Antigens 45: 139-142 (1995); de la Salle, H. *et al*., Science 265: 237-241 (1994); Glynne, R. *et al*., Eur. J. Immunol. 23: 860-866 (1993); Powis, S.H. *et al*., Immunogenetics 37: 373-380 (1993); Beck, S. *et al*., J. Mol. Biol. 228: 433-441 (1992); Bodmer, J.G. *et al*., Tissue Antigens 39: 161-173 (1992); Powis, S.H. *et al*., Proc. Natl. Acad. Sci. U.S.A. 89: 1463-1467 (1992); Kelly, A. *et al*., Nature 355: 641-644 (1992); Bahram, S. *et al*., Proc. Natl. Acad. Sci. U.S.A. 88: 10094-10098 (1991); Trowsdale, J. *et al*., Nature 348 (6303): 741-744 (1990)). TAP-2 is also called APT2, PSF2, ABC18, ABCB3, RING11, or D6S217E. In addition, the mouse TAP-1 gene is described, for example, in Accession NM_011530 (protein ID NP_035660, P36371) (Garbi, N. *et al*., Eur. J. Immunol. 33: 264-273 (2003); Marusina, K. *et al*., J. Immunol. 158: 5251-5256 (1997); Yang, Y. *et al*., J. Biol. Chem. 267: 11669-11672 (1992); Attaya, M. *et al*., Nature 355: 647-649 (1992); Monaco, J.J. *et al*., Science 250: 1723-1726 (1990)). The rat TAP-1 gene is described, for example, in Accession NM_032056 (protein ID NP_114445) (Powis, S.J. *et al*., Nature 354: 528-531 (1991)). The bovine TAP-1 gene is described, for example, in Accession NM_174222 (protein ID NP_776647) (Ambagala, A.P. *et al*., Immunogenetics 54: 30-38 (2002)).

The TAP-1 and TAP-2 genes exhibit polymorphisms. In the present invention, reference to the TAP-1 and TAP-2 genes encompasses all types of respective polymorphisms. Specifically, in the present invention, the TAP-1 and TAP-2 genes respectively include nucleic acids encoding proteins comprising amino acid sequences having 68% or more, preferably 70% or more, more preferably 75% or more, more preferably 80% or more, more preferably 85% or more, more preferably 90% or more, and more preferably 95% or more identity to above-mentioned mammalian TAP-1 (such as NP_000584, NP_114444, or NP_038711) and TAP-2 (such as NP_000535,NP_035660, P36371, NP_114445, or NP_776647) proteins. These proteins are involved in the processing of epitopes.

Amino acid sequence identity can be determined using, for example, the BLASTP program (Altschul, S. F. *et al*., J. Mol. Biol. 215: 403-410 (1990)). For example, a search may be carried out in the BLAST web page of NCBI (National Center for Biotechnology Information) using default values as parameters without use of any filters including Low complexity (Altschul, S.F. *et al*., Nature Genet. 3: 266-272 (1993); Madden, T.L. *et al*., Meth. Enzymol. 266: 131-141 (1996); Altschul, S.F. *et al*., Nucleic Acids Res. 25: 3389-3402 (1997); Zhang, J. & Madden, T.L., Genome Res. 7: 649-656 (1997)). For example, using the blast2sequences program (Tatiana A *et al*., FEMS Microbiol Lett. 174: 247-250 (1999)) for the comparison of two sequences, an alignment of the two sequences can be prepared to determine the sequence identity. For example, the identity value for the total amino acid sequences of the mammalian TAP-1 (such as NP_000584, NP_114444, or NP_038711) and TAP-2 (such as NP_000535, NP_035660, P36371, NP_114445, or NP_776647) proteins can be calculated by equating gaps with mismatches.

In addition, in the present invention, the TAP-1 and TAP-2 genes respectively include nucleic acids hybridizable to 30 nucleotides or more, more preferably 50 nucleotides or more, more preferably 100 nucleotides or more, more preferably 150 nucleotides or more, and more preferably the full-length of the protein-encoding sequences in the above-mentioned mammalian TAP-1 (such as NM_000593, NM_032055, or NM_013683) and TAP-2 (such as NM_000544, NM_011530, NM_032056, or NM_174222) genes under the stringent conditions. In the hybridization, such nucleic acids may be identified by preparing a probe either from a nucleic acid containing a coding sequence of a mammalian TAP-1 or TAP-2 gene or from a nucleic acid of a hybridization target, and then detecting the probe hybridized to the corresponding nucleic acid. The stringent hybridization conditions include, for example, hybridization in a solution containing 5x SSC (1x SSC contains 150 mM sodium chloride and 15 mM sodium citrate), 7% (w/v) SDS (sodium dodecyl sulfate), 100 µg/ml denatured salmon sperm DNA, 5x Denhardt's solution (1x Denhardt's solution contains 0.2% polyvinylpyrrolidone, 0.2% bovine serum albumin, and 0.2% Ficoll), at 48°C, preferably at 50°C, more preferably at 55°C, and more preferably at 60°C, followed by washing at the same temperature as that in hybridization in a solution containing 2x SSC and 0.1% (w/v) SDS, preferably 1x SSC and 0.1% (w/v) SDS, more preferably 0.5x SSC and 0.1% (w/v) SDS, and more preferably 0.1x SSC and 0.1% (w/v) SDS, for 2 hours with shaking.

Both TAP-1 and TAP-2 have transmembrane domains and function as transporters by forming heterodimers on the endoplasmic reticulum membrane. No antigen presentation on MHC class I molecule can be done without either one of the two. Peptides processed in the cytoplasm are transported into endoplasmic reticulum via the TAP transporter in an ATP-dependent manner, and there bind to the MHC class I molecule. In the present invention, TAP includes TAP-1 and TAP-2. Also, inhibition of TAP activity includes inhibition of TAP-1 activity and/or the TAP-2 activity. The inhibition of TAP activity may be direct or indirect. For example, inhibition of TAP activity includes direct inhibition of TAP activity, by applying an inhibitor acting directly on TAP, and indirect inhibition of TAP activity, by suppressing the expression of TAP (transcription or translation). Inhibition of TAP expression can be carried out by intracellularly expressing an antisense nucleic acid for the TAP gene, or by expressing a ribozyme that intracellularly cleaves transcripts of the TAP gene. Inhibition of the TAP gene expression can also be done by RNA interference (RNAi) using a nucleic acid having a double stranded RNA portion containing a complementary sequence of a portion of the TAP gene transcripts. All of these may be used as TAP inhibitors in the present invention.

Generally, RNAi denotes a phenomenon that expression of the target gene is inhibited through inducing the mRNA destruction of the target gene, by introducing into cells or expressing in cells RNA comprising a sense RNA having a sequence homologous to a portion of the mRNA sequence of the target gene and an antisense RNA having a sequence complementary to the sense RNA (Genes Dev. 15: 188-200 (2001); Elbashir, SM *et al*., Nature 411: 494-498 (2001)). When a double stranded RNA having an RNAi effect is introduced into cells, an enzyme called DICER (a member of RNase III nuclease family) will contact with the double stranded RNA and the double stranded RNA will be degraded into a small fragment called a small interfering RNA (siRNA). In this invention, in addition to such RNA generated by the intracellular processing, RNA molecules artificially synthesized or expressed to inhibit the expression of the target gene through RNAi are also generically called siRNA. In the present invention, RNA having the RNAi effect include these siRNAs. Expression of the target gene can be suppressed *in vivo* via siRNA (Anton P. *et al*., Nature Vol. 418: 38-39 (2002); David L. *et al*., Nature genetics Vol. 32: 107-108 (2002)).

Generally, siRNA are RNA comprising a nucleotide sequence having 15 contiguous nucleotides or more (more preferably 16 nucleotides or more, 17 nucleotides or more, 18 nucleotides or more, or 19 nucleotides or more) within the transcribed sequence (mRNA sequence) of a certain target gene and its complementary sequence, which will form a double stranded RNA with each other by hybridization. Preferably, siRNA include a sequence of 19 to 30 contiguous nucleotides, more preferably 20 to 25 contiguous nucleotides, more preferably 21 to 23 contiguous nucleotides, or its complementary sequence in one strand, and opposite strand that forms a complementary pair with it. However, there is no limitation on RNA length because even RNA having a longer sequence can be expected to be endogenously degraded into siRNA having the RNAi effect. Also, it is possible to degrade beforehand a long chain double stranded RNA corresponding to entire or almost entire region of mRNA from the target gene with, for example, DICER and to use the degradation product as siRNA. This degradation product is expected to include an RNA molecule (siRNA) having the RNAi effect. There is no need to choose a region of mRNA expected to have the RNAi effect, according to this method. Namely, it is not always necessary to define exactly the sequence having the RNAi effect against the target gene.

It is known that a double stranded RNA having an overhang with a few nucleotides at the end generally has a high RNAi effect. It is desirable, but not essential, that siRNA used in the present invention have the overhang with a few nucleotides at the end (preferably at the 3'end). Though there is no limitation on length of the nucleotide sequence that forms the overhang, a 2 nucleotide overhang is preferred. In the present invention, for example, a double stranded RNA having an overhang with TT (two thymines), UU (two uracils), or other nucleotides (most preferred molecule has a 19 base paired double stranded RNA portion and a 2 nucleotides' (TT) overhang portion) may suitably be used. In the present invention, siRNA include such molecules having DNA that forms the overhang.

Also, two strands that form base pairs within the siRNA may be connected via a spacer sequence. Namely, a double stranded RNA generated by a loop formation of the spacer and annealing with each other of the remaining two sequences up and down wards from the loop may also be suitably used. Though there is no limitation on the spacer length, it may be 3 to 23 nucleotides, for example.

Moreover, vectors capable of expressing the above siRNA may also be used in the present invention. Namely, the present invention relates to use of a vector capable of expressing RNA having the RNAi effect. For example, the above vector may be a nucleic acid in which one strand of the double stranded siRNA and the other strand are linked to different promoters respectively, so that each strand is expressed separately. Optionally, alternative splicing may be used to transcribe the two kinds of RNA from one promoter. Optionally, the vector may express a single stranded RNA in which a sense strand and an antisense strand are linked via a spacer (that forms a loop). This RNA expressed from the vector suppresses expression of a target gene by forming an RNA stem having the RNAi effect. The stem length is similar to that of above siRNA, and it may be 19 to 29 nucleotides, for example. Though there is no limitation on the spacer length, it may be 3 to 23 nucleotides, for example. The 5' and/or 3' ends of the stem may or may not have an overhang of a few nucleotides. These vectors may be easily prepared using genetic engineering techniques generally known to those skilled in the art (Brummelkamp, T.R. *et al*., Science 296: 550-553 (2002); Lee, N.S. *et al*., Nature Biotechnology 19: 500-505 (2001); Miyagishi, M., and Taira, K., Nature Biotechnology 19: 497-500 (2002); Paddison, P.J. *et al*., Proc. Natl. Acad. Sci. USA 99: 1443-1448 (2002); Paul, C.P. *et al*., Nature Biotechnology 19: 505-508 (2002); Sui, G. *et al*., Proc Natl Acad Sci USA 99(8): 5515-5520 (2002); Proc Natl Acad Sci USA 99: 14943-14945 (2002); Paddison, P.J. *et al*., Genes Dev. 16:948-958 (2002)). More specifically, it is possible to construct recombinant vectors by inserting DNA encoding the target RNA sequence into various kinds of known expression vectors appropriately. Preferred promoters include the RNA polymerase III promoter. Specifically, the U6 Pol III promoter, the H1 RNA promoter (H1 RNA is one component that constitutes RNase P), and the like, may be used, for example.

One preferred example of siRNA is described below; however, siRNA that can be used in the present invention are not limited thereto. First of all, a transcriptional region located at least 50 nucleotides, preferably 60 nucleotides, and more preferably 70 nucleotides downstream from the initiation codon of the target gene, is selected. Preferably, an AA sequence may be found there. Seventeen to 20 nucleotides following the AA sequence (for example, 19 nucleotides following AA) are then selected. There is no limitation on the nucleotide next to AA, but preferably G or C is selected. Herein, the GC content of the sequence to be selected is preferably 20 to 80%, more preferably 30 to 70%, and more preferably 35 to 65%. In addition, the selected sequence is preferably specific for a target gene among the genes expressed in tissues to which siRNA is administered. For example, preferably, it is confirmed that there is no gene having the same transcriptional sequence except for the target gene among the genes present in individuals to which siRNA is to be administered, by searching public gene sequence databases using the selected sequence as a query. Also, the sequence is preferably selected from the protein coding sequence (CDS) of the target gene. Suitable siRNA may include sequences having these selected sequences without initial AA (preferably, UU or TT may be added at the 3'end) and their complementary sequences (preferably, UU or TT may be added at the 3'end). In a similar way to that described above, instead of searching the sequences following the AA sequence, sequences following a CA sequence may be searched. Alternatively, other sequences than AA or CA may also be searched. It may also be possible to appropriately select an RNA having the most suitable RNAi effect from a number of siRNAs thus prepared.

RNA having the RNAi effect can appropriately be prepared by those skilled in the art, based on the nucleotide sequence of a target gene. The nucleotide sequence of the target TAP gene can easily be obtained from a public gene database as described above, by the screening of a mammalian cDNA library using a known TAP gene as a probe, or by RT-PCR. Based on the obtained nucleotide sequence of the gene, siRNA may be designed according to the above description.

It is not always necessary to know the entire nucleotide sequence of the target gene. One need only know of a contiguous RNA region to be selectable as siRNA (for example, 20 to 30 nucleotides). Therefore, it is possible to prepare a siRNA even from a gene fragment such as an EST (Expressed Sequence Tag) for which the entire nucleotide sequence is not known but for a portion of mRNA, based on the nucleotide sequence of the gene fragment. When a synthesized siRNA is used clinically, it may be modified.

On the other hand, when antisense nucleic acids are used to suppress gene expression, they may comprise antisense sequences against 13 contiguous nucleotides or more, preferably 14 nucleotides or more, and more preferably 15 nucleotides or more (18 nucleotides or more, 20 nucleotides or more, or 25 nucleotides or more) within the transcriptional sequence of the target TAP gene. For example, preferred antisense nucleic acids include antisense sequences against an exon-intron boundary within an early transcriptional sequence, an intron-exon boundary, a region containing a translational initiation codon, an untranslated region around the 5'end, and a nucleotide sequence of 13 contiguous nucleotides or more, preferably 14 nucleotides or more, and more preferably 15 nucleotides or more within the protein coding region (CDS) of a mature mRNA. In addition, when antisense nucleic acids are to be used clinically, they generally comprise synthetic oligomers. Antisense nucleic acids may be DNA, and they may be modified. For example, it is generally known to use an S oligo (a phosphorothioate type oligonucleotide) in which the O (oxygen) atom of a phosphodiester bond is substituted with an S (sulfur) atom so as to decrease in sensitivity to nuclease digestion yet maintain the activity as an antisense nucleic acid. Clinical studies of injection of this S oligo directly into an affected area as an antisense drug are in presently in progress. This S oligo is also suitable for use in the present invention. While the antisense nucleic acid sequence is preferably complementary to a target gene or its portion, it need not necessarily be completely complementary, so long as it is capable of effectively suppressing gene expression. The transcribed RNA is preferably be 90% or more, most preferably 95% or more complementary to the target gene transcript. Chain length of antisense nucleic acid is preferably 17 nucleotides or more, more preferably 20 nucleotides or more, more preferably 25 nucleotides or more, more preferably 30 nucleotides or more, more preferably 40 nucleotides or more, more preferably 50 nucleotides or more, and most preferably 100 nucleotides or more, for an effective suppression of a target gene expression. Antisense RNA can also be expressed intracellularly. Such can be done by constructing a vector connected with a nucleic acid encoding an RNA of interest downstream of a promoter effective in the target cells, and then introducing it into the cells.

As for vectors, virus vectors, including retrovirus vectors, adenovirus vectors, adeno-associated virus vectors, minus strand RNA virus vectors, and non-viral vectors including liposomes, may be used. Using these vector systems or carriers for gene transfer, gene therapy may be performed by administering them to patients using an *ex vivo* method, an *in vivo* method, or the like.

Alternatively, one may suppress the TAP gene expression using a ribozyme, or a vector encoding the ribozyme. The term "ribozyme" refers to an RNA molecule having a catalytic activity. Since there is a variety of ribozymes having different activities, it may also be possible to design a ribozyme capable of site-specifically cleaving RNA. While ribozymes include those having 400 nucleotides or more in length, such as group I intron type RNAs and the M1 RNA contained in RNase P, those having active domains with about 40 nucleotides in length, which are called hammerhead type (Rossi *et al*., Pharmac. Ther. 50: 245-254 (1991)) or hair pin type (Hampel *et al*., Nucl. Acids Res. 18: 299-304 (1990), and U.S. Pat. No. 5,254,678) ribozymes, are also known (Makoto Koizumi and Eiko Ohtsuka, Protein, Nucleic acid and Enzyme 35: 2191 (1990)).

For example, while the self-cleaving domain of the hammerhead type ribozymes cleaves the 3' side of C15 within the sequence G13U14C15, formation of a base pair between U14 and A9 is thought to be important for the activity, and it has been shown that the cleavage also occurred at the 3'side of A15 or U15 instead of C15 (Koizumi, M. *et al*., FEBS Lett. 228: 228 (1988)). An restriction enzyme-like RNA-cleaving ribozyme that recognizes UC, UU, or UA sequence within the target RNA may be constructed, when a ribozyme is designed in such a way that the substrate-binding site is complementary to an RNA sequence near the target site (Koizumi, M. *et al*., FEBS Lett. 239: 285 (1988); Makoto Koizumi and Eiko Ohtsuka, Protein, Nucleic acid and Enzyme 35: 2191 (1990); Koizumi, M. *et al*., Nucl. Acids Res. 17: 7059 (1989)).

Also, a hairpin type ribozyme may be useful for the purpose of the present invention. This type of ribozyme is found, for example, in the minus strand of satellite RNA from tobacco ring spot virus (Buzayan, JM., Nature 323: 349 (1986)). It has been shown that a target-specific RNA cleaving ribozyme can also be constructed from the hairpin type ribozyme (Kikuchi, Y. & Sasaki, N., Nucl Acids Res. 19: 6751 (1991); Kikuchi, Y., Kagaku to Seibutu 30: 112 (1992)). Thus, expression of a target gene can be suppressed by specifically cleaving the transcript of the target gene using ribozymes.

Alternatively, to inhibit TAP activity, a mutation may be introduced into the TAP gene, or the TAP gene may be deleted by gene targeting. In the present invention, "inhibiting TAP activity" may also include providing or preparing a cell defective in the expression of the TAP gene. In the present invention, TAP inhibitors include such compounds that inhibit TAP activity directly or indirectly. Namely, TAP inhibitors include these antisense nucleic acids, ribozymes, siRNA, and further more, factors that suppress expression of the TAP gene. There is no limitation on the TAP inhibitors so long as they have the TAP-inhibitory activity; they include proteins, peptide compounds, non-peptide compounds, nucleic acids, nucleic acid analogues, and low molecular compounds.

When a TAP inhibitor can be encoded by a nucleic acid, it may be expressed by introducing a vector that expresses the nucleic acid into cells. Examples of TAP-inhibitory proteins include ICP47 derived from herpes simplex virus (Hill, A. *et al*., Nature 375: 411-415 (1995); Fruh, K. *et al*., Nature 375: 415-418 (1995)) and US6 derived from cytomegalovirus (Ahn, K. *et al*., Immunity 6: 613-621 (1997); Hengel, H. *et al*., Immunity 6: 623-632 (1997)).

Specifically, ICP47 from Human herpes virus type I is referenced in Accession NC_001806 (complementary strand of 145311-145577) (SEQ ID NO: 51) (protein ID NP_044675, P03170 (SEQ ID NO: 52)) (Dolan, A. *et al*., J. Gen. Virol. 73 (Pt 4): 971-973 (1992); Perry, L.J. and McGeoch, D.J.; J. Gen. Virol. 69 (Pt 11): 2831-2846 (1988); McGeoch, D.J. *et al*., J. Gen. Virol. 69 (Pt 7): 1531-1574 (1988); McGeoch, D.J. *et al*., Nucleic Acids Res. 14 (4): 1727-1745 (1986); McGeoch, D.J. *et al*., J. Mol. Biol. 181 (1): 1-13 (1985)). In addition, ICP47 from Human herpes virus type 2 is referenced in Accession NC_001798 (complementary strand of 147775-148035) (protein ID NP_044543) (Barnett, B.C. *et al*., J. Gen. Virol. 73 (Pt 8): 2167-2171 (1992); McGeoch, D.J. *et al*., J. Gen. Virol. 72 (Pt 12): 3057-3075 (1991); Everett, R.D. and Fenwick, M.L., J. Gen. Virol. 71 (Pt 6): 1387-1390 (1990); McGeoch, D.J. *et al*., J. Gen. Virol. 68 (Pt 1): 19-38 (1987)).

For US6 from cytomegalovirus, for example, US6 from human CMV is referenced, in Accession AY072775 (SEQ ID NO: 53) (protein ID AAL67143 (SEQ ID NO: 54), NP_040091), and US6 from chimpanzee CMV is referenced in Accession NC_003521 (protein ID NP_612779) (Davison, A.J. *et al*., J. Gen. Virol. 84 (Pt 1): 17-28 (2003)).

The ICP47 molecule inhibits TAP-mediated peptide transport by binding to the peptide-binding site of the TAP molecule (Tomazin, R. *et al*., EMBO J. 15: 3256-3266 (1996)). On the other hand, the US6 molecule inhibits binding of ATP to TAP-1, which leads to inhibition in peptide transport (Hewitt, E. *et al*., EMBO J. 20: 387-396 (2001)). Both molecules may be used to inhibit TAP activity in the present invention. The above mentioned viruses include a plurality of subspecies and strains, and they may preferably be used in this invention, so long as the proteins encoded by the corresponding genes have the TAP-inhibitory activity. In addition, these proteins include not only wild-type proteins but also the modified proteins, so long as they maintain TAP-inhibitory activity. For example, a partial peptide having TAP inhibitory activity may be used. Furthermore, other peptides, such as tags, may be added. For example, a protein with a His tag may be used (see Examples).

Specifically, in the present invention, the ICP47 and US6 proteins include proteins comprising amino acid sequences having 68% or more, preferably 70% or more, more preferably 75% or more, more preferably 80% or more, more preferably 85% or more, more preferably 90% or more, and more preferably 95% or more identity to amino acid sequence of the above described ICP47 protein (such as NP_044675, P03170 (SEQ ID NO: 52), or NP_044543) and the US6 protein (such as AAL67143 (SEQ ID NO: 54), NP_040091, or NP_612779), respectively. Identity of amino acid sequence may be calculated according to the method mentioned in the present description. For example, the identity score to the entire amino acid sequence of the ICP47 protein (such as NP_044675, P03170 (SEQ ID NO: 52), or NP_044543) or the US6 protein (such as AAL67143 (SEQ ID NO: 54), NP_040091, or NP_612779) may be calculated.

Moreover, in the present invention, the ICP47 and US6 proteins include those encoded by nucleic acids hybridizable to 30 nucleotides or more, preferably 50 nucleotides or more, more preferably 100 nucleotides or more, more preferably 150 nucleotides or more, and more preferably the entire sequence within the protein coding sequences for the above described ICP47 protein (such as 145311-145577 of NC_001806 or 147775-148035 of NC_001798) and for the US6 protein (such as AY072775 or NC_003521) under the stringent conditions, respectively. The stringent hybridization conditions include those described above.

While any desired expression vector may be used for the expression of the TAP inhibitory protein, mammalian cell-infecting virus vectors are preferred. The vector may be constructed in a way capable of co-expressing an epitope-fused MHC class I heavy chain or an epitope-fused β2m, which will be described later, in addition to expressing the TAP inhibitor.

While inhibition of TAP activity may be transient, it is preferably continued for a time (for example, 3 hours or more, preferably 6, 18, 24, or 30 hours or more) sufficient to allow for the expression the epitope-fused MHC class I heavy chain or β2m. For example, in the step of expressing the epitope-fused MHC class I heavy chain or β2m in the cells, inhibition of TAP activity may be continued until an exogenous epitope is significantly displayed.

More preferably, an acid treatment step is included in addition to the step of inhibiting TAP activity in the method of the present invention to exhibit an enhanced display of the exogenous epitope. There is no restriction on the order of the acid treatment. It may be carried out before, after, or at the same time the step of inhibiting TAP activity or expressing the epitope-fused protein in the cells. For example, one specific method of the acid treatment in the case of co-expressing the TAP inhibitor and the epitope-fused protein using an expression vector comprises the steps of: (i) expressing the TAP inhibitory protein and the epitope-fused MHC class I heavy chain or β2m in cells; and (ii) treating the cells with an acid. While endogenous antigen processing will be intercepted by the step of inhibiting TAP activity, already displayed epitope peptides on the cell surface may be dissociated by acid treatment of the cells. Herein, acid treatment denotes exposure of the cells to an acidic environment such that an epitope peptide displayed on the cell surface is significantly dissociated. The acid treatment may be carried out, for example, by treating cells with a peptide-stripping buffer (0.13 M citric acid (pH3), 66 mM Na₂HPO₄, 150 mM NaCl, and 17 µg/ml Phenol Red), at 4°C for 1 min, followed by neutralization using sufficient amounts of culture medium (for example, RPMI1640). Those skilled in the art are able to perform the acid treatment via other protocols achieving similar effect.

Any desired epitope may be used to be bound to the MHC class I heavy chain or β2m. As used herein, the term "epitope" refers to a peptide recognized by immune cells. Such immune cells include T cells, B cells, NK cells, and NKT cells. In the present invention, a preferred epitope includes a peptide recognized by T cells. The epitopes include not only those displayed on antigen-presenting cells, but also any desired peptide, so long as it can be recognized by an immune cell. For example, a peptide having an artificially prepared amino acid sequence may also be used as the epitope. More preferably, the epitope is a peptide displayed on an antigen-presenting cell (APC) or a peptide containing a portion thereof. In the present invention, a "portion" of a protein or a peptide to be used as an epitope contains generally 8 or more, preferably 9 or more, more preferably 10 or more, 12 or more, or 15 or more contiguous amino acids. In addition, in the present invention, while epitope peptides displayed through an antigen processing within the cells are referred to as endogenously displayed epitopes within the cells, other epitopes are referred to as "exogenous epitopes". For example, even if an amino acid sequence of an epitope is identical to that of an endogenously displayed epitope, the epitope added exogenously or displayed without the endogenous antigen processing is included in exogenous epitopes in the present invention. Also, epitope peptides covalently bound to such proteins that constitute MHC, including the MHC class I heavy chain and β2m in a form of fusion protein, are included in exogenous epitopes in the present invention. The method of the present invention using TAP inhibitors enables the display of an exogenous epitope on the cells in high frequency by inhibiting the intracellular antigen processing.

The exogenous epitope may be bound to either an MHC class I heavy chain or β2m. There is no limitation on the epitope to be used in the present invention, and a variety of desired epitopes having therapeutic effects against, for example, tumors, infectious diseases, and other general diseases may be used. Chain length of the peptide as the epitope may be appropriately changed, but generally an 8 or more amino acid peptide, for example, approximately a 10 amino acid peptide is preferably used. When an epitope is bound to β2m, the epitope may be connected with any site of β2m, but preferably, for example, it is connected to the N-terminus of the secreted β2m molecule. For this purpose, the epitope is bound next to the secretory signal of β2m, and then linked to the β2m protein via a spacer sequence if necessary, to produce the desired fusion protein. Alternatively, the epitope may be linked directly to β2m without a spacer. The amino acid sequence of the spacer is not limited in any way; however, preferred peptides used as the spacer include those comprising an amino acid sequence, for example, Gly-Gly-Gly-Ser (SEQ ID NO: 1) or repeated sequences thereof, (Gly-Gly-Gly-Ser)ₙ. The number of repetition (n) is not limited, and it can be, for example, 1 to 5 (1, 2, 3, 4, or 5). When used, it is preferable to use a spacer having a length of up to 16 amino acids, for example, 4, 8, or 16 amino acids. Any desired mammalian β2m molecule may be used as β2m without limitations. When used for humans, human β2m is preferred. The human β2m gene may be isolated according to the method as described in the Examples below, for example. In addition, β2m may be a wild type protein or a mutant type protein with altered amino acid sequences.

When an epitope is bound to the MHC class I heavy chain, the epitope may be bound to any desired site of the MHC class I heavy chain, but preferably it is bound to a site where the epitope is placed at the N-terminus of the MHC class I molecule after the secretion. For this purpose, the epitope may be bound next to a secretory signal sequence of the MHC class I heavy chain, and then linked to the MHC class I heavy chain via a spacer if necessary, to produce the desired fusion protein. Alternatively, the epitope may be linked directly to the MHC class I heavy chain without a spacer.

The amino acid sequence of the spacer is not limited in any way, but preferred peptides used as the spacer include those comprising an amino acid sequence, for example, Gly-Gly-Gly-Gly-Ser (SEQ ID NO: 2) or repeated sequences thereof, (Gly-Gly-Gly-Gly-Ser)ₙ. The number of repetition (n) is not limited, and it can be, for example, 1 to 5. According to a report using the mouse MHC class I molecule H2-K^{d}, when spacer sequences were compared among a 10 amino acid spacer GGPGGGSGGG (SEQ ID NO: 3), a 20 amino acid spacer GGP(GGGGS)₂GGGSGGG (SEQ ID NO: 4), and a 30 amino acid spacer GGP(GGGGS)₄GGGSGGG (SEQ ID NO: 5), the 10 amino acid spacer sequence was recognized most effectively (Mottez, E. *et al*., J. Exp. Med. 181: 493-502 (1995)). Moreover, according to a report using the human MHC class I molecule HLA-A2, a melanoma antigen epitope was effectively displayed using a 10 amino acid spacer sequence (GGSGGGASGG) (SEQ ID NO: 6) (Kang, X. *et al*., Cancer Res. 57: 202-205 (1997)). Thus, binding of epitope via these spacer sequences may be preferred.

There is no particular limitation on the origin of the MHC class I heavy chains. Human MHC class I molecules (HLA) are preferably used for human applications. Human MHC class I molecules are called human leukocyte antigens (HLA) and are quite rich in variety. Any of these molecules can be used as the HLA in the present invention. For example, the present invention may be applied to any type of HLA whose target epitope has been identified in infectious diseases and cancers, and they may be used as custom-made or tailor-made reagents or medicines.

In particular, HLA-A24 (approximately 60 to 70%), HLA-A2 (approximately 40%), and HLA-A26 (approximately 20%) are high frequent HLA types in Japanese, followed by HLA-A11 (20%), HLA-A31 (up to 20%), and HLA-A33 (up to 20%). These HLA types are particularly preferred when the invention is applied to a Japanese subject.

For the purpose of developing reagents or medicines applicable to a certain extent of many unspecified individuals in, for example a Japanese subject, HLA types having 5% or more allele frequency among Japanese population (namely, 10% or more HLA type frequency among Japanese) are preferred. Such HLA types include:

| | |
|---|---|
| A locus | B locus |
| A*0201 | B*0702 |
| A*0206 | B*1501 |

| | |
|---|---|
| A*1101 | B*3501 |
| A*2601 | B*4001 |
| A*31012 | B*4002 |
| A*3303 | B*44031 |
| | B*4601 |
| | B*52011 |
| | B*5401 |

In the Examples, the present inventors used A24-restricted HLA class I heavy chain as the MHC class I molecule and an HIV-1 derived epitope displayed on the A24-restricted HLA class I molecule as the epitope. Approximately 60 to 70% of the Japanese population have the genotype HLA-A*2402. Therefore, the A24-restricted HLA class I heavy chain, particularly A*2402 (Litte, A.-M., Immunogenetics 35: 41-45 (1992)) is suitable when applied to the Japanese population. The MHC class I heavy chain may be a wild type protein or a mutant type protein with an altered amino acid sequence.

An epitope-fused MHC class I heavy chain or β2m and a TAP inhibitory protein may be prepared as recombinant proteins using publicly known recombinant gene technologies (J. Sambrook and D.W. Russell eds., "Molecular cloning: a laboratory manual", 3rd ed., Cold Spring Harbor Laboratory Press, Cold Spring Harbor, NY (2001)). Any desired expression system, including plasmid vectors and virus vectors, may be used, without limitations, as expression vectors for the expression of recombinant proteins. A variety of expression vector-host cell systems for recombinant protein production is known, and any desired host cells, for example, bacteria, yeast, animal cells, and plant cells, can be used. Recombinant proteins thus expressed may be purified in a substantially pure form using known methods for the purification of proteins. In one preferred embodiment of the present invention, an epitope-fused MHC class I heavy chain or β2m may be expressed in mammalian cells. This expression in mammalian cells may enhance efficiency of antigen presentation to immune cells compared with those in *E.coli*, since expression in mammalian cells allows normal modifications of the expressed protein with sugar chains or the like. Vectors expressible in mammalian cells may be used not only to produce a recombinant protein, but also to introduce it into cells and to display an exogenous epitope on the surface of a target cell. Expression of an epitope-fused MHC class I heavy chain or β2m, and a TAP inhibitory protein in mammalian cells may preferably be done by introducing a mammalian cell-infecting virus vector into the cells.

Both genes encoding an epitope-linked-β2m and a membrane bound form of the MHC class I heavy chain may be introduced into cells to express large amounts of MHC class I/peptide complex that will display a specific epitope on the cell surface. These cells have an excellent antigen presenting ability against epitope specific CTL.

Namely, the present invention provides:
(1) A method for producing an MHC class I heavy chain fused with an epitope or β2m fused with said epitope, wherein said method comprises the steps of:
   (a) inhibiting TAP activity in a cell; and
   (b) expressing said MHC class I heavy chain fused with said epitope or said β2m fused with said epitope in said cell.
(2) The method of (1), wherein said method further comprises a step of an acid treatment.
(3) The method of (1) or (2), wherein said step of inhibiting TAP activity in a cell is a step of contacting a protein having a TAP inhibitory activity with said cell or introducing a vector encoding said protein into said cell.
(4) The method of (3), wherein said protein having a TAP inhibitory activity is US6 or ICP47.
(5) The method of (3), wherein said vector is a mammalian cell-infecting virus vector.
(6) The method of (5), wherein said vector is a Sendai virus vector.
(7) A composition comprising an MHC class I heavy chain fused with an epitope or β2m fused with said epitope, wherein said MHC class I heavy chain fused with said epitope or said β2m fused with said epitope is produced by a method of any one of (1) to (6).

The present invention also relates to a method for the production of mammalian cells that express the epitope-fused MHC class I heavy chain or β2m. Specifically, the method comprises the steps of: (a) inhibiting TAP activity in mammalian cells; and (b) expressing a gene encoding the MHC class I heavy chain or β2m fused with an epitope in the cells. There is no restriction on the order of the steps (a) and (b). Either step may be done first, or both steps may be done at the same time. Cells thus obtained may be useful for inducing an antigen-specific cellular immunity. For example, antigen-specific cellular immunity may be induced inside of a patient's body, by expressing a desired exogenous epitope in a high frequency in cells derived from the patient (for example, dendritic cells) using this method. In particular, the step of inhibiting TAP activity in mammalian cells may preferably be carried out through introduction of a vector encoding a protein having the TAP inhibitory activity into the cells. This leads to the expression of the TAP inhibitory protein in the cells. When the steps (a) and (b) are carried out simultaneously, the present invention also includes a step of co-introducing vectors encoding a protein having the TAP inhibitory activity and the epitope-fused MHC class I heavy chain or β2m into cells, and a step of co-expressing the both proteins from a single vector by inserting the gene encoding the protein having the TAP inhibitory activity into the vector encoding the epitope-fused MHC class I heavy chain or β2m. Alternatively, the step (b) may be applied to cells in which the TAP gene has been deleted or its expression has been suppressed. The epitope-fused MHC class I heavy chain or β2m thus produced may be recovered from these cells or the culture supernatant. The cells with deletion of the TAP gene can be prepared by, for example, a known gene targeting method. A conditional control of the TAP gene expression may also be possible using the Cre-loxP system or the like. The present invention provides mammalian cells in which TAP activity is inhibited and yet containing a gene encoding the epitope-fused MHC class I heavy chain or β2m in an expressible way. In addition, the present invention provides mammalian cells that contain a TAP inhibitor or a gene encoding the TAP inhibitor in an expressible way, and also contain a gene encoding the epitope-fused MHC class I heavy chain or β2m in an expressible way. Herein, the phrase "a cell contains a gene in an expressible way" means that the gene is expressed in the cell or the gene is retained within the cell so that its expression is inducible using drugs or the like. For example, it includes cells capable of inducibly expressing the gene using an inducible promoter or the Cre-loxP system.

Namely, the present invention provides:
(1) A mammalian cell (i) in which TAP activity is inhibited, and (ii) carrying a gene encoding an MHC class I heavy chain fused with an epitope or a gene encoding β2m fused with the epitope in an expressible way.
(2) A mammalian cell (i) in which expression of a TAP gene is inhibited, containing a TAP inhibitor, or carrying a gene encoding the TAP inhibitor in an expressible way, and (ii) carrying the gene encoding the MHC class I heavy chain fused with the epitope or the gene encoding β2m fused with the epitope in an expressible way.
(3) The method of (2), in which the TAP inhibitor is US6 or ICP47.
(4) The method of any one of (1) to (3), in which the gene encoding the TAP inhibitor and/or the gene encoding the MHC class I heavy chain fused with the epitope or β2m fused with the epitope have been introduced into the cell via a mammalian cell-infecting virus vector.
(5) The method of (4), in which the vector is a Sendai virus vector.
(6) A composition comprising the mammalian cell of any one of (1) to (5).

There is no limitation on the mammalian cell-infecting virus vector and any desired virus vector may be used when a TAP inhibitory protein and/or an epitope-linked β2m or MHC class I heavy chain is expressed using a mammalian cell-infecting virus vector. In addition, the mammalian cell-infecting virus vector used in the present invention may have an ability to infect cells other than mammalian cells. Preferably, the vector is less toxic to host cells and achieves a high expression level of a transgenes. The virus vectors suitable for this invention include, for example, adenovirus vectors, adeno-associated virus vectors, herpes simplex virus vectors, retrovirus vectors, lentivirus vectors, Semliki forest virus vectors, Sindvis virus vectors, vaccinia virus vectors, fowl pox virus vectors, and Sendai virus vectors, but the invention is not limited thereto. Using these vector systems, a recombinant virus vector expressing a TAP inhibitor, the epitope-linked-MHC class I heavy chain or β2m, or a TAP inhibitor and the epitope-linked-MHC class I heavy chain or β2m, may be constructed. The phrase "recombinant virus vector" as used herein refers to a virus vector generated via a recombinant polynucleotide. The recombinant polynucleotide is defined as a polynucleotide whose end(s) is/are not bound as in natural state. More specifically, it refers to artificially recombined polynucleotide chains or newly generated polynucleotides. The "recombinant" virus vector includes, for example, those constructed by gene manipulation and that prepared by gene amplification of the constructed vectors. The recombinant virus vectors may be generated by reconstituting virus particles through the expression of recombinant virus cDNA in host cells.

The recombinant virus vectors may be prepared according to methods known to those skilled in the art. For example, an adenovirus vector that is most frequently used for gene therapy can be constructed according to the method of Saito *et al*. (Miyake *et al*., Proc. Natl. Acad. Sci. USA 93: 1320-24 (1996); Kanegae *et al*., "Biomanual Series 4- Gene Transfer and Expression, Methods of Analysis" (in Japanese), 43-58(1994), Yodosha). For example, a 42 kb cosmid pAdexlcw (Kanegae *et al*., Saiboukougaku, 13 (8): 757-763 (1994)) that contains a 31 kb Ad DNA, almost the full-length adenovirus (Ad) type 5 genome except for E1 and E3 regions, is cleaved with an appropriate restriction enzyme (for example, *Swa*I), and then properly desalted and purified by gel filtration. An expression unit for exogenous genes is then used for a ligation reaction with the cleaved cosmid using T4 DNA ligase, and the enzyme is inactivated by heat treatment. The resulting ligate is desalted by gel filtration, and then cleaved with *Swa*I. After phenol treatment and gel filtration, the cleaved product is subjected again to *Swa*I cleavage. A portion of the product is used for *in vitro* packaging using Gigapack XL (Stratagene, USA). The ligated product is then introduced into *E.coli* cells. Cosmids are prepared from some of ampicillin-resistant transformants of the cells, their structures are analyzed by digestion with restriction enzymes to isolate recombinant cosmids wherein the exogenous gene expression unit is inserted in the desired direction (opposite direction to that of the transcription of E1A and E1B). In another series, a DNA-terminal protein complex (DNA-TPC) of adenovirus type 5 (Ad5 strain d1X) that lacks E1 and E3 regions is prepared by the method of Saito *et al*. (Kanegae *et al*., supra). This complex is digested, for example, with *Eco*T22I, and then desalted and purified by gel filtration and such. The *Eco*T22I-digested fragments are mixed with the recombinant cosmid obtained as described above, and then introduced into 293 cells using, for example, Cellphect kit (Pharmacia, Sweden). On the next day, the transfected 293 cells are suspended, and the suspension and 10-fold to 100-fold dilution thereof are inoculated onto 96-well plates. Approximately 2 weeks after, a culture solution containing dead cells is isolated from the wells wherein propagation of recombinant Adenovirus (generated via the recombination in the 293 cells) could be observed. The culture solution is repeatedly frozen and thawed to release the adenovirus particles from cells. After centrifugation, the supernatant (primary virus solution) is used for infection of 293 cells spread onto 24-well plates. Three days later, culture solution containing dead cells is isolated. A portion of the solution is frozen and thawed, and centrifuged as for the preparation of the primary virus solution to obtain the supernatant (secondary virus solution). The residual solution is centrifuged to obtain cells. DNA is prepared from the cells and the structure of the recombinant adenovirus DNA is analyzed by digestion with restriction enzymes to select clones that have been confirmed to have the desired DNA structure. The secondary virus solution is used for infection of 293 cells in larger amounts, the culture solution is similarly frozen, thawed and centrifuged to obtain the third virus solution. After determining the titer of the third virus solution according to the method of Saito *et al*. (Kanegae *et al*., supra), the recombinant adenovirus vector can be obtained (Miyake *et al*., supra; Kanegae *et al*., Acta Paediatr. Jpn, 38: 182-188 (1996)).

In addition, for example, retrovirus vectors (Wakimoto *et al*., Protein Nucleic Acid and Enzyme 40: 2508-2513 (1995)) and adeno-associated virus vectors (Tamaki *et al*., Protein Nucleic Acid and Enzyme 40: 2532-2538 (1995)) may also be used. Methods of efficiently producing these vectors are known in the art.

In the interest of producing other vectors that can be used for gene transfer into mammalian cells, Published Japanese Translation of International Publication No. Hei 6-502069, Examined Published Japanese Patent Application No. (JP-B) Hei 6-95937, and JP-B Hei 6-71429 discloses detailed methods for producing recombinant vaccinia viruses. Furthermore, JP-B Hei 6-34727 and Published Japanese Translation of International Publication No. Hei 6-505626 disclose methods for producing recombinant papilloma viruses. Moreover, Unexamined Published Japanese Patent Application No. (JP-A) Hei 5-308975 discloses a method for producing recombinant adeno-associated virus, and Published Japanese Translation of International Publication No. Hei 6-508039 discloses a method for producing recombinant adenovirus.

In the present invention, particularly preferred mammalian cell-infecting virus vectors include Paramyxovirus vectors. Herein, the phrase "Paramyxovirus vectors" refers to vectors (or carriers) derived from Paramyxovirus that transfer genes into host cells. Sendai virus (SeV), which belongs to the family *Pramyxoviridae,* has recently been developed as a gene transfer vector (Kato, A. *et al*., EMBO J. 16: 578-589 (1997); WO 97/16538; WO 97/16539). The SeV vector is less toxic and expresses extremely high amounts of proteins from a transferred gene. Furthermore, since genes inserted into the vector are not introduced into the host chromosomes, the SeV vector has an advantage of safety. The SeV vector has a high genome stability, and according to a heterogeneous gene expression experiment, it is shown to stably express the inserted heterogeneous gene for a long time with little mutations in the bases of the gene in spite of continuous multiple passages (Yu, D. *et al*., Genes Cells 2, 457-466 (1997)). In addition, the size of the gene that can be introduced and the flexibility of packaging due to the lack of capsid structure protein is another advantageous property of the SeV vector. The SeV vector with replication ability allows for the introduction of an exogenous gene of at least up to 4 kb. In addition, two or more genes may be simultaneously expressed by adding transcription units to the vector. Virus vectors replicated from a vector based on the replicon of SeV reinfect into adjacent cells. Then, multiple copies of RNP replicated in the cytoplasm of the infected cells distribute over daughter cells during cell division, and thus the vector is expected to continuously express. Moreover, the SeV vector has a wide tissue adaptability.

Since Sendai virus vectors are not pathogenic in humans, they are preferred for clinical trials of human gene therapy in view of safety. It is a major obstacle in high efficient gene transfer that, in most cases, introduced DNA must be transported into the nucleus or nuclear membrane must be eliminated for the expression of a transgene via plasmid DNA or such. In the case of Sendai virus, however, expression of an exogenous gene is driven by both cellular tubulin and its RNA polymerase (L protein) in the cytoplasm when viruses replicate. This suggests that the Sendai virus does not interact with chromosomes of host cells, which avoids risks such as cancerization and immortalization of cells. Furthermore, the Sendai virus is known to be pathogenic in rodents causing pneumonia, but is not pathogenic in humans, which is supported by studies showing that the intranasal administration of the wild-type Sendai virus does not cause harm in nonhuman primates (Hurwitz, J. L. *et al*., Vaccine, 15: 533-540 (1997)). These features suggest that Sendai virus vector can be utilized in human therapy, and further support the notion that Sendai virus vectors are one of the promising tools for gene therapy *in vivo* or *ex vivo.*

Herein, the term "Paramyxovirus" is defined as a virus of the *Paramyxoviridae* family or a derivative thereof. Paramyxoviruses used in the present invention include, for example, viruses belonging to the *Paramyxoviridae,* such as Sendai virus, Newcastle disease virus, Mumps virus, Measles virus, Respiratory syncytial virus, rinderpest virus, distemper virus, simian parainfluenza virus (SV5), and type I, II, and III human parainfluenza virus. In the present invention, preferred paramyxoviruses include those belonging to the subfamily *Paramyxoviridae* (including the genera of Respirovirus, Rubulavirus, and Mobillivirus), more preferably the genus Respirovirus (also called Paramyxovirus) or a derivative thereof. Respiroviruses that can be used in the present invention include, for example, type I human parainfluenza virus (HPIV-1), type III human parainfluenza virus (HPIV-3), type III bovine parainfluenza virus (BPIV-3), Sendai virus (also called type I mouse parainfluenza virus), and type X simian parainfluenza virus (SPIV-10). In the invention, most preferred Paramyxovirus is Sendai virus. These viruses may be naturally occurring, wild-type, mutant, laboratory-passaged, artificially constructed strains, etc. Incomplete viruses such as the DI particle (Willenbrink, W. and Neubert, W.J., J. Virol. 68: 8413-8417 (1994)) and synthesized oligonucleotides may be utilized as a material for generating the virus vector.

The recombinant Paramyxovirus vector may be generated by constructing a recombinant DNA containing a DNA encoding the virus genome linked to an appropriate promoter, transcribing the construct *in vitro* or *in vivo,* reconstituting RNP in the presence of Paramyxovirus L, P, and NP proteins, and forming a virus particle containing the RNP. Reconstitution of virus from the vector DNA may be carried out according to the methods known to those skilled in the art (Hasan, M.K. *et al*., J. Gen. Virol. 78: 2813-2820 (1997); Kato, A. *et al*., EMBO J. 16: 578-587 (1997); Yu, D. *et al*., Genes Cells 2: 457-466 (1997); WO 97/16539; WO 97/16538; Durbin, A.P. *et al*., Virol. 235: 323-332 (1997); Whelan, S.P. *et al*., Proc. Natl. Acad. Sci. USA 92: 8388-8392 (1995); Schnell, M.J. *et al*., EMBO J. 13: 4195-4203 (1994); Radecke, F. *et al*., EMBO J. 14: 5773-5784 (1995); Lawson, N.D. *et al*., Proc. Natl. Acad. Sci. USA 92: 4477-4481 (1995); Garcin, D. *et al*., EMBO J. 14: 6087-6094 (1995); Kato, A. *et al*., Genes Cells 1: 569-579 (1996); Baron, M.D. and Barrett, T., J. Virology 71: 1265-1271 (1997); Bridgen, A. and Elliott, R.M., Proc. Natl. Acad. Sci. USA 93: 15400-15404 (1996)). These methods enable the reconstitution of desirable Paramyxovirus vectors, including the parainfluenza virus, vesicular stomatitis virus, rabies virus, measles virus, rinderpest virus, and Sendai virus vectors, and the other (-) strand RNA viral vectors from DNA. Paramyxovirus vectors are generally prepared by the steps of: (a) transcribing a vector DNA encoding a negative single-stranded RNA or its complementary strand (positive strand) derived from Paramyxovirus in cells (helper cells) expressing the NP, P, and L proteins; and (b) culturing the cells and recovering virus particles from the culture supernatants. The RNA transcribed from the vector DNA forms an RNP complex with the NP, L, and P proteins, leading to the generation of virus particles encapsulated in outer envelope containing envelope proteins.

Additional virus vectors in the present invention include those with deletions of virus genes. For example, a replication deficient type virus vector in which an essential gene for the replication of virus has been deleted may be used. When the F gene and/or the HN gene or such are deleted in the Paramyxovirus vector DNA, the virus itself does not form infectious virus particles. Infectious virus particles can be assembled by introducing these deleted genes and/or genes encoding envelope proteins from another virus into the virus-producing cells and expressing them separately. These deletion type virus vectors can also be produced according to or based on the methods known to those skilled in the art (WO 00/70055 and WO 00/70070). Expression levels of the exogenous gene from the vector can be controlled by, for example, the type of transcriptional initiation sequence, which will be added upstream of the gene (WO 01/18223). In addition, virus vectors also include pseudotype virus vectors that contain envelope proteins from other viruses. Such an envelope protein may be VSV-G, for example (WO 00/70055 and WO 00/70070).

The collected animal cell-infectious virus vector may be purified to be substantially pure. The purification can be carried out by known purification/separation methods, including filtration, centrifugation, and column purification, or combinations thereof. The phrase "substantially pure" means that the virus vector is the major portion of a sample in which it is present as a component. Typically, a sample can be confirmed to be a substantially pure virus vector when proteins derived from the virus vector occupy 10% or more, preferably 20% or more, more preferably 50% or more, more preferably 70% or more, more preferably 80% or more, and even more preferably 90% or more, of the total proteins (but excluding proteins added as carriers or stabilizers) in the sample. Specific examples of purification methods for Paramyxovirus include methods using cellulose sulfate ester or cross-linked polysaccharide sulfate ester (JP-B Sho 62-30752; JP-B Sho 62-33879; and JP-B Sho 62-30753), and those including adsorption of the virus with fucose sulfuric acid-containing polysaccharide and/or its degradation product (WO 97/32010).

A high frequency expression of a desired epitope within the cells may be achieved by introducing a mammalian cell-infecting virus vector encoding a TAP inhibitory protein and epitope-linked-β2m or MHC class I heavy chain in an expressible way into mammalian cells. The present invention provides a method for producing an epitope-linked-β2m or an epitope-linked MHC class I heavy chain, which comprises a step of introducing into mammalian cells (one or more) mammalian cell-infecting virus vectors encoding: (i) a TAP inhibitory protein; and (ii) an epitope-linked-β2m or an epitope-linked MHC class I heavy chain in an expressible way. The mammalian cell-infecting virus vectors encoding (i) and (ii) may be different or the same, in the present invention.

Moreover, the present invention provides mammalian cells in which (one or more) mammalian cell-infecting virus vectors encoding in an expressible manner: (i) a TAP inhibitory protein; and (ii) an epitope-linked-β2m or an epitope-linked MHC class I heavy chain have been introduced. The mammalian cell-infecting virus vectors encoding (i) and (ii) may be different or the same, in the present invention. Moreover, the present invention provides the above mammalian cells in which a mammalian cell-infecting virus vector encoding an epitope-free MHC class I heavy chain in an expressible manner has further been introduced when an epitope is linked to β2m, or that encoding an epitope-free β2m in an expressible manner has further been introduced when the epitope is linked to MHC class I heavy chain. These vectors may be the same or different as those encoding the TAP inhibitory protein and the epitope-linked proteins. US6 and ICP47 are the specific examples of the TAP inhibitory protein. In addition, Paramyxovirus vectors are especially preferred as mammalian cell-infecting virus vectors.

An epitope-linked-β2m expressed by the method of the present invention may be used for an *in vitro* CTL assay. Target cells for the CTL assay generally include cell lines derived from the same individual as the CTL. For example, cells of Epstein-Barr virus-transformed B cell line (auto-lymphoblastoid cell line, auto-LCL) pulsed with a synthetic peptide may be used as the target cells. Alternatively, instead of using the synthetic peptide, epitope-linked-β2m prepared by the method of the present invention may be added or the virus vector expressing, for example, the TAP inhibitory protein and the epitope-linked-β2m may be infected into the auto-LCL. Cells of human cell lines can be used similarly through co-infection of virus vectors that express, for example, TAP inhibitory protein, a desired MHC class I heavy chain (membrane-bound form), and an epitope-linked-β2m, which will be displayed on the MHC class I heavy chain, respectively.

Moreover, epitope-linked-β2m produced by the method of this invention may be used to establish epitope-specific cytotoxic T lymphocytes (CTL). Such epitope-specific CTL can be established via specific single or multiple stimulations *in vitro.* For example, HIV Nef138-10-specific CTL can be established by stimulating HIV-infected individual's peripheral blood mononuclear cells (PBMCs) with irradiated autologous PBMC pulsed with the HIV Nef138-10 peptide, and then co-culturing in the presence of IL-2. Herein, a synthetic peptide is usually used, but culture supernatant of cells infected with a virus vector that expresses the epitope-linked-β2m (*i*.*e*., free form epitope-linked-β2m protein) shows a similar effect. Epitope-specific CTL may also be established by infection of a virus vector expressing, for example, the TAP inhibitory protein and the epitope-linked-β2m to the stimulator cells.

The epitope-linked-β2m obtainable by the method of the present invention may be used to induce an antigen-specific cellular immune response. Namely, the epitope-linked-β2m obtained from the present invention may act as an inducer of an antigen-specific cellular immune response. The epitope-linked-β2m obtained according to the present invention may be used to induce the antigen-specific cellular immune response.

Moreover, an epitope-linked-β2m expressed via an SeV expression system, which binds to the HLA-A*2402 molecule on the cell surface as a secretory protein, can present an antigen to CTL efficiently. The epitope-linked-β2m produced by the method of the present invention may be used as a protein preparation. That is, a clinically useful inducer of an antigen-specific cellular immune response may be obtained by purifying the epitope-linked-β2m and removing contaminant viruses from it. The epitope-linked-β2m produced by the method of the present invention may be useful as a component of pharmaceutical compositions.

For example, induction of an antigen-specific cellular immune response using the epitope-linked-β2m may be conducted specifically by a method comprising a step of contacting the epitope-linked-β2m with (epitope-specific) CD8-positive T cells.

For example, the epitope-linked-β2m is useful for establishing *in vitro* epitope-specific CTL cell lines. Epitope-specific CTL cell lines can be established by one to several rounds of specific stimulations *in vitro.* For example, to establish HIV Nef138-10-specific CTL cell lines, PBMC from HIV-1-infected individuals are stimulated with irradiated autologous PBMC pulsed with the HIV Nef138-10 peptide, and then co-cultured in the presence of IL-2. Herein, synthetic peptides are generally used for the stimulation; however, a similar effect can be achieved by addition of the epitope-linked-β2m produced according to the method of the present invention.

In addition, epitope-linked-β2m is useful to pulse target cells in CTL assays *in vitro.* Target cells in the CTL assays may be pulsed with the epitope-linked-β2m produced according to the method of the present invention instead of using the synthetic peptide, which gives the same result as with the synthetic peptide. Generally, a complex of an MHC class I heavy chain (membrane-bound form)+ β2m + peptide is already expressed on the cell surface. Pulsing with a peptide is intended to replace the peptide portion of the complex with the pulsed peptide. An efficient exchange of the β2m and peptide portion of the complex is suggested to occur when pulsed with the epitope-linked-β2m. The pre-existing epitope peptides may be removed by an acid treatment.

Epitope-linked-β2m is also useful to pulse patient's dendritic cells *ex vivo.* Peripheral blood mononuclear cells are isolated from a patient and then differentiation to dendritic cells is induced by a standard method. For example, dendritic cells in which virus vectors expressing an epitope-linked-β2m and a TAP inhibitory protein have been introduced are administered to the patient subcutaneously. As a result, the epitope-linked-β2m is expected to induce epitope-specific CTL due to its expression on the cell surface as a complex of an MHC class I heavy chain and the epitope-linked-β2m similarly as in the *in vitro* experiment. Since the dendritic cells pulsed with the epitope-linked-β2m is expected to express the epitope on the MHC class I heavy chain stably for a long time with higher density than cells pulsed with a peptide, it may be used as a cellular vaccine (therapeutic vaccine).

The mammalian cell-infecting virus vector encoding an epitope-linked-β2m or an MHC class I heavy chain in an expressible way can be used to induce an antigen-specific cellular immune response in combination with a vector capable of expressing a TAP inhibitor or a TAP inhibitory protein. The present invention relates to a composition comprising: (i) a TAP inhibitor or a vector encoding the TAP inhibitor in an expressible way; and (ii) (a) an epitope-linked-β2m or an epitope-linked MHC class I heavy chain, or (b) a vector encoding an epitope-linked-β2m or an epitope-linked MHC class I heavy chain in an expressible way. The vector may be suspended appropriately in a pharmaceutically acceptable solution. For example, the vector may be contained in a physiological saline, a culture solution, or serum. The composition may be useful as an enhancer for displaying an exogenous epitope on MHC class I. The composition may also useful as an inducer of an antigen-specific cellular immune response. The vector encoding the TAP inhibitory protein may be different from that encoding the epitope-linked-β2m or encoding the MHC class I heavy chain, or alternatively, a single vector may encode these proteins. Namely, the present invention also includes a vector encoding both the TAP inhibitor and either of the epitope-linked-β2m or MHC class I heavy chain in an expressible way. Moreover, the present invention relates to uses of: (i) a TAP inhibitor or a vector encoding the TAP inhibitor in an expressible way; and (ii) an epitope-linked-β2m or an MHC class I heavy chain, or a vector encoding the epitope-linked-β2m or MHC class I heavy chain in an expressible way, to raise the exogenous epitope levels displayed on MHC class I, and to induce an antigen-specific cellular immune response. These virus vectors are useful for gene therapy. The present invention provides pharmaceutical compositions comprising: (i) a TAP inhibitor or a vector encoding the TAP inhibitor in an expressive way; and (ii) an epitope-linked-β2m or an MHC class I heavy chain, or a vector encoding the epitope-linked-β2m or MHC class I heavy chain in an expressible way. In addition, the present invention relates to a kit for displaying an epitope, comprising: (i) a TAP inhibitor or a vector capable of expressing the TAP inhibitor; and (ii) an epitope-fused MHC class I heavy chain or β2m, or a vector capable of expressing the epitope-fused MHC class I heavy chain or β2m. (i) and (ii) may be separated if possible, or mixed. The kit may include multiple kinds of TAP inhibitors or vectors capable of expressing them, and may also include multiple kinds of MHC class I heavy chains or β2m, or vectors capable of expressing the multiple kinds of MHC class I heavy chains or β2m. These may be appropriately dissolved or suspended in a pharmaceutically acceptable solution. Particularly preferred vectors are animal cell-infecting virus vectors. The compositions containing the vector and the kit of the present invention are useful to induce antigen-specific immune responses for infectious diseases, cancers, and such, due to their antigen-specific cellular immune response-inducing ability. Applications of the compositions or the kit of the present invention to gene therapy may be achieved through gene expression by either a direct (*in vivo*) administration or an indirect (*ex vivo*) administration of the compositions or the kit.

For example, in the process of *in vitro* establishment of epitope-specific CTL cell lines, stimulation of stimulator cells may be achieved by applying the above compositions or kit instead of using a synthetic peptide.

The compositions and the kit of the present invention may also be used to pulse target cells in an *in vitro* CTL assay. Auto-LCL cells infected with a virus vector expressing epitope-linked-β2m under conditions wherein TAP is inhibited, for example, instead of using a synthetic peptide, may be used as target cells to detect cytotoxic activities. In addition, to use human cell lines as target cells, they may be co-infected with a virus vector expressing a desired MHC class I heavy chain (membrane-bound form) and a virus vector that expresses an epitope-linked-β2m presented by the MHC class I heavy chain and a TAP inhibitory protein.

Furthermore, the composition and the kit of the present invention are useful for *ex vivo* expression of an epitope in patient's dendritic cells. Peripheral blood mononuclear cells are isolated from a patient and induced to differentiate into dendritic cells by a standard method. The composition of the present invention is administered to the dendritic cells, and then the cells are inoculated to the patient subcutaneously. Especially, a gene transfer using a virus vector may be suitable. The important point here is how long the expression of a pulsed peptide or the epitope-linked-β2m protein continues on the surface of the pulsed dendritic cells. In fact, when the cells are pulsed with a peptide, the expression of the epitope on the cell surface of the dendritic cells disappears in a short term. In contrast, a long time expression of the epitope-linked-β2m can be achieved via the infection of the dendritic cells with the virus vector to allow intracellular expression of the epitope-linked-β2m. Furthermore, the compositions and the kit of the present invention are also useful for *in vivo* gene therapy wherein epitope-specific CTL is induced by *in vivo* administration of the compositions and the kit.

Target cells for gene transfer to express the exogenous epitope preferably include those having antigen presenting ability. Such cells particularly include dendritic cells (DC). For example, a desired MHC class I/peptide complex may be formed in a high frequency in the dendritic cells by introducing a vector expressing an exogenous epitope-linked-β2m or an MHC class I heavy chain into the dendritic cells *ex vivo,* using the method of the present invention. The resultant cells may be used for the activation of antigen-specific T cells. Mammalian cells in which a gene encoding the epitope-linked-β2m or an MHC class I heavy chain has exogenously been introduced and yet TAP activity has been inhibited, are included in the present invention. Desired isolated cells having an antigen-presenting ability may be used as the mammalian cells. In particular, an exogenous gene encoding a subunit of an MHC class I/peptide complex, which is not linked to the epitope, may also be introduced into the cells, to form a large amount of the MHC class I/peptide complex. The transgene may be encoded by an expression vector outside the chromosome or integrated into the cellular chromosome. When a membrane-bound form of the MHC class I heavy chain is expressed, a membrane-bound MHC class I/peptide complex is expressed on the cell surface. Such cells have an excellent antigen-presenting ability to epitope-specific CTL. Above mentioned mammalian cells in which an exogenous gene encoding the epitope-linked-β2m or the epitope-linked MHC class I heavy chain has been introduced and yet TAP activity has been inhibited may also be used as a component of pharmaceutical compositions.

The compositions of the present invention may be combined with desired pharmaceutically acceptable carriers or vehicles as necessary. Herein, the phrase "pharmaceutically acceptable carriers or vehicles" denotes materials capable of being administered to cells and that do not significantly inhibit activities of the active ingredients. For example, the composition of the present invention may include physiological saline, phosphate-buffered saline (PBS), etc. When a Paramyxovirus vector has been propagated in chicken eggs, the composition containing the vector may contain allantoic fluid. Moreover, the composition containing cells may be suspended in physiological saline, PBS, culture solution, or the like. In addition, the composition of the present invention may contain carriers or vehicles such as deionized water and 5% dextrose solution. Furthermore, it may contain others, including vegetable oils, suspending agents, detergents, stabilizers, and antibiotics. In addition, preservatives and other additives may be added. The compositions of the prevent invention are useful as reagent and medicines. The compositions are further useful as vaccines. The present invention also relates to uses of the compositions of the present invention as reagents, medicines, or vaccines. To enhance immunogenicity, the vaccine compositions may also contain immune accelerators including cytokines, cholera toxins, and salmonella toxins. Furthermore, the vaccines may be combined with adjuvants including alum, incomplete Freund's adjuvant, MF59 (oil emulsion), MTP-PE (muramyl tripeptide derived from Mycobacterium cell wall), and QS-21 (derived from soapbark tree *Quilaja saponaria*).

Moreover, cytokines can be effectively combined to enhance the adjuvant effect of the pharmaceutical compositions of the present invention for administration. Such combinations include: i) combination of IL-2 and single-stranded IL-12 (Proc. Natl. Acad. Sci. USA 96(15): 8591-8596 (1999)), ii) combination of IL-2 and interferon-γ (US Pat. No. 5,798,100), iii) GM-CSF alone, and iv) combination of GM-CSF and IL-4 for the treatment of brain tumors (J. Neurosurgery 90(6): 1115-1124 (1999)).

The vaccines of the present invention may be applied, for example, to tumors, infectious diseases, and other general diseases. For the treatment of infectious diseases, for example, epitopes of antigen proteins of infectious microorganism are analyzed, and then β2m or MHC class I heavy chain fused with the epitopes are produced. Antigen proteins include, for example, envelope of virulent strain type H5N1 for influenza; for Japanese encephalitis, the envelope protein (Vaccine 17(15-16): 1869-1882 (1999)); HIV gag or SIV gag proteins (J. Immunology 164: 4968-4978 (2000)), HIV envelope proteins, the Nef protein and other virus proteins for AIDS; B subunit (CTB) of cholera toxin (Arakawa, T. *et al*., Nature Biotechnology, 16(10): 934-8 (1998); Arakawa, T. *et al*., Nature Biotechnology, 16(3): 292-7 (1998)) as an example of cholera antigens; glycoprotein of rabies virus (Lodmell, D.L. *et al*., Nature Medicine 4(8): 949-52 (1998)) as an example of rabies antigens; and capsid protein L1 of human papilloma virus type 6 (J. Med. Virol 60: 200-204 (2000)) as an example of cervical cancer antigens. Furthermore, the present invention may be applied to pathogenic Paramyxovirus, such as Measles virus and epidemic parotiditis virus, that highly require the use of vaccines. Moreover, epitopes of antigen proteins including JE-E antigen protein of Japanese encephalitis (JP-A Sho 64-74948, JP-A Hei 1-285498), gD2 protein of human herpes simplex virus (JP-A Hei 5-252965), hepatitis C virus-derived peptide (JP-A Hei 5-192160), and pseudorabies virus-derived polypeptide (Published Japanese Translation of International Publication No. 7-502173), may also be used. For example, cells from patients infected with these pathogenic microorganisms are analyzed to identify epitopes of the antigen proteins presented on antigen-presenting cells (APC). An epitope for the desired HLA type can be identified by selecting a proper HLA type.

It is clinically significant in treating tumor to develop diverse and new therapeutic strategies and methods, focusing on tumor-specific antigens. For example, in tumor therapy, an epitope-linked-β2m or MHC class I heavy chain may be expressed in antigen-presenting cells (APC), such as tumor cells or DC cells, or protein products, such as epitope-linked-β2m and MHC class I/peptide complex, may be administered.

General means to develop a vaccine therapy includes the use of DC cells known to have antigen-presenting ability to helper T cells (Th) as well as high antigen-presenting ability to CTL via MHC class I molecules (Mayordomo, J.I. *et al*., Nature Med. 1(12): 1279-1302 (1995)). As an example of tumor immunotherapy, studies to establish a tumor vaccine therapy utilizing the MAGE antigen, which expression is confirmed in a variety of malignant tumors, as a target are in progress by Yasushi Fuji *et al*. (National Hospital Kyushu Cancer Center). The method was applied to 7 cases, including 5 cases of recurrent gastric tumor and a case of recurrent esophagus tumor. As a result, shrinkage of the metastatic lymph node, a decrease in tumor markers, and improvement of clinical symptoms (hoarseness) were observed in the recurrent esophagus tumor case. In the gastric tumor cases, a decrease in tumor markers was observed in 4 of 6 cases. No side effects were observed, suggesting the safety of the tumor immunotherapy. Moreover, in many cases, improvement of clinical symptoms and a decrease in tumor markers were observed, indicating the possibility that the immunotherapy serves as an effective therapeutic method through selection of the administration route, adaptable cases, and such. Clinical studies on the DC vaccine therapy using the MAGE-3 peptide have been practically started, and are suggested to be a safe and tumor-specific immunotherapy against progressive recurrent digestive organ cancer cases. However, for preventive administration of such vaccines to many patients, it is clearly troublesome and unpractical to find an optimal condition for administration of the dendritic cells as "natural adjuvant" to each patient. Therefore, efficient methods are desired in the art (Yamagishi, H. *et al*., Oral presentation in the General meeting of Japan society of Cancer Therapy: October 12 (1999), Gifu City). The application of the present invention to such a tumor vaccine therapy may be quite effective.

The prevention of tumorigenesis triggered by viruses may be achieved by preventing infection using a virus vaccine. Thus, virus-caused tumors can be more practically prevented compared with those caused due to other factors. For example, hepatitis C virus (HCV) associated with hepatic tumors, human papillomavirus (HPV) associated with uterine cancer, and HTLV-1 associated with adult T cell leukemia are candidates for vaccines aiming at prevention and therapy of infections. Hepatic tumors share a high ratio in Japanese tumors. Infection by hepatitis C virus occurs non-orally, and at high frequency becomes chronic even in healthy adults with normal immunocompetence. Approximately 20% of the infected individuals are predicted to suffer chronic hepatitis or cirrhosis. Moreover, many of the cirrhosis patients acquire liver cell cancer. While interferon treatment of hepatitis C made it possible to cure a part of the patients, its efficacy was not as initially expected and even now, there is no effective therapy for more than half of the patients. Generally, viral infections are prevented by inducing neutralizing antibodies via vaccination. However, hepatitis C virus is susceptible to mutation and the existence of a neutralizing antibody that interrupts its infection has not been proven so far. Virus infections are known to be protected by inducing CTL in addition to the induction of neutralizing antibodies. The present invention is expected to induce activation of CTL in such virus infections.

Moriyama, T. *et al*. (Jichi Medical School) have been studying methods using genes themselves that encode pathogenic antigens as vaccines (DNA vaccines) as a novel method to induce CTL. The method has problems, such as insufficient expression and the administration site being limited to the muscles, as described in their articles (Kurane, I. "DNA vaccine, present situation and new findings", Current Concepts in Infectious Diseases 19(3): 6-9 (2000)). Another article describes a cancer vaccine that utilizes HER2, a membrane type glycoprotein having tyrosine kinase activity, that is found overexpressed in breast cancers (Kageyama, S., Watanabe, M., Hiasa, A., and Suku, K., "Cancer and immunity, cancer-specific immune therapy, vaccine therapy against breast cancer using a HER-derived peptide", New Horizon for Medicine 32(5): 1167-1172 (2000)). For example, cancer vaccines produced using virus vectors according to the present invention are expected to exert higher effect than these DNA vaccines. Highly effective vaccines may be provided by constructing vectors using virus vectors that have a wide expression range among mammal tissues (adenovirus vectors, adeno-associated virus vectors, herpes simplex virus vectors, retrovirus vectors, Lentivirus vectors, Semliki forest virus vectors, Sindbis virus vectors, vaccinia virus vectors, Sendai virus vectors, etc.).

Although uterine cancer is a female-specific tumor, the significance to develop vaccines for the prevention and therapy of HPV infection is the same as other tumors. The main infection route of HTLV-1 is mother-to-child transmission. However, there are also other routes. Although the pathogenicity of recently discovered HGV is unclear, it is widespread in society like HCV, indicating the need to prevent such virus at the perspective of public health. Thus, such virus is also a target for the application of the present invention.

Investigations concerning applications of antigen-presenting cells and the route of administration are also important. The most extensively studied route of administration for vaccine therapy comprises the steps of: (1) differentiating monocytes contained in the peripheral blood into DC *in vitro;* and (2) returning them into the patient's body via intravenous injection. This method utilizes the ability of DC cells to present antigens via MHC class I molecules to CTL. This system requires respectable facilities and time for culture. Recently, vaccine therapy utilizes skin is featured. The investigations of anti-virus vaccine therapy and cancer vaccine therapy utilizing dendritic cells including Langerhans cells (LC) that were recently found to efficiently present endogenous antigens, such as virus antigens and cancer antigens, to CTL via MHC class I molecules on the cell surface are attracting attention. Since many LC cells are on the epidermis of skin, efficient DNA vaccine therapy may be developed by applying virus peptides or genes encoding antigens on the skin. However, LC cells in normal skin are in the dormant state, and have low antigen presenting activity to Th and CTL, as well as poor mobility into the lymph node. Therefore, it is difficult to realize a virus vaccine therapy or cancer vaccine therapy using normal skin. To solve these problems, Naohiro Seo and Masahiro Takigawa (Hamamatsu Univ. School of Medicine), along with colleagues, demonstrated that LC cells on the skin become activated, then move into the lymph node and efficiently present antigens to Th cells by disrupting the most external skin layer, the corneum barrier, through 8 to 15 times repeated tape stripping (TS) (Japanese Patent Application No. Hei 10-316585, "Percutaneous peptide immunization via corneum barrier-disrupted murine skin for experimental tumor immunoprophylaxis"; Proc. Natl. Acad. Sci. USA 97: 371-376 (2000)). This method expands the possibility of virus vaccines and cancer therapy due to its potential to enable application of virus peptides, cancer peptides or antigen DNAs to the barrier-disrupted skin. These administration methods can be applied to the present invention.

To accomplish more confident clinical applications, it is important to construct human HLA-restricted cancer-specific CTL cell lines, and clone genes encoding cancer-reactive CTL-inducing antigens recognized by the cell lines to develop target molecules for clinically applicable tumor-specific immunotherapy against cancer patients. Immune response is expected to be induced more efficiently by identifying an epitope presented by an HLA of the same type as that of a patient, and producing a vaccine vector or peptide vaccine using the identified epitope.

Cancers that frequently occur in the Japanese population include, for example, lung cancer, digestive tract cancer (esophagus, stomach, and colon cancers), liver cancer, head and neck cancer, breast cancer, uterine cancer, oophoroma, nephoroma, and leukemia. Among these cancers, it is clinically useful to choose the tissue type of squamous cell carcinoma and adenocarcinoma as therapeutic targets, because they share most of these cancers in Japanese. Epidermal cancers share not only the majority of adult malignant tumors in Japanese, but also occur most frequently in the world. Therefore, epitopes of epidermal cancer cell-specific antigens are suitably used in the present invention. Moreover, as for HLA, the identification of HLA-A24 (approximately 60% of cancer patients), HLA-A2 (approximately 40%), and HLA-A26 (approximately 20%)-restricted CTL-recognized cancer-regressive antigens are particularly important, since these HLA types are also dominant in Japan. Following these types, identification of antigens intended to HLA-A11 (20%), HLA-A31 (up to 20%), and HLA-33 (up to 20%) are also important. Ninety five percent or more of the Japanese population possess at least one of HLA-A24, -A2, -A26, -A31, and -A33. Furthermore, these HLA alleles are distributed widely beyond the ethnic difference. Therefore, it is preferable to identify genes encoding cancer-reactive CTL-inducing antigens from cells having these HLA types, and apply them to the present invention.

Kyogo Ito (Medical school of Kurume Univ.) *et al*. have established many specific killer T cells against human HLA class-restricted epidermal cancers (adenocarcinoma and squamous cell carcinoma) which frequently occur among the Japanese, and cloned genes encoding antigens recognized by the cells and that have the ability to induce epidermal cancer-reactive CTL. Furthermore, they identified cancer antigen peptides encoded by the genes, and have been analyzing their *in vitro* killer T cell-inducing abilities (Tou, U., Yamana, H., Sueyoshi, S., Shintani, F., Tanaka, T., Kubota, M., Mine, T., Sasahara, H., and Ito, K., "Cloning and analysis of an antigen gene from the peripheral blood lymphocytes after the specific adoptive immunotherapy by a local injection of CTL from a patient carrying esophagus cancer", The Japanese Society of Gastroenterological Surgery 33(7): 1191 (2000)). Hitherto, 4 genes (SART-1 to SART-4) and 3 genes from squamous cell carcinoma and adenocarcinoma cDNA libraries, respectively, were cloned and their coding proteins were analyzed. Selective apoptosis was induced in cancer cells introduced with the SART-1 gene. In addition, the HLA-A24-restricted peptide (SART-1 690-698) strongly induced CTL, and the HLA-A26-restricted peptide (SART-1 736-744) induced CTL in cancer patients with HLA-A2601, -A2602, or -A2603 (Yamana, H. and Ito, K. (Medical School of Kurume Univ.), "Antigen peptide therapy of tumors; cancer immunotherapy using the SART-1 antigen peptide", Journal of Clinical and Experimental Medicine 190(2): 129-133 (1999); Inoue, K. (Research Institute of National Cancer Center), Nakao, M., Matsunaga, K., Matsuoka-kikuchi, S., Yamana, H., and Ito, K. (Medical School of Kurume Univ.), "Induction of CTL in peripheral blood lymphocytes from HLA-A26-positive healthy subjects and cancer patients with different HLA subtypes, using the SART-1 peptide", General meeting abstracts of Japanese Cancer Association). Furthermore, the 140 kD SART-3-reactive cancer-reactive CTL-inducing antigen was found to selectively express in proliferating cells and also in the nucleus of cancer cells, and two nonapeptides that have CTL-inducing ability to the lymphocytes of HLA-A24-positive cancer patients were identified within the antigen (Yamana, H., Sasatomi, T., Miyagi, Y, Tou, U., Shiromizu, K., and Ito, K. (Medical School of Kurume Univ.), Japan Surgical Society (supplement) 101: 417 (2000)). In the interest of the expression of these cancer-reactive CTL-inducing antigens at the protein level in various cancers, the SART-1 antigen was expressed in 60 to 80% of squamous cell carcinomas and in 40 to 50% of adenocarcinomas except for breast cancer; SART-2 in more than 60% of squamous cell carcinomas; and SART-3 in most malignant tumors including adenocarcinomas and squamous cell carcinomas. In contrast, these antigens are not expressed in normal tissues except the testicle (Ito, K., Shichijo, S., and Yamana, H. (Medical School of Kurume Univ.), "Tumor immunity 9, Human cancer-specific T cell-recognized antigen 2, Squamous cell carcinoma-reactive CTL-inducing antigen SART-1 and peptide vaccines", Immunology Frontier 9(3): 195-204 (1999)). HLA-A26 and HLA-A*2402 are shared in 22% and approximately 60%, respectively, in Japanese. Therefore, these peptide vaccines are expected to be applicable to many squamous cell carcinoma patients in Japan. Phase 1 clinical studies using the above peptides are being planned in Kurume Univ. (Yamana, H. and Ito, K. (Medical School of Kurume Univ.), "An explanation of guideline to clinical studies", A proposal regarding the first clinical studies on tumor peptide vaccines, Cellular Molecular Medicine 1(1): 89-95 (2000)). The application of the present invention may be useful for epitopes derived from these antigen peptides.

An effective immunotherapy can be conducted against tumors, pathogenic infectious microorganisms, or the like, by applying genes encoding the above-described peptide antigens to the present invention. Apart from these antigens, epitopes include the cancer antigen Muc-1 and the Muc-1-like mucin tandem repeat peptide (US Pat. No. 5,744,144), and the melanoma gp100 antigen. Immunotherapy using these genes has been applied to a variety of cancers, including breast cancers, colorectal cancers, pancreatic cancers, prostate cancers, lung cancer, etc. In addition, the gene encoding the above-mentioned tumor antigen peptide HER2 was found to be overexpressed or amplified in about 20 to 40% of cases of breast cancers, oophoromas, stomach cancers, and non-small cell pulmonary carcinomas, indicating high tendency of tumor specificity. Two nonapeptides (HER2p63-71 and HER2p780-788) originating from HER 2, which peptides are derived from dendritic cells purified from peripheral blood mononuclear cells of oophoroma patients and normal healthy subjects are exemplified as epitopes of the present invention (Eur. J. Immunol. 30: 3338-3346 (2000)). Moreover, the pharmaceutical compositions of the present invention may be applied to cancer vaccine therapy for CEA-positive progressive solid tumors using CEA epitope peptides (Kim, C. *et al*., Cancer Immunol. Immunother. 47: W 90-96 (1998)). For example, large amounts of peripheral blood mononuclear cells may be isolated from a patient by selected collection of blood components, dendritic cells are induced by the addition of IL-4 and GM-CSF from the mononuclear cell fraction, induced dendritic cells are then introduced with β2m containing CEA epitope peptide produced by the method of the present invention or the vector itself, and finally the dendritic cells are subcutaneously administered as "DC vaccine" (Okamoto, K., Shirokazu, T., Sakakura, C., Otsuji, E.,Kitamura, K., and Yamagishi, K. (Kyoto Prefectural University of Medicine), "A case of bone metastatic lung cancer wherein dissociation between serum CEA values and anti-tumor effect was achieved via CEA-specific active immunotherapy", International Association of Surgeons and Gastoenterologists).

Optionally, the present invention may be applied to general diseases. In the interest of diabetes, for example, an insulin fragment peptide may be used as an epitope to type I diabetes animal model (Coon, B. *et al*., J. Clin. Invest. 104(2): 189-194 (1999)).

When used as vaccines, the compositions of the present invention may be administered at a quantity sufficient to at least partially induce antigen-specific cellular immune response. However, the administration dose of the protein or expression level of the gene should be determined by considering its effective level and intoxication level. The route of administration may be appropriately selected from, for example, percutaneous, pernasal, perbronchial, intramuscular, intraperitoneal, intravenous, intraarticular, and subcutaneous administrations, but is not limited thereto. They may be administered local or systemically. The induction of cellular immunity can be detected by such as CTL assay as described in the present invention.

The expression levels of genes introduced into cells using the vector-containing composition may be assayed by methods known to those skilled in the art. Transcription products from the genes may be detected and/or quantified by, for example, Northern hybridization, RT-PCR, or RNA protection assay. The detection by Northern hybridization and RNA protection assay can be also performed *in situ*. Western blotting, immunoprecipitation, RIA, ELISA, Pull-down assay, and such, using antibodies may be conducted for the detection of translation products. Moreover, for easier detection of expression products, tags may be attached to proteins to be expressed or reporter genes may be inserted into the vectors so that they are expressed. Examples of the reporter genes include β-galactosidase, CAT, alkaline phosphatase, and GFP proteins; however, they are not limited thereto.

The dose of protein compositions, such as the epitope-linked-β2m or the MHC class I/peptide complexes, may vary depending on the disease to be treated, body weight, age, sex, and symptom of the patient, the purpose of administration, the type of the administered composition, the route of administration, and such, but it can be appropriately determined by those skilled in the art. The protein dose may be, for example, within the range of 10 ng/kg to 100 µg/kg, preferably 100 ng/kg to 50 µg/kg, and more preferably 1 µg/kg to 5 µg/kg. When a combination of multiple epitopes is used for administration, each of the epitopes may be administered at the above dose. The protein compositions such as epitope-linked-β2m or the MHC class I/peptide complexes may be properly combined with pharmaceutically acceptable carriers.

The dose of the vector compositions may vary depending on the disease, body weight, age, sex, symptom, purpose of administration, transgene, and such, but it can be appropriately determined by those skilled in the art. A Paramyxovirus vector is preferably administered at a concentration within the range of about 10⁵ pfu/ml to about 10¹¹ pfu/ml, more preferably about 10⁷ pfu/ml to about 10⁹ pfu/ml, and most preferably about 1x 10⁸ pfu/ml to about 5x 10⁸ pfu/ml, within a pharmaceutically acceptable carrier. The dose is preferably about 10⁵ pfu/round to 10¹¹ pfu/round, more preferably about 10⁷ pfu/round to 10⁹ pfu/round, and most preferably about 10⁸ pfu/round to 10⁹ pfu/round. When different vectors are administered in combination, each of the vectors may be administered in the above dose.

The compositions of the present invention may be administered to all mammalian animal, including humans, monkeys, mice, rats, rabbits, sheep, cattle, and dogs.

### Brief Description of the Drawings

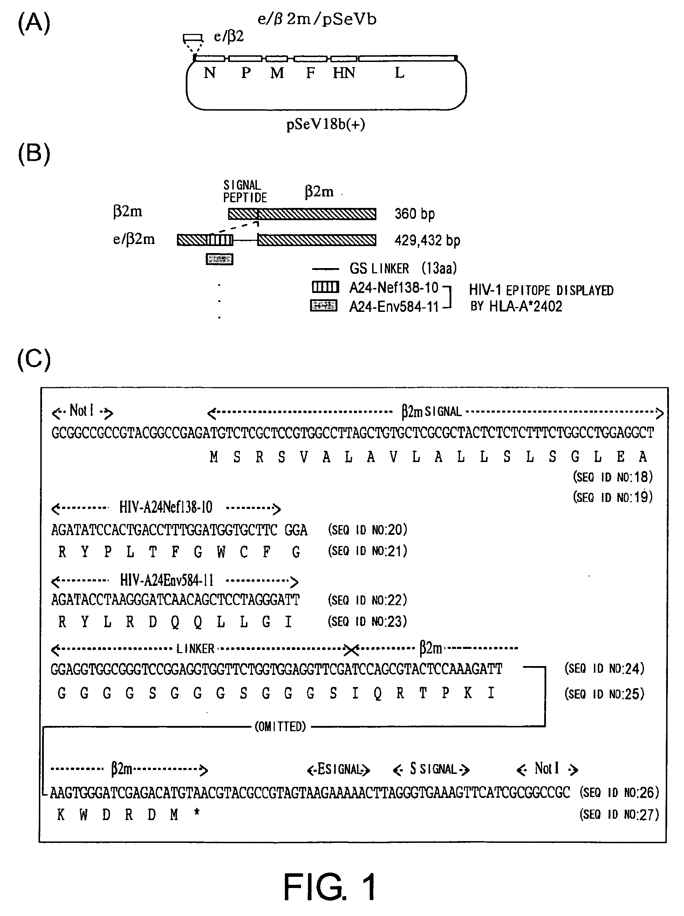

Fig. 1 shows the structure of the plasmid (e/β2m/pSeVb) for the construction of the SeV vector that expresses epitope-linked-β2m (e/β2m). (A) depicts the structure of e/β2m/pSeVb with an insertion of the coding sequence for e/β2m at the *Not*I site of pSeV18b (+). (B) depicts the structure of β2 microglobulin and epitope-liked-β2 microglobulin encoded by the vector.
   (C) depicts the nucleotide sequence of the inserted *Not*I fragment.

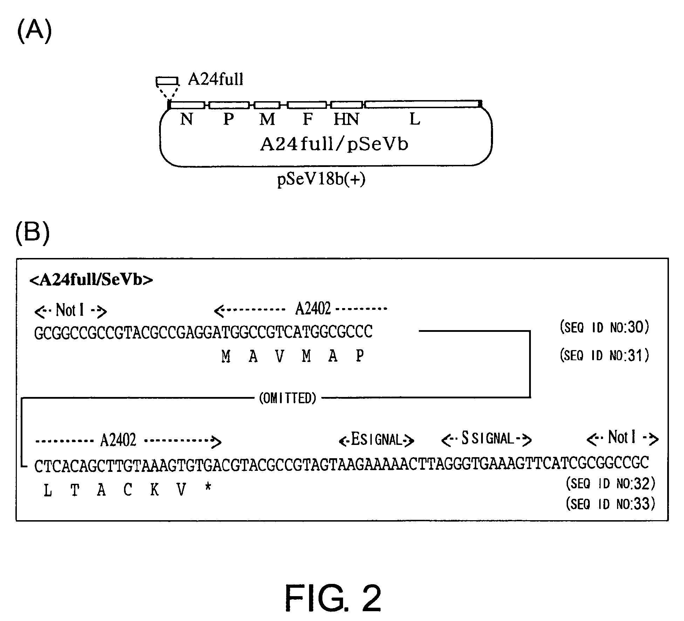

Fig. 2 shows the structure of the plasmid (A24full/pSeVb) for the construction of the SeV vector that expresses the membrane-bound form of HLA-A*2402. (A) depicts the structure of A24full/pSeVb with an insertion of the coding sequence for A24full at the *Not*I site of pSeV18b(+). (B) depicts the nucleotide sequence of the inserted *Not*I fragment.

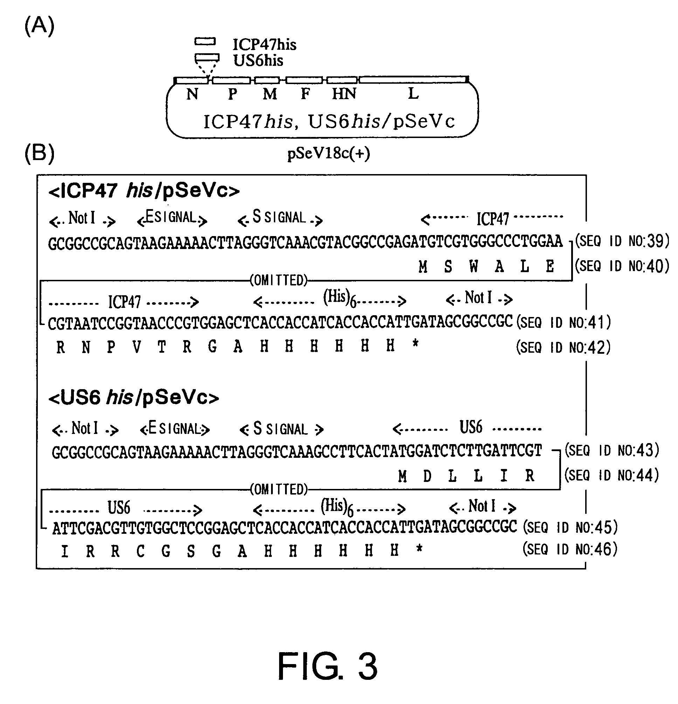

Fig. 3 shows the structure of the plasmid for the construction of the SeV vector that expresses the TAP inhibitory factor. (A) depicts the structures of ICP47*his*/pSeVc and US6*his*/pSeVc with insertions of the coding sequences for ICP47*his* and US6*his*A24full, respectively, at the *Not*I site of pSeV18c (+) (between the N and P genes). (B) depicts the nucleotide sequences of the inserted *Not*I fragments.

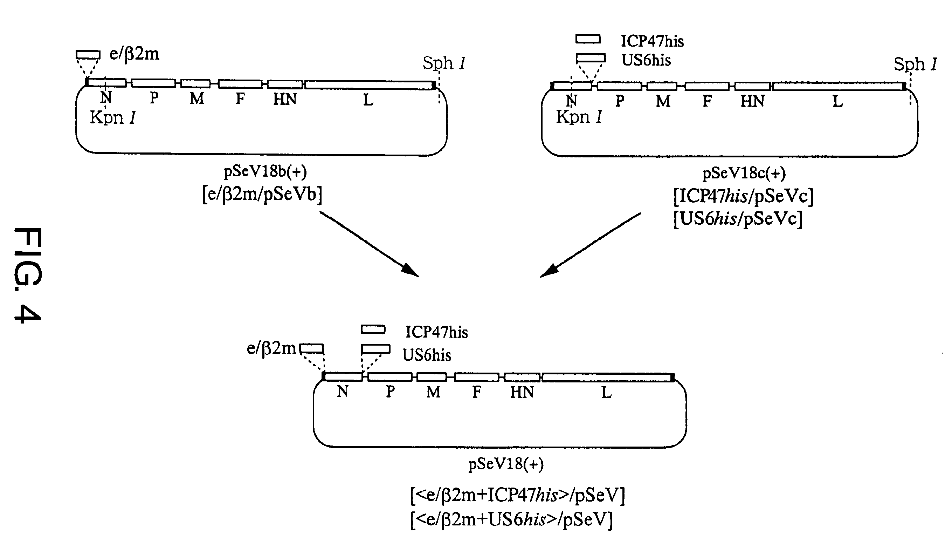

Fig. 4 shows the structure of the SeV vector co-expressing the epitope-linked-β2m (e/β2m) and the TAP inhibitory protein (ICP47his or US6his). <e/β2m+ICP47*his*>/pSeV was constructed from e/β2m/pSeVb and ICP47*his*/pSeVc, and <e/β2m+US6*his*>/pSeV was constructed from e/β2m/pSeVb and US6*his*/pSeVc.

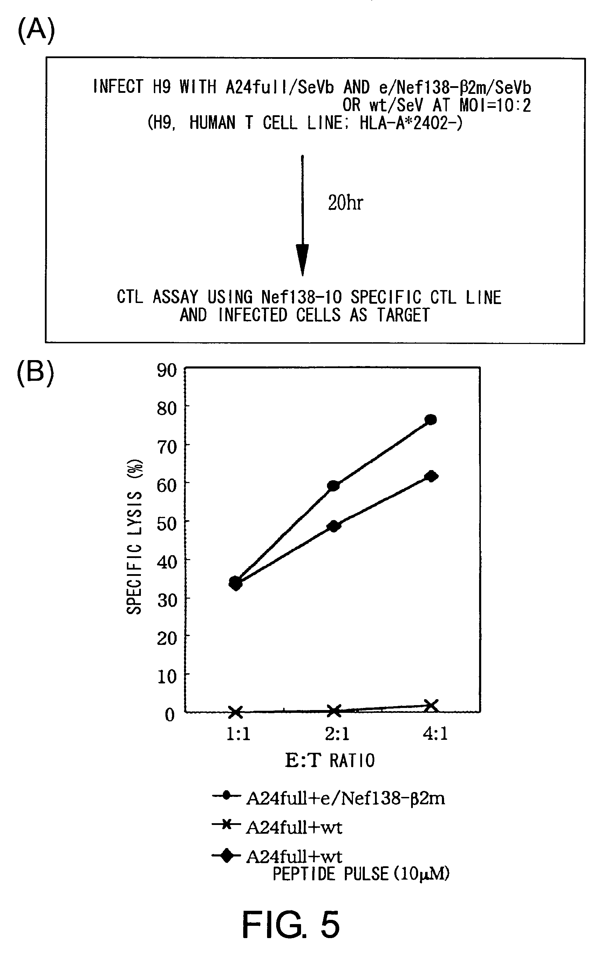

Fig. 5 shows the effect of the membrane-expressed form of the MHC class I/peptide complex. H9 cells (HLA-A*2402-human T cell line) were co-infected with A24full/SeVb and e/Nef138-β2m/SeVb at multiplicity of infection (m.o.i.) 10 and 2, respectively (indicated as "A24full+e/Nef138-β2m" in the figure). As a negative control, H9 cells co-infected with A24full/SeVb and wt/SeV instead of e/Nef138-β2m/SeVb were used (indicated as "A24full+wt" in the figure). In addition, H9 cells were pulsed with a peptide, as a comparative experiment (indicated as "A24full+wt peptide pulse (10 µl)" in the figure). Eighteen hours later, the cells were labeled with 100 µCi Na₂⁵¹CrO₄ for 2 hours to perform ⁵¹Cr release assay using the Nef138-10-specific CTL clone. (A) shows the procedure for the assay, and (B) shows the result.

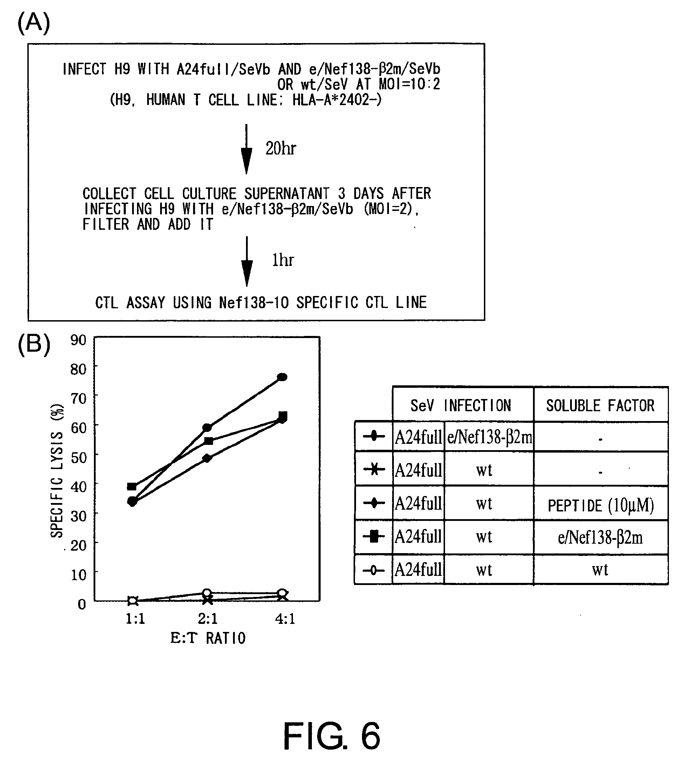

Fig. 6 shows effects of the secreted form of the epitope-linked-β2m. H9 cells (HLA-A*2402 human T cell line) were infected with e/Nef138-β2m/SeVb or wt/SeV as the control at m.o.i. 2, and 3 days later, the culture supernatant was collected and filtered using a 0.22 µm filter.
   As target cells, H9 cells (HLA-A*2402 human T cell line) were co-infected with A24full/SeVb and wt/SeV at m.o.i. 10 and 2, respectively. Eighteen hours after the infection, the target cells were labeled with 100 µCi Na₂⁵¹CrO₄ for 2 hours. After that, cells were pulsed with a culture supernatant containing e/Nef138-β2m prepared as above (closed squares), 10 µM synthetic Nef138-10 peptide (closed diamonds) as a positive control, or a culture supernatant of wt/SeV-infected cells as a negative control (open circles). One hour later, the ⁵¹Cr release assay was performed using the Nef138-10-specific CTL clone. As a comparative experiment, H9 cells were co-infected with A24full/SeVb and e/Nef138-β2m/SeVb at m.o.i. 10 and 2, respectively, and used for the assay in a similar way (closed circles). (A) shows the procedure for the assay, and (B) shows the result.

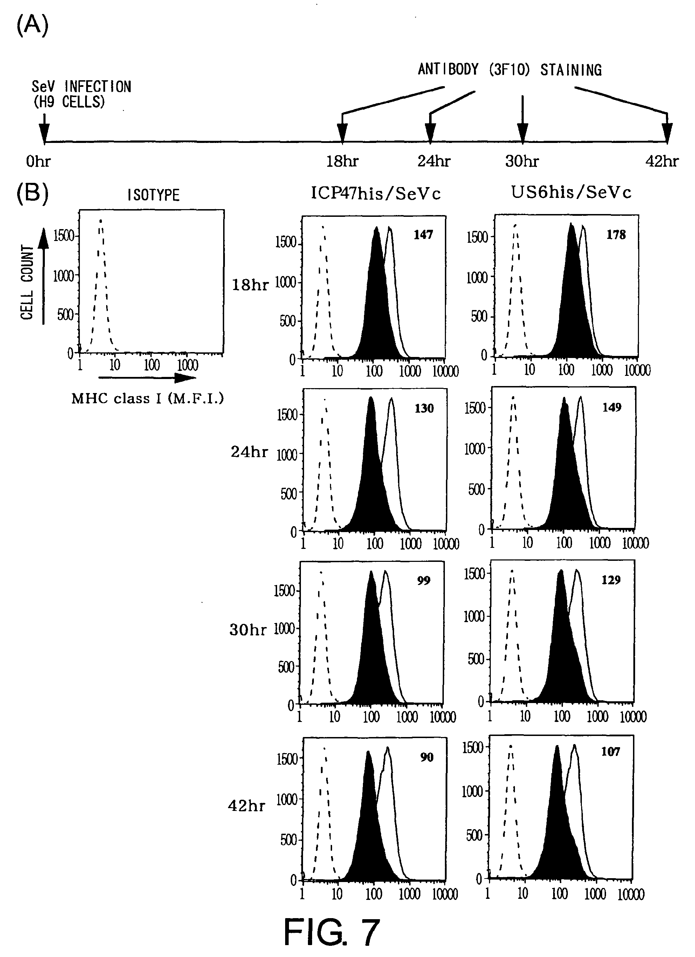

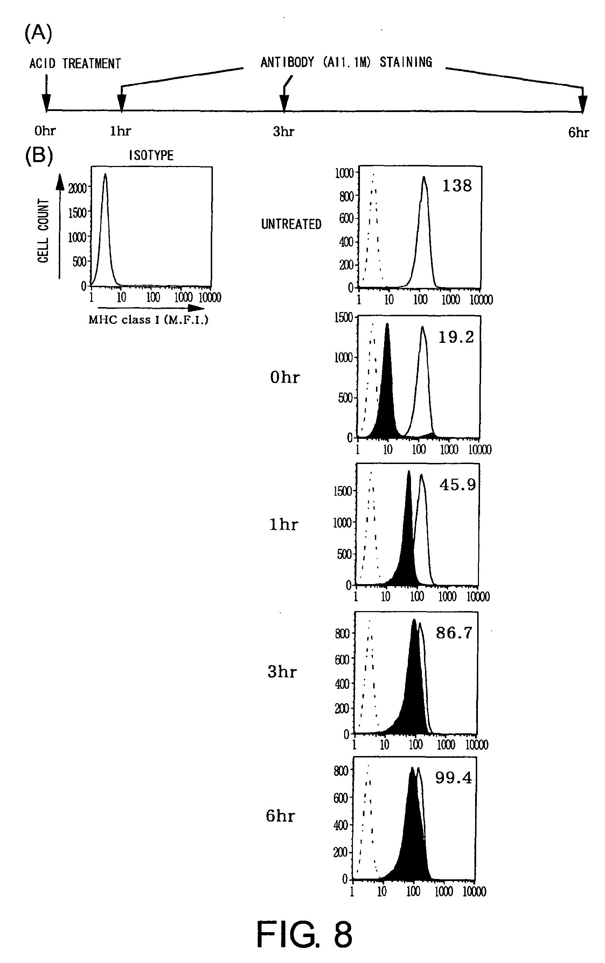

Fig. 7 shows the decrease in MHC class I/peptide complex levels on the cell surface by introducing the SeV vector expressing the TAP inhibitory protein. H9 cells were infected with ICP47*his*/SeVc or US6*his*/SeVc at m.o.i. 3, and the cells were collected 18, 24, 30, and 42 hours after the infection, followed by staining with an anti-MHC class I antibody, and expression levels of the MHC class I/peptide complex on the cell surface were analyzed by flow cytometry (A). Values in panel (B) indicate M.F.I. (Mean Fluorescent Intensity). In addition, the dotted line indicates cells infected with an isotype control SeV, whereas the solid line indicates those infected with wild type SeV (same symbols were used in Figs. 8, 9, and 11 below). Expression of a new MHC class I/peptide complex on the cell membrane surface was inhibited due to the expression of the TAP inhibitory protein, which led to a decrease in MHC class I/peptide complex levels over time. However, the decreased levels were low, since the existing MHC class I/peptide complexes were not affected.
Fig. 8 shows the effects of acid treatment on the existing MHC class I/peptide complex. One, 3, and 6 hours after the acid treatment, MT-2 cells were stained with the anti-MHC class I antibody, and then expression levels of the MHC class I/peptide complex on the cell surface were analyzed by flow cytometry (A). The MHC class I/peptide complex levels were decreased due to the acid treatment to remove the peptide from the MHC class I/peptide complex on the cell membrane surface (B). After that, nearly the same expression levels of the MHC class I/peptide complex on the cell membrane surface before the acid treatment were recovered within about 6 hours.

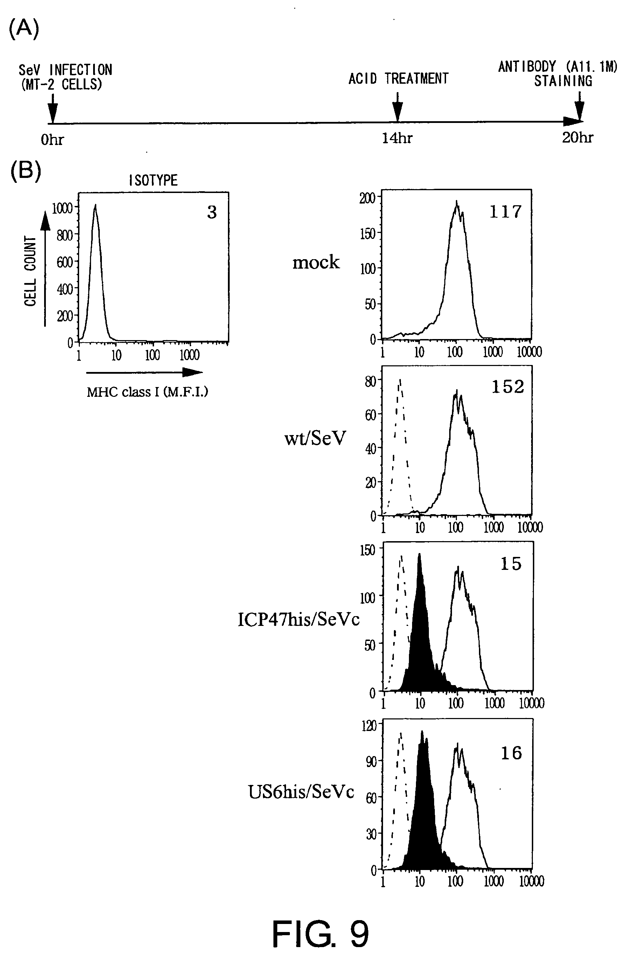

Fig. 9 shows the effects of acid treatment and the introduction of an SeV vector expressing the TAP inhibitory protein on the suppression of expression of the MHC class I/peptide complex. MT-2 cells were infected with ICP47*his*/SeVc or US6*his*/SeVc at m.o.i. 3, treated with the acid 14 hours later, stained with an anti-HLA-A24 antibody 6 hours after the acid treatment. Expression levels of the MHC class I/peptide complex on the cell membrane surface were then analyzed by flow cytometry (A). As a result of the removal of the existing MHC class I/peptide complex by the acid treatment, coupled with suppression of the expression of a new MHC class I/peptide complex on the cell surface by the TAP inhibitory protein, the expression levels of the MHC class I/peptide complex on the cell surface remained low even 6 hours after the acid treatment (B).

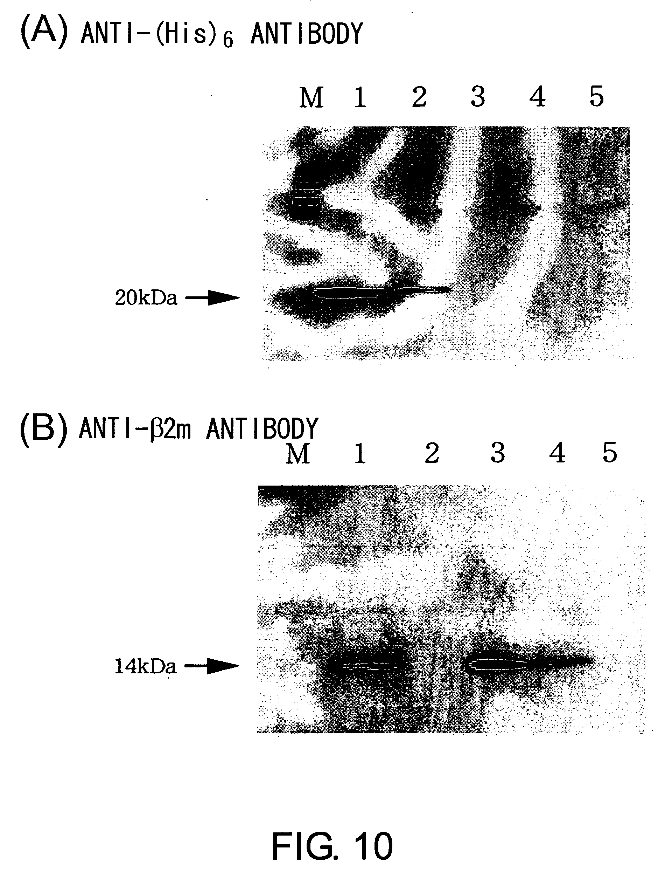

Fig. 10 depicts photographs of the epitope-linked-β2m and the TAP inhibitory protein co-expressed by the SeV vectors introduced into the cells. The two proteins were detected with an anti-(His)₆ antibody (A) and anti-β2m antibody (B). These photographs show that both e/Nef138-β2m and US6his are expressed in the cells infected with <e/Nef138-β2m+US6*his*>/SeV Lane M, size marker; lane 1, <e/Nef138-β2m+US6*his*>/SeV; lane 2, US6*his*/SeVc; lane 3, e/Nef138-β2m/SeVb; lane 4, e/Nef138-β2m/SeVc; and lane 5, wt (wild type) SeV.

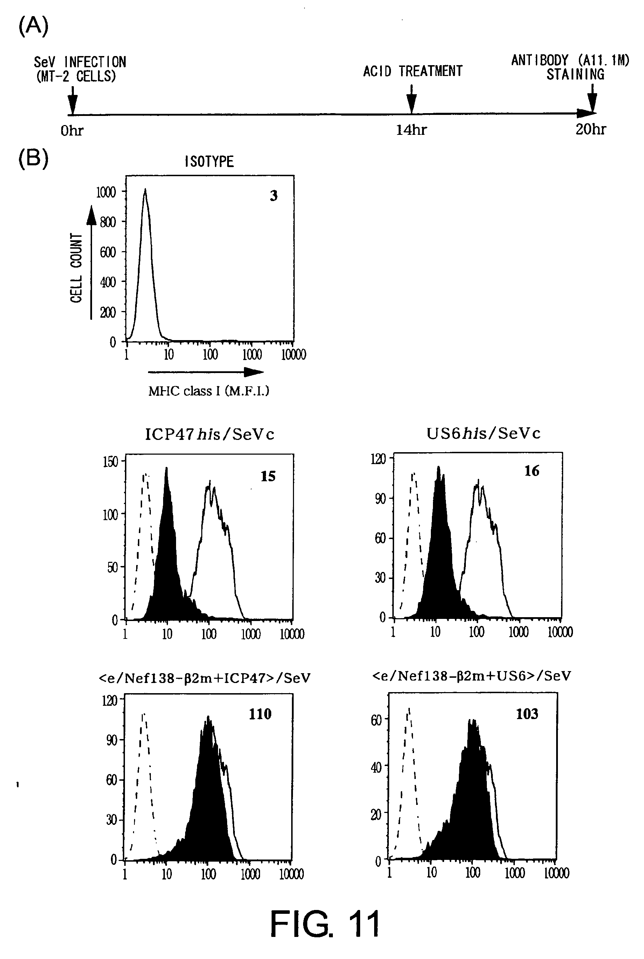

Fig. 11 shows the expression of the epitope-linked-β2m-bound MHC class I/peptide complex resulting from the introduction of the SeV vector co-expressing the epitope-linked-β2m and the TAP inhibitory protein. (A) shows the procedure for the assay, and (B) shows the results. While the MHC class I/peptide complex levels on the cell surface remained low even 6 hours after the acid treatment in the cells in which the SeV vector expressing ICP47his or US6his (<e/Nef138-β2m+ICP47*his*>/SeV or <e/Nef138-β2m+US6*his*>/SeV) alone was introduced, the MHC class I/peptide complex levels on the cell surface before the acid treatment were recovered 6 hours after the acid treatment in the cells in which the SeV vector co-expressing e/Nef138-β2m and ICP47his or US6his was introduced.

### Best Mode for Carrying out the Invention

The present invention is specifically illustrated below with reference to the Examples, but is not to be construed as being limited thereto. Furthermore, all references cited throughout this specification are incorporated by reference.

### [Example 1] Isolation of the HLA-A*2402 gene and the human β2m gene

The HLA-A*2402 gene, a human MHC class I gene, and the human β2m gene were cloned from messenger RNAs (mRNA) in peripheral blood mononuclear cells (PBMC) taken from a normal healthy subject carrying the HLA-A24. Micro-FastTrack Kit (Invitrogen) was used for the separation of mRNA and AMV-RT First-strand cDNA synthesis kit (LIFE SCIENCE) was used for the synthesis of cDNA.

Using the obtained cDNA as a template, PCR was conducted using primer sets HLA-5P2 and HLA-3B, and b2m-5'and b2m-3' for the HLA-A*2402 and β2m genes, respectively.

HLA-5P2, 5'-GGGCGGATCCGGACTCAGAATCTCCCCAGACGCCGAG-3' (SEQ
ID NO: 7)

HLA-3B, 5'-CCGCCTCGAGCTGGGGAGGAAACAGGTCAGCATGGGAAC-3'
(SEQ ID NO: 8)

b2m-5', 5'-GGCACGAGCCGAGATGTCTCGCTCCGTGGC-3' (SEQ ID NO: 9)

b2m-3', 5'-AATTTGGAATTCATCCAATCCAAATGCGGC-3' (SEQ ID NO: 10)

PCR was carried out by 35 cycles of 94°C for 30 sec, 58°C for 30 sec, and 72°C for 1 min, followed by extension reaction at 72° for 7 min. PCR was carried out using Ex Taq (TaKaRa). PCR products thus obtained were cloned using the pGEM-T vector system (Promega) (dubbed A*2402/pGEM and β2m/pGEM, respectively), and their nucleotide sequences were confirmed by sequence reactions. The sequence reactions were carried out using dye terminator chemistry (Big-Dye terminator cycle sequencing Ready Reaction Kit; Applied Biosystems) by electrophoresis onAB1-377 DNA Sequencer.

### [Example 2] Construction of epitope-linked-β2m expression vector

The following steps were performed to construct the plasmid (e/β2m/pSeVb) that encodes an SeV vector expressing an epitope-linked-β2m. The insertion of the sequence for each epitope and linkers downstream of the β2m signal sequence, and the attachment of E and S signals of Sendai virus and *Not*I recognition site were performed by PCR (Fig.1). The amino acid sequence of the linker (GGGSGGGSGGGS/SEQ ID NO: 11) was designed to have 3 repeated sequences of GGGS (SEQ ID NO: 1).
Primers used were as follows:
e/b2m-a1, 5' e(Nef)-a2, 5' 
-TCTGGCCTGGAGGCTAGATATCCACTGACCTTTGGATGGTGCTTCGGAGGAGGTGGC
GGGTCC-3' (SEQ ID NO: 13)
 e(Env)-a2, 5' 
-TCTGGCCTGGAGGCTAGATACCTAAGGGATCAACAGCTCCTAGGGATTGGAGGTGGC
GGGTCC-3' (SEQ ID NO: 14)
 e/b2m-a3, 5' 
-TGCGGCCGCCGTACGGCCGAGATGTCTCGCTCCGTGGCCTTAGCTGTGCTCGCGCTA
CTCTCTCTTTCTGGCCTGGAGGCT-3' (SEQ ID NO: 15)
 b2m-d, 5'

-TTGCGGCCGCGATGAACTTTCACCCTAAGTTTTCTTACTACGGCGTACGTTACATGT
CTCGATCCCACTT-3' (SEQ ID NO: 16)

PCR was carried out using β2m/pGEM as a template and a set of primers, e/b2m-a1 and e/b2m-d, by 15 cycles of 94°C for 1 min, 48°C for 1 min, and 72°C for 1 min, followed by an extension reaction at 72°C for 7 min. The obtained PCR product was used as a template for further PCR using e(Nef)-a2 or e(Env)-a2 and b2m-d under the same conditions, followed by another PCR using these products as a templates and e/b2m-a3 and b2m-d under the same conditions to obtain e/Nef138-β2m and e/Env584-β2m fragments, respectively. Each of these fragments was cloned using pGEM-T vector system (Promega) and their sequences were confirmed by sequence reactions. Then, each vector containing either of the fragments was digested with *Not*I and inserted into the *Not*I-digested site of pSeV18⁺b(+), and the nucleotide sequences were confirmed again. Thus, e/Nef138-β2m/pSeVb and e/Env584-β2m/pSeVb were obtained (generically dubbed as "e/β2m/pSeVb"). On the other hand, Sendai virus β2m/pSeVb expressing native β2m alone was also constructed similarly as described above, using b2m-a (5'-TGCGGCCGCCGTACGGCCGAGATGTCTCGCTCCGTGGCCTTA-3' (SEQ ID NO: 17) and b2m-d. The amino acid sequences of the Nef epitope (Nef138-10) and the Env epitope (Env584-11) are shown in SEQ ID NOs: 21 and 23, respectively.

### [Example 3] Construction of vector expressing a membrane-bound form of an MHC class I heavy chain

The addition of the E and S signals of Sendai virus and the *Not*I site was performed by PCR (Fig. 2).
Primers used were as follows: 
A24-a#, 5'-TGCGGCCGCCGTACGCCGAGGATGGCCGTCATGGCGCCCCG-3' (SEQ ID
NO: 28)
 A24-d4,

5'-TTGCGGCCGCGATGAACTTTCACCCTAAGTTTTTCTTACTACGGCGTACGTCACACT
TTACAAGCTGTGAG-3' (SEQ ID NO: 29)

PCR was carried out using A*2402/pGEM as a template and a set of primers A24-a# and A24-d4 by 15 cycles of 94°C for 1 min, 48°C for 1 min, and 72°C for 1 min, followed by an extension reaction at 72°C for 7 min, yielding A24full fragment. A24full/pSeVb was obtained similarly to the generation of e/β2m/pSeVb.

### [Example 4] Construction of an expression vector for ICP47 and co-expression vector for both ICP47 and epitope

The ICP47 (US12) gene derived from Herpes simplex virus type 1 (HSV-1) has been cloned into the plasmid US12/pGEX-5X-2 as described previously (McGeoch, D.J., Nucleic Acids Res. 14(4): 1727-1745 (1986)). PCR was used for the attachment of the histidine tag (his), the E and S signals of Sendai virus, and a *Not*I site to the ICP47 gene fragment (Fig. 3).
Primers used were as follows:
ICP-Esn, 
5'-TGCGGCCGCAGTAAGAAAAACTTAGGGTCAAACGTACGGCCGAGATGTCGTGGGC
CCTGGAAAT-3' (SEQ ID NO: 34)
 ICPhis-R,

5'-TTGCGGCCGCTATCAATGGTGGTGATGGTGGTGAGCTCCACGGGTTACCGGATTAC-
3' (SEQ ID NO: 35)

PCR was carried out using US12/pGEX-5X-2 as a template by 15 cycles of 94°C for 1 min, 48°C for 1 min, and 72°C for 1 min, followed by an extension reaction at 72°C for 7 min, yielding ICP47his fragment. ICP47*his*/pSeVc was obtained similarly to the generation of e/β2m/pSeV by introducing the ICP47his fragment into pSeV18⁺c(+) (Kato, A. *et al*., J. Virol. 73(11): 9237-9246 (1999)).

e/Nef138-β2m/pSeVb and ICP47*his*/pSeVc were digested with *Kpn*I and *Spn*I (New England BioLab) to yield an e/Nef138-β2m-containing fragment (4 kb) of e/Nef138-β2m/pSeVb and an ICP47*his*-containing fragment (15 kb) of ICP47*his*/pSeVc, respectively, followed by their ligation using the Ligation Kit Ver.2 (TaKaRa), yielding <e/Nef138-p2m+ICP47*his*>/pSeV (Fig. 4).

### [Example 5] Construction of an expression vector for US6 and a co-expression vector for US6 and epitope

The human cytomegalovirus (CMV) strain AD 169 solution has been described previously (Chen, Z. *et al*., Virology 258(2): 240-248 (1999)). PCR was carried out using a DNA extract from CMV strain AD 169 solution as a template (Fig.3).
Primers used were as follows: US6-a, 5'-TGCGGCCGCCACTCCTTCACTATGGATCTCTTG-3' (SEQ ID NO: 36) US6his-d1, 
5'-CTACGGCGTACGTCAATGGTGGTGATGGTGGTGAGCTCCGGAGCCACAACGTCGA
AT-3' (SEQ ID NO: 37)
 US6his-d2,

5'-TTGCGGCCGCGATGAACTTTCACCCTAAGTTTTTCTTACTACGGCGTACGTCA-3'
(SEQ ID NO: 38)

PCR was carried out using a set of primers US6-a and US6his-d1 by 15 cycles of 94°C for 1 min, 48°C for 1 min, and 72°C for 1 min, followed by an extension reaction at 72°C for 7 min, to yield a US6his fragment. US6*his*/pSeVc was obtained similarly to the generation of e/β2m/pSeVb.

In a similar manner to that of the construction of <e/Nef138-β2m+ICP47*his*>/pSeV, <e/Nef138-β2m+US6*his*>/pSeV was obtained from recombination of e/Nef138-β2m/pSeVb and US6*his*/pSeVc (Fig. 4).

### [Example 6] Reconstitution and infection of Sendai virus vector

pSeV18⁺b(+), pGEM-L, pGEM-P, pGEM-N, and vTF7-3 have been described previously (Hasan, M.K. *et al*., J. Gen. Virol. 78: 2813-2820 (1997); Kato, A. *et al*., EMBO. J. 16: 578-587 (1997); Yu, D. *et al*., Genes Cells, 2: 457-466 (1997)). Reconstitution of Sendai virus vector was performed according to the methods described in the above references. The Sendai virus vectors e/Nef138-β2m/SeVb and e/Env584-β2m/SeVb (collectively called as e/β2m/SeVb) were generated from e/Nef138-β2m/pSeVb and e/Env584-β2m/pSeVb, respectively. Furthermore, the Sendai virus vector A24full/SeVb was generated from A24full/pSeVb.

The Sendai virus vectors ICP47*his*/SeVc and <e/Nef138-β2m+ICP47*his*>/SeV were obtained from ICP47*his*/pSeVc and <e/Nef138-β2m+ICP47*his*>/pSeV, respectively. The Sendai virus vectors US6*his*/SeVc and <e/Nef138-β2m+US6*his*>/SeV were obtained from US6*his*/pSeVc and <e/Nef138-β2m+US6*his*>/pSeV, respectively.

Simian kidney cell lines CV-1 and LLCMK2 were cultured in MEM (Sigma) supplemented with 10% fetal calf serum (FCS), 100 U/ml streptomycin, and 100 U/ml penicillin (Life Technologies) (M10). Unless otherwise stated, CV-1 cells were infected with recombinant Sendai virus at each indicated m.o.i and cultured in a serum-free medium for 3 days in following infection with Sendai virus.

### [Example 7] Recovery and quantification of epitope-linked-β2m (e/β2m)

The culture supernatant of CV-1 cells infected with e/β2m/SeVb or β2m/SeVb (no epitope) was centrifuged at 40,000x g to remove Sendai virus particles and the supernatant was collected.

Sandwich enzyme-linked immunosorbent assay (ELISA) was used for the quantification of e/β2m in the culture supernatant. 2.5 µg/ml anti-human β2 microglobulin monoclonal antibody (DAKO) was used as a capture antibody, and 500 ng/ml peroxidase-labeled anti-human β2 microglobulin monoclonal antibody (DAKO) as a detector antibody. TMB peroxidase color development kit (BIO-RAD) was used for color development.

Purified human β2 microglobulin (Biogenesis) was used as the standard sample.

### [Example 8] Establishment of an Nef 138-10-specific CTL clone

PBMC of HIV-1-infected subject carrying HLA-A*2402 was cultured overnight at 3x 10⁵/96-well in 100 µl/well R10 medium. On the next day, stimulator cells (autologous PHA-blast activated in RPMI1640 (Sigma) supplemented with 0.2 µg/ml PHA (Sigma) and 10% Lymphocult-T (Biotest) (R10) overnight, irradiated at 3000 rad, and pulsed with 10 µM Nef138-10 for 1 hour) were added and cultured for 2 weeks in the presence of 1 µg/ml anti-human CD28. The cells were further stimulated with stimulator cells (autologous Epstein-Barr virus transformed B cell line (B-LCL) irradiated at 10,000 rad and pulsed with 10 µM Nef138-10) ever 2 weeks. After 2 to 4 stimulations, when CTL activity was confirmed, the cells were cloned.

The cloning was carried out by incubating the cells at 0.8 cells/well with 1x 10⁵ cells/well stimulator cells (autologous B-LCL irradiated at 10,000 rad and pulsed with 10 µM Nef138-10) and 5x 10⁴ cells/well feeder cells (PBMC from normal healthy subject irradiated at 3,000 rad) in the presence of 10% Lymphocult-T and 2.5% PHA-sup (culture supernatant of PBMC (3x 10⁶/ml) from normal healthy subject stimulated with 0.2 µg/ml PHA for 48 hours) for 3 to 4 weeks.

### [Example 9] Recognition by CTL of SeV-introduced cells forming a membrane-bound form of MHC class I/peptide complex

CTL ⁵¹Cr release assay was performed as follows. Human CD4⁺ T lymphocyte cell line H9 was cultured in RPMI1640 (Sigma) supplemented with 10% FCS, 100 U/ml streptomycin, and 100 U/ml penicillin (R10). The cells (2x 10³ cells/well; target cells) were labeled with 100 µCi of Na₂⁵¹CrO₄ for 2 hours, washed three times with R10, and pulsed with 10 µM peptide (Nef138-10) for an hour. When SeV vector-introduced cells were used as target cells, the cells were infected with SeV vectors in combinations as indicated in Fig. 5 at m.o.i 10:2, 17 hours before the labeling (namely, 20 hours before the addition of the effector cells). The cells of Example 8 were added as effector cells at each E:T ratio (effector cells:target cells). The cells were incubated for 4 hours at 37°C, and the amount of ⁵¹Cr released in the culture supernatant was determined using a ycounter. R10 and 4% Triton X-100/PBS were added instead of the effector cells for the determination of Spontaneous release and Maximum release, respectively.

Specific lysis (%) was calculated as (cpm of each sample - cpm of Spontaneous release)/(cpm of Maximum release - cpm of Spontaneous release) x 100.

Fig. 5 shows the results of recognition by CTL of the membrane-bound form of the MHC class I/peptide complexes. It was revealed that antigen-specific CTL activation could be detected in the cells co-infected with A24full/SeVb and e/Nef138-β2m/SeVb.

### [Example 10] Effect of epitope-linked-β2m recovered from SeV-infected cells

In order to prepare epitope-linked-β2m produced by SeV-infected cells, H9 cells were infected with e/Nef138-β2m/SeVb at m.o.i 2. Three days later, culture supernatant was collected and filtered, yielding a solution containing the epitope-linked-β2m. Similarly as in Example 9, CTL assay was carried out using H9 cells infected with A24full/SeVb as target cells to compare the effect of pulsing with peptides and that with the above-obtained epitope-linked-β2m-containing solution.

Fig. 6 shows the effects of epitope-linked-β2m. Antigen-specific CTL activity was detected upon the addition of the epitope-linked-β2m recovered from the culture supernatant of SeV-infected cells to the culture solution of the target cells.

### [Example 11] Down regulation of the MHC class I/peptide complex on the cell surface by ICP47 or US6

Human CD4⁺ T lymphocyte cell line H9 cells, MT-2 cells, and mouse hybridoma A11.1M (Foung, S.K.H., *et al*., Human Immunol. 15: 316-319 (1986)) cells were cultured in RPMI1640 (SIGMA) supplemented with 10% FCS, 100 U/ml streptomycin, and 100 U/ml penicillin (R10). CTL line and CTL clone induced from an HIV-1 patient were cultured in R10 medium containing 10% Lymphocult-T (Biotest).

Human CD4⁺ T lymphocyte cell line H9 cells were co-infected with ICP47*his*/pSeVc and US6*his*/pSeVc at m.o.i. 3, stained 18, 24, 30, and 42 hours later with an anti-MHC class I monoclonal antibody 3F10 labeled with FITC (Ancell), and then analyzed by flow cytometry. Namely, H9 cells were reacted with a 100-fold diluted anti-MHC class I antibody 3F10-FITC at 4°C for 20 min, followed by washing three times, and fixed with PBS (Phosphate Buffered Saline) containing 1 % paraformaldehyde. PBS containing 2% fetal calf serum and 0.1% sodium azide was used for staining and washing. Flow cytometry was carried out using FACS Caliber (Beckton Dickinson) and analyzed using FlowJo Ver. 3.3 (Treestar).

While a significant change in the expression of the MHC class I/peptide complexes was not detected when the cells were infected with wild type SeV as a control, the MHC class I/peptide complex levels decreased over time when the cells were infected with ICP47*his*/SeVc or US6*his*/SeVc, suggesting that expression of new MHC class I/peptide complexes on the membrane surface was suppressed (Fig. 7). However, no remarkable decrease was observed due to the presence of the existing MHC class I/peptide complexes.

### [Example 12] Effect of acid treatment on the existing MHC class I/peptide complexes

Since expression of new MHC class I/peptide complexes was suppressed by the inhibition of TAP activity, the effects of acid treatment on the existing MHC class I/peptide complexes was then examined.

Acid treatment to release peptides was carried out by treating MT-2 cells (HLA-A24+) with a 200 µl peptide stripping buffer (0.13 M citric acid (pH3), 66 mM Na₂HPO₄, 150 mM NaCl, 17 µg/ml Phenol Red) at 4°C for 1 min, followed by neutralization with 12 ml RPMI1640. Immediately after (0 hour), 1, 3, and 6 hours after the treatment, the cells were stained with a culture supernatant of an anti-HLA-A24 antibody-producing hybridoma A11.1M, followed by anti-mouse IgG antibody labeled with FITC (Immunotech), and analyzed by flow cytometry as described above (Fig. 8).

Peptides were shown to be removed from the MHC class I/peptide complexes on the membrane surface by the acid (pH 3) treatment, leading to a decrease in the MHC class I/peptide complex levels on the cell surface. Levels before the treatment were shown to be recovered 6 hours later.

### [Example 13] Decrease in the MHC class I/peptide complex levels on the cell surface by acid treatment and using the expression vector for the TAP inhibitory protein

Since the acid treatment was shown to be effective to decrease the levels of existing MHC class I/peptide complexes, the effects of the TAP inhibitory protein on the suppression of new MHC class I/peptide complexes on the cell membrane surface was then examined, by infection of the cells with the expression vector for the TAP inhibitory protein, followed by acid treatment of the cells after the TAP inhibitory protein was expressed sufficiently (14 hours after the infection) to remove the existing MHC class I/peptide complexes, and by analyzing the MHC class I/peptide complex levels on the cell membrane surface 6 hours after the acid treatment.

MT-2 cells were infected with ICP47*his*/SeVc or US6*his*/SeVc at m.o.i. 3, treated with the acid 14 hours later as described in Example 12, stained 6 hours after the treatment with the MHC class I/peptide complex (A11.1M) as described in Example 12, and analyzed by flow cytometry (Fig. 9). As a result, both ICP47 and US6 were shown to effectively suppress the expression of new MHC class I/peptide complexes on the cell surface. In addition, it was confirmed that ICP47 and US6 had nearly the same suppressive effect.

### [Example 14] Co-expression of the TAP inhibitory protein and epitope-linked-β2m by the co-expression vector for the TAP inhibitory protein and epitope-linked-β2m

Western blotting was used to analyze whether the vector expressing both the TAP inhibitory protein and epitope-linked-β2m constructed in Example 5 actually expresses the both proteins.

CV-1 cells were infected with SeV vectors expressing e/Nef138-β2m and US6*his*, separately, or co-expressing both, and with wild type SeV at m.o.i. 3, and the infected cells were collected 24 hours later. The collected infected cells were lysed with TNE Buffer (10 mM Tris-HCl (pH7.8), 1% NP40, 0.15 M NaCl, 1 mM EDTA, 10 µg/ml aprotinin) and the solubilized fraction was applied to SDS-PAGE, followed by Western blotting using an anti-(His)₆ antibody and an anti-β2m antibody to confirm the expression of the US6*his* and e/Nef138-β2m proteins (Fig. 10). After the samples were blotted onto PVDF membrane, followed by blocking using Block Ace (Dainippon Pharmaceutical Co., Ltd.), the membrane was reacted with the 1000-fold diluted anti-(His)₆ antibody, Penta-His Antibody (QIAGEN), or an anti-β2m antibody (Immunotech) at 4°C for one hour. After washing 4 times, the membrane was reacted with a 2000-fold diluted anti-mouse IgG antibody labeled with HRP (Roche) at room temperature for one hour, followed by washing 4 times, and allowed to color development using Lumi-Light plus substrate (Roche). Lumi Imager F1 (Boehringer Manheim) was used for the detection. TBS-T (20 mM Tris-HCl (pH7.5), 500 mM NaCl, 0.05% Tween-20) was used for the washing and 10-fold diluted Block Ace was used for the dilution of antibodies. As a result, in the cells infected with <e/Nef138-β2m+US6*his*>/SeV, expression of both US6his and e/Nef138-β2m was confirmed. Also, expression levels of US6*his* were almost the same as those in the cells infected with US6*his*/SeVc.

### [Example 15] Recovery of the MHC class I/peptide complex by the vector co-expressing the TAP inhibitory protein and epitope-linked-β2m after the acid treatment

From the results of Examples 13 and 14, it is expected that, in the acid-treated cells that were infected with the vector co-expressing the TAP inhibitory protein and epitope-linked-β2m, expression of the MHC class I/peptide complexes displaying endogenous protein-derived epitopes on the cell membrane surface will be suppressed, resulting in a high frequency of expression of MHC class I/peptide complex having an epitope-linked-β2m that does not require the TAP-mediated peptide transfer.

Accordingly, MT-2 cells were infected with ICP47*his*/SeVc or US6his/SeV, and <e/Nef138-β2m+ICP47*his*>/SeV or <e/Nef138-β2m+US6*his*>/SeV at m.o.i. 3, treated with the acid 14 hours later as described in Example 13, stained with the MHC class I/peptide complex (A11.1M) 6 hours later as in Example 13, and analyzed by flow cytometry (Fig. 11). Whereas expression of the MHC class I/peptide complexes on the cell membrane surface was suppressed in the cells infected with ICP47*his*/SeVc or US6*his*/SeVc, almost the same level of the MHC class I/peptide complexes as that in the cells infected with wild type SeV was recovered in the cell infected with <e/Nef138-β2m+ICP47*his*>/SeV or <e/Nef138-β2m+US6*his*>/SeV. This indicates that the MHC class I/peptide complex bound to epitope-linked-β2m is expressed in high density in the cells infected with the vector co-expressing the TAP inhibitory protein and epitope-liked-β2m.

### Industrial Applicability

The present invention enabled the efficient display of a desired exogenous epitope by suppressing the display of endogenous epitopes, when an epitope-linked MHC class I or β2m was expressed in mammalian cells. Thus, the present invention enables a high frequency of expression of the MHC class I/peptide complex displaying a certain specific (single) epitope on the cell surface. It is possible to effectively induce a desired antigen-specific cellular immune response by applying the method of the present invention to *introduce, in vivo* or *ex vivo,* a vector expressing an epitope-linked MHC class I or β2m. The present invention finds utility in the induction of protective immunity against infectious deceases, gene therapy in the immunotherapy for cancers, and the like.

## Claims

1. A method for enhancing exogenous epitope display on an MHC class I molecule, wherein said method comprises the steps of:
(a) inhibiting TAP activity in a cell; and
(b) expressing an epitope-fused MHC class I heavy chain or an epitope-fused β2m in said cell.

2. The method of claim 1, wherein said method further comprises the step of acid treatment.

3. The method of claim 1 or 2, wherein said step of inhibiting TAP activity in a cell comprises the step of contacting said cell with a protein having a TAP inhibitory activity, or the step of introducing a vector encoding said protein into said cell.

4. The method of claim 3, wherein said protein having said TAP inhibitory activity is US6 or ICP47.

5. The method of claim 3, wherein said vector is a mammalian cell-infecting virus vector.

6. The method of claim 5, wherein said vector is a Sendai virus vector.

7. A mammalian cell, wherein (i) expression of a TAP gene in said cell has been inhibited, said cell comprises a TAP inhibitor, or said cell bears a gene encoding said TAP inhibitor in an expressible way, and wherein (ii) said cell bears a gene encoding an epitope-fused MHC class I heavy chain or an epitope-fused-β2m in an expressible way.

8. A kit for displaying an epitope comprising: (i) a TAP inhibitor or a vector capable of expressing said inhibitor; and (ii) an epitope-fused MHC class I heavy chain or an epitope-fused β2m, or a vector capable of expressing said epitope-fused MHC class I heavy chain or said epitope-fused β2m.

9. A vector capable of expressing both (i) and (ii):
(i) a TAP inhibitor; and
(ii) an epitope-fused MHC class I heavy chain or an epitope-fused β2m.


SEQUENCE LISTING

<110> DNAVEC RESEARCH INC.

<120> Methods for enhancing exogenous epitope display on MHC class
      through inhibition of TAP activity

<130> D3-A0102P

<140>
<141>

<150> JP  2002-288394
<151> 2002-10-01

<160> 54

<170> PatentIn Ver. 2.0

<210> 1
<211> 4
<212> PRT
<213> Artificial Sequence

<220>
<223> Description of Artificial Sequence: artificially
      synthesized sequence

<400> 1
Gly Gly Gly Ser
  1

<210> 2
<211> 5
<212> PRT
<213> Artificial Sequence

<220>
<223> Description of Artificial Sequence: artificially
      synthesized sequence

<400> 2
Gly Gly Gly Gly Ser

1                5


<210> 3
<211> 10
<212> PRT
<213> Artificial Sequence

<220>
<223> Description of Artificial Sequence: artificially
      synthesized sequence

<400> 3
Gly Gly Pro Gly Gly Gly Ser Gly Gly Gly
1               5                   10


<210> 4
<211> 20
<212> PRT
<213> Artificial Sequence

<220>
<223> Description of Artificial Sequence: artificially
      synthesized sequence

<400> 4
Gly Gly Pro Gly Gly Gly Gly Ser Gly Gly Gly Gly Ser Gly Gly Gly
1               5                   10                  15

Ser Gly Gly Gly
            20


<210> 5
<211> 30
<212> PRT
<213> Artificial Sequence

<220>
<223> Description of Artificial Sequence: artificially
      synthesized sequence

<400> 5

Gly Gly Pro Gly Gly Gly Gly Ser Gly Gly Gly Gly Ser Gly Gly Gly
1               5                   10                  15

Gly Ser Gly Gly Gly Gly Ser Gly Gly Gly Ser Gly Gly Gly
            20                  25                  30

<210> 6
<211> 10
<212> PRT
<213> Artificial Sequence

<220>
<223> Description of Artificial Sequence: artificially
      synthesized sequence

<400> 6
Gly Gly Ser Gly Gly Gly Ala Ser Gly Gly
1               5                   10

<210> 7
<211> 37
<212> DNA
<213> Artificial Sequence

<220>
<223> Description of Artificial Sequence: artificially
      synthesized sequence

<400> 7
gggcggatcc ggactcagaa tctccccaga cgccgag                              37

<210> 8
<211> 39
<212> DNA
<213> Artificial Sequence

<220>
<223> Description of Artificial Sequence: artificially
      synthesized sequence

<400> 8

ccgcctcgag ctggggagga aacaggtcag catgggaac 39

<210> 9
<211> 30
<212> DNA
<213> Artificial Sequence

<220>
<223> Description of Artificial Sequence: artificially
      synthesized sequence

<400> 9
ggcacgagcc gagatgtctc gctccgtggc 30

<210> 10
<211> 30
<212> DNA
<213> Artificial Sequence

<220>
<223> Description of Artificial Sequence: artificially
      synthesized sequence

<400> 10
aatttggaat tcatccaatc caaatgcggc 30

<210> 11
<211> 12
<212> PRT
<213> Artificial Sequence

<220>
<223> Description of Artificial Sequence: artificially
      synthesized sequence

<400> 11
Gly Gly Gly Ser Gly Gly Gly Ser Gly Gly Gly Ser
 1               5                   10

<210> 12

<211> 60
<212> DNA
<213> Artificial Sequence

<220>
<223> Description of Artificial Sequence: artificially
      synthesized sequence

<400> 12
ggaggtggcg ggtccggagg tggttctggt ggaggttcga tccagcgtac tccaaagatt 60


<210> 13
<211> 63
<212> DNA
<213> Artificial Sequence

<220>
<223> Description of Artificial Sequence: artificially
      synthesized sequence

<400> 13
tctggcctgg aggctagata tccactgacc tttggatggt gcttcggagg aggtggcggg 60

tcc                                                               63


<210> 14
<211> 63
<212> DNA
<213> Artificial Sequence

<220>
<223> Description of Artificial Sequence: artificially
      synthesized sequence

<400> 14
tctggcctgg aggctagata cctaagggat caacagctcc tagggattgg aggtggcggg 60

tcc                                                               63


<210> 15
<211> 81

<212> DNA
<213> Artificial Sequence

<220>
<223> Description of Artificial Sequence: artificially
      synthesized sequence

<400> 15
tgcggccgcc gtacggccga gatgtctcgc tccgtggcct tagctgtgct cgcgctactc 60

tctctttctg gcctggaggc t                                           81


<210> 16
<211> 71
<212> DNA
<213> Artificial Sequence

<220>
<223> Description of Artificial Sequence: artificially
      synthesized sequence

<400> 16
ttgcggccgc gatgaacttt caccctaagt ttttcttact acggcgtacg ttacatgtct 60

cgatcccact t                                                      71


<210> 12
<211> 42
<212> DNA
<213> Artificial Sequence

<220>
<223> Description of Artificial Sequence: artificially
      synthesized sequence

<400> 17
tgcggccgcc gtacggccga gatgtctcgc tccgtggcct ta                    42


<210> 18
<211> 80
<212> DNA

<213> Artificial Sequence

<220>
<223> Description of Artificial Sequence: artificially
      synthesized sequence

<220>
<221> CDS
<222> (21)..(80)

<400> 18
gcggccgccg tacggccgag atg tct cgc tcc gtg gcc tta gct gtg ctc gcg 53
                      Met Ser Arg Ser Val Ala Leu Ala Val Leu Ala
                        1               5                   10

cta ctc tct ctt tct ggc ctg gag gct                                80
Leu Leu Ser Leu Ser Gly Leu Glu Ala
            15                  20


<210> 19
<211> 20
<212> PRT
<213> Artificial Sequence

<400> 19
Met Ser Arg Ser Val Ala Leu Ala Val Leu Ala Leu Leu Ser Leu Ser
  1               5                  10                  15

Gly Leu Glu Ala
            20


<210> 20
<211> 30
<212> DNA
<213> Artificial Sequence

<220>
<223> Description of Artificial Sequence: artificially
      synthesized sequence

<220>
<221> CDS

<222> (1)..(30)

<400> 20
aga tat cca ctg acc ttt gga tgg tgc ttc                                30
Arg Tyr Pro Leu Thr Phe Gly Trp Cys Phe
  1               5                  10


<210> 21
<211> 10
<212> PRT
<213> Artificial Sequence

<400> 21
Arg Tyr Pro Leu Thr Phe Gly Trp Cys Phe
  1               5                  10


<210> 22
<211> 33
<212> DNA
<213> Artificial Sequence

<220>
<223> Description of Artificial Sequence: artificially
      synthesized sequence

<220>
<221> CDS
<222> (1)..(33)

<400> 22
aga tac cta agg gat caa cag ctc cta ggg att                            33
Arg Tyr Leu Arg Asp Gln Gln Leu Leu Gly Ile
  1               5                  10


<210> 23
<211> 11
<212> PRT
<213> Artificial Sequence

<400> 23
Arg Tyr Leu Arg Asp Gln Gln Leu Leu Gly Ile

1                   5                   10


<210> 24
<211> 60
<212> DNA
<213> Artificial Sequence

<220>
<223> Description of Artificial Sequence: artificially
      synthesized sequence

<220>
<221> CDS
<222> (1)..(60)

<400> 24
gga ggt ggc ggg tcc gga ggt ggt tct ggt gga ggt tcg atc cag cgt    48
Gly Gly Gly Gly Ser Gly Gly Gly Ser Gly Gly Gly Ser Ile Gln Arg
  1               5                  10                  15

act cca aag att                                                    60
Thr Pro Lys Ile
             20


<210> 25
<211> 20
<212> PRT
<213> Artificial Sequence

<400> 25
Gly Gly Gly Gly Ser Gly Gly Gly Ser Gly Gly Gly Ser Ile Gln Arg
  1               5                  10                  15

Thr Pro Lys Ile
             20


<210> 26
<211> 69
<212> DNA
<213> Artificial Sequence

<220>
<223> Description of Artificial Sequence: artificially
      synthesized sequence

<220>
<221> CDS
<222> (1)..(18)

<400> 26
aag tgg gat cga gac atg taacgtacgc cgtagtaaga aaaacttagg          48
Lys Trp Asp Arg Asp Met
  1               5

gtgaaagttc atcgcggccg c                                           69


<210> 27
<211> 6
<212> PRT
<213> Artificial Sequence

<400> 27
Lys Trp Asp Arg Asp Met
  1               5


<210> 28
<211> 41
<212> DNA
<213> Artificial Sequence

<220>
<223> Description of Artificial Sequence: artificially
      synthesized sequence

<400> 28
tgcggccgcc gtacgccgag gatggccgtc atggcgcccc g                     41


<210> 29
<211> 71
<212> DNA
<213> Artificial Sequence

<220>
<223> Description of Artificial Sequence: artificially
      synthesized sequence

<400> 29
ttgcggccgc gatgaacttt caccctaagt ttttcttact acggcgtacg tcacacttta 60

caagctgtga g                                                      71


<210> 30
<211> 38
<212> DNA
<213> Artificial Sequence

<220>
<223> Description of Artificial Sequence: artificially
      synthesized sequence

<220>
<221> CDS
<222> (21)..(38)

<400> 30
gcggccgccg tacgccgagg atg gcc gtc atg gcg ccc                    38
                      Met Ala Val Met Ala Pro
                        1               5


<210> 31
<211> 6
<212> PRT
<213> Artificial Sequence

<400> 31
Met Ala Val Met Ala Pro
  1               5


<210> 32
<211> 69
<212> DNA
<213> Artificial Sequence

<220>
<223> Description of Artificial Sequence: artificially
      synthesized sequence

<220>
<221> CDS
<222> (1)..(18)

<400> 32
ctc aca gct tgt aaa gtg tgacgtacgc cgtagtaaga aaaacttagg          48
Leu Thr Ala Cys Lys Val
  1               5

gtgaaagttc atcgcggccg c                                           69


<210> 33
<211> 6
<212> PRT
<213> Artificial Sequence

<400> 33
Leu Thr Ala Cys Lys Val
  1               5


<210> 34
<211> 64
<212> DNA
<213> Artificial Sequence

<220>
<223> Description of Artificial Sequence: artificially
      synthesized sequence

<400> 34
tgcggccgca gtaagaaaaa cttagggtca aacgtacggc cgagatgtcg tgggccctgg 60

aaat                                                              64


<210> 35
<211> 56
<212> DNA

<213> Artificial Sequence

<220>
<223> Description of Artificial Sequence: artificially
      synthesized sequence

<400> 35
ttgcggccgc tatcaatggt ggtgatggtg gtgagctcca cgggttaccg gattac      56


<210> 36
<211> 33
<212> DNA
<213> Artificial Sequence

<220>
<223> Description of Artificial Sequence: artificially
      synthesized sequence

<400> 36
tgcggccgcc actccttcac tatggatctc ttg                               33


<210> 37
<211> 57
<212> DNA
<213> Artificial Sequence

<220>
<223> Description of Artificial Sequence: artificially
      synthesized sequence

<400> 37
ctacggcgta cgtcaatggt ggtgatggtg gtgagctccg gagccacaac gtcgaat     57


<210> 38
<211> 53
<212> DNA
<213> Artificial Sequence

<220>
<223> Description of Artificial Sequence: artificially
      synthesized sequence

<400> 38
ttgcggccgc gatgaacttt caccctaagt ttttcttact acggcgtacg tca          53


<210> 39
<211> 60
<212> DNA
<213> Artificial Sequence

<220>
<223> Description of Artificial Sequence: artificially
      synthesized sequence

<220>
<221> CDS
<222> (43)..(60)

<400> 39
gcggccgcag taagaaaaac ttagggtcaa acgtacggcc gag atg tcg tgg gcc   54
                                                Met Ser Trp Ala
                                                 1

ctg gaa                                                           60
Leu Glu
5


<210> 40
<211> 6
<212> PRT
<213> Artificial Sequence

<400> 40
Met Ser Trp Ala Leu Glu
 1               5


<210> 41
<211> 55
<212> DNA
<213> Artificial Sequence

<220>

<223> Description of Artificial Sequence: artificially
      synthesized sequence

<220>
<221> CDS
<222> (1)..(42)

<400> 41
cgt aat ccg gta acc cgt gga gct cac cac cat cac cac cat          42
Arg Asn Pro Val Thr Arg Gly Ala His His His His His His
  1               5                   10

tgatagcggc cgc                                                   55


<210> 42
<211> 14
<212> PRT
<213> Artificial Sequence

<400> 42
Arg Asn Pro Val Thr Arg Gly Ala His His His His His His
  1               5                   10


<210> 43
<211> 58
<212> DNA
<213> Artificial Sequence

<220>
<223> Description of Artificial Sequence: artificially
      synthesized sequence

<220>
<221> CDS
<222> (41)..(58)

<400> 43
gcggccgcag taagaaaaac ttagggtcaa agccttcact atg gat ctc ttg att   55
                                            Met Asp Leu Leu Ile
                                              1               5

cgt                                                              58

Arg

<210> 44
<211> 6
<212> PRT
<213> Artificial Sequence

<400> 44
Met Asp Leu Leu Ile Arg
1               5

<210> 45
<211> 55
<212> DNA
<213> Artificial Sequence

<220>
<223> Description of Artificial Sequence: artificially
      synthesized sequence

<220>
<221> CDS
<222> (1)..(42)

<400> 45
att cga cgt tgt ggc tcc gga gct cac cac cat cac cac cat          42
Ile Arg Arg Cys Gly Ser Gly Ala His His His His His His
1               5                   10

tgatagcggc cgc                                                   55

<210> 46
<211> 14
<212> PRT
<213> Artificial Sequence

<400> 46
Ile Arg Arg Cys Gly Ser Gly Ala His His His His His His
1               5                   10

<210> 47
<211> 2960
<212> DNA
<213> Homo sapiens

<220>
<221> CDS
<222> (165)..(2588)
<223>

<400> 47
ggcacgaggg tgtgcgtgat ggagaaaatt gggcaccagg gctgctcccg agattctcag 60

atctgatttc cacgcttgct accaaaatag tctgggcagg ccacttttgg aagtaggcgt 120

tatctagtga gcaggcggcc gctttcgatt tcgctttccc ctaa atg gct gag ctt 176
Met Ala Glu Leu
1

ctc gcc agc gca gga tca gcc tgt tcc tgg gac ttt ccg aga gcc ccg 224
Leu Ala Ser Ala Gly Ser Ala Cys Ser Trp Asp Phe Pro Arg Ala Pro
5                   10                  15                  20

ccc tcg ttc cct ccc cca gcc gcc agt agg gga gga ctc ggc ggt acc 272
Pro Ser Phe Pro Pro Pro Ala Ala Ser Arg Gly Gly Leu Gly Gly Thr
                25                  30                  35

cgg agc ttc agg ccc cac cgg ggc gcg gag agt ccc agg ccc ggc cgg 320
Arg Ser Phe Arg Pro His Arg Gly Ala Glu Ser Pro Arg Pro Gly Arg
            40                  45                  50

gac cgg gac ggc gtc cga gtg cca atg gct agc tct agg tgt ccc gct 368
Asp Arg Asp Gly Val Arg Val Pro Met Ala Ser Ser Arg Cys Pro Ala
        55                  60                  65

ccc cgc ggg tgc cgc tgc ctc ccc gga gct tct ctc gca tgg ctg ggg 416
Pro Arg Gly Cys Arg Cys Leu Pro Gly Ala Ser Leu Ala Trp Leu Gly
    70                  75                  80

aca gta ctg cta ctt ctc gcc gac tgg gtg ctg ctc cgg acc gcg ctg 464
Thr Val Leu Leu Leu Leu Ala Asp Trp Val Leu Leu Arg Thr Ala Leu
85                  90                  95                  100

ccc cgc ata ttc tcc ctg ctg gtg ccc acc gcg ctg cca ctg ctc cgg 512

Pro Arg Ile Phe Ser Leu Leu Val Pro Thr Ala Leu Pro Leu Leu Arg
                105                 110                 115

gtc tgg gcg gtg ggc ctg agc cgc tgg gcc gtg ctc tgg ctg ggg gcc 560
Val Trp Ala Val Gly Leu Ser Arg Trp Ala Val Leu Trp Leu Gly Ala
            120                 125                 130

tgc ggg gtc ctc agg gca acg gtt ggc tcc aag agc gaa aac gca ggt 608
Cys Gly Val Leu Arg Ala Thr Val Gly Ser Lys Ser Glu Asn Ala Gly
        135                 140                 145

gcc cag ggc tgg ctg gct gct ttg aag cca tta gct gcg gca ctg ggc 656
Ala Gln Gly Trp Leu Ala Ala Leu Lys Pro Leu Ala Ala Ala Leu Gly
    150                 155                 160

ttg gcc ctg ccg gga ctt gcc ttg ttc cga gag ctg atc tca tgg gga 704
Leu Ala Leu Pro Gly Leu Ala Leu Phe Arg Glu Leu Ile Ser Trp Gly
165                 170                 175                 180

gcc ccc ggg tcc gcg gat agc acc agg cta ctg cac tgg gga agt cac 752
Ala Pro Gly Ser Ala Asp Ser Thr Arg Leu Leu His Trp Gly Ser His
                185                 190                 195

cct acc gcc ttc gtt gtc agt tat gca gcg gca ctg ccc gca gca gcc 800
Pro Thr Ala Phe Val Val Ser Tyr Ala Ala Ala Leu Pro Ala Ala Ala
            200                 205                 210

ctg tgg cac aaa ctc ggg agc ctc tgg gtg ccc ggc ggt cag ggc ggc 848
Leu Trp His Lys Leu Gly Ser Leu Trp Val Pro Gly Gly Gln Gly Gly
        215                 220                 225

tct gga aac cct gtg cgt cgg ctt cta ggc tgc ctg ggc tcg gag acg 896
Ser Gly Asn Pro Val Arg Arg Leu Leu Gly Cys Leu Gly Ser Glu Thr
    230                 235                 240

cgc cgc ctc tcg ctg ttc ctg gtc ctg gtg gtc ctc tcc tct ctt ggg 944
Arg Arg Leu Ser Leu Phe Leu Val Leu Val Val Leu Ser Ser Leu Gly
245                 250                 255                 260

gag atg gcc att cca ttc ttt acg ggc cgc ctc act gac tgg att cta 992
Glu Met Ala Ile Pro Phe Phe Thr Gly Arg Leu Thr Asp Trp Ile Leu
                265                 270                 275

caa gat ggc tca gcc gat acc ttc act cga aac tta act ctc atg tcc 1040

Gln Asp Gly Ser Ala Asp Thr Phe Thr Arg Asn Leu Thr Leu Met Ser
            280                 285                 290

att ctc acc ata gcc agt gca gtg ctg gag ttc gtg ggt gac ggg atc 1088
Ile Leu Thr Ile Ala Ser Ala Val Leu Glu Phe Val Gly Asp Gly Ile
        295                 300                 305

tat aac aac acc atg ggc cac gtg cac agc cac ttg cag gga gag gtg 1136
Tyr Asn Asn Thr Met Gly His Val His Ser His Leu Gln Gly Glu Val
    310                 315                 320

ttt ggg gct gtc ctg cgc cag gag acg gag ttt ttc caa cag aac cag 1184
Phe Gly Ala Val Leu Arg Gln Glu Thr Glu Phe Phe Gln Gln Asn Gln
325                 330                 335                 340

aca ggt aac atc atg tct cgg gta aca gag gac acg tcc acc ctg agt 1232
Thr Gly Asn Ile Met Ser Arg Val Thr Glu Asp Thr Ser Thr Leu Ser
                345                 350                 355

gat tct ctg agt gag aat ctg agc tta ttt ctg tgg tac ctg gtg cga 1280
Asp Ser Leu Ser Glu Asn Leu Ser Leu Phe Leu Trp Tyr Leu Val Arg
            360                 365                 370

ggc cta tgt ctc ttg ggg atc atg ctc tgg gga tca gtg tcc ctc acc 1328
Gly Leu Cys Leu Leu Gly Ile Met Leu Trp Gly Ser Val Ser Leu Thr
        375                 380                 385

atg gtc acc ctg atc acc ctg cct ctg ctt ttc ctt ctg ccc aag aag 1376
Met Val Thr Leu Ile Thr Leu Pro Leu Leu Phe Leu Leu Pro Lys Lys
    390                 395                 400

gtg gga aaa tgg tac cag ttg ctg gaa gtg cag gtg cgg gaa tct ctg 1424
Val Gly Lys Trp Tyr Gln Leu Leu Glu Val Gln Val Arg Glu Ser Leu
405                 410                 415                 420

gca aag tcc agc cag gtg gcc att gag gct ctg tcg gcc atg cct aca 1472
Ala Lys Ser Ser Gln Val Ala Ile Glu Ala Leu Ser Ala Met Pro Thr
                425                 430                 435

gtt cga agc ttt gcc aac gag gag ggc gaa gcc cag aag ttt agg gaa 1520
Val Arg Ser Phe Ala Asn Glu Glu Gly Glu Ala Gln Lys Phe Arg Glu
            440                 445                 450

aag ctg caa gaa ata aag aca ctc aac cag aag gag gct gtg gcc tat 1568

Lys Leu Gln Glu Ile Lys Thr Leu Asn Gln Lys Glu Ala Val Ala Tyr
        455                 460                 465

gca gtc aac tcc tgg acc act agt att tca ggt atg ctg ctg aaa gtg 1616
Ala Val Asn Ser Trp Thr Thr Ser Ile Ser Gly Met Leu Leu Lys Val
    470                 475                 480

gga atc ctc tac att ggt ggg cag ctg gtg acc agt ggg gct gta agc 1664
Gly Ile Leu Tyr Ile Gly Gly Gln Leu Val Thr Ser Gly Ala Val Ser
485                 490                 495                 500

agt ggg aac ctt gtc aca ttt gtt ctc tac cag atg cag ttc acc cag 1712
Ser Gly Asn Leu Val Thr Phe Val Leu Tyr Gln Met Gln Phe Thr Gln
                505                 510                 515

gct gtg gag gta ctg ctc tcc atc tac ccc aga gta cag aag gct gtg 1760
Ala Val Glu Val Leu Leu Ser Ile Tyr Pro Arg Val Gln Lys Ala Val
            520                 525                 530

ggc tcc tca gag aaa ata ttt gag tac ctg gac cgc acc cct cgc tgc 1808
Gly Ser Ser Glu Lys Ile Phe Glu Tyr Leu Asp Arg Thr Pro Arg Cys
        535                 540                 545

cca ccc agt ggt ctg ttg act ccc tta cac ttg gag ggc ctt gtc cag 1856
Pro Pro Ser Gly Leu Leu Thr Pro Leu His Leu Glu Gly Leu Val Gln
    550                 555                 560

ttc caa gat gtc tcc ttt gcc tac cca aac cgc cca gat gtc tta gtg 1904
Phe Gln Asp Val Ser Phe Ala Tyr Pro Asn Arg Pro Asp Val Leu Val
565                 570                 575                 580

cta cag ggg ctg aca ttc acc cta cgc cct ggc gag gtg acg gcg ctg 1952
Leu Gln Gly Leu Thr Phe Thr Leu Arg Pro Gly Glu Val Thr Ala Leu
                585                 590                 595

gtg gga ccc aat ggg tct ggg aag agc aca gtg gct gcc ctg ctg cag 2000
Val Gly Pro Asn Gly Ser Gly Lys Ser Thr Val Ala Ala Leu Leu Gln
            600                 605                 610

aat ctg tac cag ccc acc ggg gga cag ctg ctg ttg gat ggg aag ccc 2048
Asn Leu Tyr Gln Pro Thr Gly Gly Gln Leu Leu Leu Asp Gly Lys Pro
        615                 620                 625

ctt ccc caa tat gag cac cgc tac ctg cac agg cag gtg gct gca gtg 2096

Leu Pro Gln Tyr Glu His Arg Tyr Leu His Arg Gln Val Ala Ala Val
    630                 635                 640

gga caa gag cca cag gta ttt gga aga agt ctt caa gaa aat att gcc 2144
Gly Gln Glu Pro Gln Val Phe Gly Arg Ser Leu Gln Glu Asn Ile Ala
645                 650                 655                 660

tat ggc ctg acc cag aag cca act atg gag gaa atc aca gct gct gca 2192
Tyr Gly Leu Thr Gln Lys Pro Thr Met Glu Glu Ile Thr Ala Ala Ala
                665                 670                 675

gta aag tct ggg gcc cat agt ttc atc tct gga ctc cct cag ggc tat 2240
Val Lys Ser Gly Ala His Ser Phe Ile Ser Gly Leu Pro Gln Gly Tyr
            680                 685                 690

gac aca gag gta gac gag gct ggg agc cag ctg tca ggg ggt cag cga 2288
Asp Thr Glu Val Asp Glu Ala Gly Ser Gln Leu Ser Gly Gly Gln Arg
        695                 700                 705

cag gca gtg gcg ttg gcc cga gca ttg atc cgg aaa ccg tgt gta ctt 2336
Gln Ala Val Ala Leu Ala Arg Ala Leu Ile Arg Lys Pro Cys Val Leu
    710                 715                 720

atc ctg gat gat gcc acc agt gcc ctg gat gca aac agc cag tta cag 2384
Ile Leu Asp Asp Ala Thr Ser Ala Leu Asp Ala Asn Ser Gln Leu Gln
725                 730                 735                 740

gtg gag cag ctc ctg tac gaa agc cct gag cgg tac tcc cgc tca gtg 2432
Val Glu Gln Leu Leu Tyr Glu Ser Pro Glu Arg Tyr Ser Arg Ser Val
                745                 750                 755

ctt ctc atc acc cag cac ctc agc ctg gtg gag cag gct gac cac atc 2480
Leu Leu Ile Thr Gln His Leu Ser Leu Val Glu Gln Ala Asp His Ile
            760                 765                 770

ctc ttt ctg gaa gga ggc gct atc cgg gag ggg gga acc cac cag cag 2528
Leu Phe Leu Glu Gly Gly Ala Ile Arg Glu Gly Gly Thr His Gln Gln
        775                 780                 785

ctc atg gag aaa aag ggg tgc tac tgg gcc atg gtg cag gct cct gca 2576
Leu Met Glu Lys Lys Gly Cys Tyr Trp Ala Met Val Gln Ala Pro Ala
    790                 795                 800

gat gct cca gaa tgaaagcctt ctcagacctg cgcactccat ctccctccct 2628

Asp Ala Pro Glu
805

tttcttctct ctgtggtgga gaaccacagc tgcagagtag gcagctgcct ccaggatgag 2688

ttacttgaaa tttgccttga gtgtgttacc tcctttccaa gctcctcgtg ataatgcaga 2748

cttcctggag tacaaacaca ggatttgtaa ttccttactg taacggagtt tagagccagg 2808

gctgatgctt tggtgtggcc agcactctga aactgagaaa tgttcagaat gtacggaaag 2868

atgatcagct attttcaaca taactgaagg catatgctgg cccataaaca ccctgtaggt 2928

tcttgatatt tataataaaa ttggtgtttt gt 2960

<210> 48
<211> 808
<212> PRT
<213> Homo sapiens

<400> 48

Met Ala Glu Leu Leu Ala Ser Ala Gly Ser Ala Cys Ser Trp Asp Phe
1               5                   10                  15

Pro Arg Ala Pro Pro Ser Phe Pro Pro Pro Ala Ala Ser Arg Gly Gly
            20                  25                  30

Leu Gly Gly Thr Arg Ser Phe Arg Pro His Arg Gly Ala Glu Ser Pro
        35                  40                  45

Arg Pro Gly Arg Asp Arg Asp Gly Val Arg Val Pro Met Ala Ser Ser
    50                  55                  60

Arg Cys Pro Ala Pro Arg Gly Cys Arg Cys Leu Pro Gly Ala Ser Leu
65                  70                  75                  80

Ala Trp Leu Gly Thr Val Leu Leu Leu Leu Ala Asp Trp Val Leu Leu
                85                  90                  95

Arg Thr Ala Leu Pro Arg Ile Phe Ser Leu Leu Val Pro Thr Ala Leu
            100                 105                 110

Pro Leu Leu Arg Val Trp Ala Val Gly Leu Ser Arg Trp Ala Val Leu
        115                 120                 125

Trp Leu Gly Ala Cys Gly Val Leu Arg Ala Thr Val Gly Ser Lys Ser
    130                 135                 140

Glu Asn Ala Gly Ala Gln Gly Trp Leu Ala Ala Leu Lys Pro Leu Ala
145                 150                 155                 160

Ala Ala Leu Gly Leu Ala Leu Pro Gly Leu Ala Leu Phe Arg Glu Leu
                165                 170                 175

Ile Ser Trp Gly Ala Pro Gly Ser Ala Asp Ser Thr Arg Leu Leu His
            180                 185                 190

Trp Gly Ser His Pro Thr Ala Phe Val Val Ser Tyr Ala Ala Ala Leu
        195                 200                 205

Pro Ala Ala Ala Leu Trp His Lys Leu Gly Ser Leu Trp Val Pro Gly
    210                 215                 220

Gly Gln Gly Gly Ser Gly Asn Pro Val Arg Arg Leu Leu Gly Cys Leu
225                 230                 235                 240

Gly Ser Glu Thr Arg Arg Leu Ser Leu Phe Leu Val Leu Val Val Leu
                245                 250                 255

Ser Ser Leu Gly Glu Met Ala Ile Pro Phe Phe Thr Gly Arg Leu Thr
            260                 265                 270

Asp Trp Ile Leu Gln Asp Gly Ser Ala Asp Thr Phe Thr Arg Asn Leu
        275                 280                 285

Thr Leu Met Ser Ile Leu Thr Ile Ala Ser Ala Val Leu Glu Phe Val
    290                 295                 300

Gly Asp Gly Ile Tyr Asn Asn Thr Met Gly His Val His Ser His Leu
305                 310                 315                 320

Gln Gly Glu Val Phe Gly Ala Val Leu Arg Gln Glu Thr Glu Phe Phe
                325                 330                 335

Gln Gln Asn Gln Thr Gly Asn Ile Met Ser Arg Val Thr Glu Asp Thr
            340                 345                 350

Ser Thr Leu Ser Asp Ser Leu Ser Glu Asn Leu Ser Leu Phe Leu Trp
        355                 360                 365

Tyr Leu Val Arg Gly Leu Cys Leu Leu Gly Ile Met Leu Trp Gly Ser
    370                 375                 380

Val Ser Leu Thr Met Val Thr Leu Ile Thr Leu Pro Leu Leu Phe Leu
385                 390                 395                 400

Leu Pro Lys Lys Val Gly Lys Trp Tyr Gln Leu Leu Glu Val Gln Val
                405                 410                 415

Arg Glu Ser Leu Ala Lys Ser Ser Gln Val Ala Ile Glu Ala Leu Ser
            420                 425                 430

Ala Met Pro Thr Val Arg Ser Phe Ala Asn Glu Glu Gly Glu Ala Gln
        435                 440                 445

Lys Phe Arg Glu Lys Leu Gln Glu Ile Lys Thr Leu Asn Gln Lys Glu
    450                 455                 460

Ala Val Ala Tyr Ala Val Asn Ser Trp Thr Thr Ser Ile Ser Gly Met
465                 470                 475                 480

Leu Leu Lys Val Gly Ile Leu Tyr Ile Gly Gly Gln Leu Val Thr Ser
                485                 490                 495

Gly Ala Val Ser Ser Gly Asn Leu Val Thr Phe Val Leu Tyr Gln Met
            500                 505                 510

Gln Phe Thr Gln Ala Val Glu Val Leu Leu Ser Ile Tyr Pro Arg Val
        515                 520                 525

Gln Lys Ala Val Gly Ser Ser Glu Lys Ile Phe Glu Tyr Leu Asp Arg
    530                 535                 540

Thr Pro Arg Cys Pro Pro Ser Gly Leu Leu Thr Pro Leu His Leu Glu
545                 550                 555                 560

Gly Leu Val Gln Phe Gln Asp Val Ser Phe Ala Tyr Pro Asn Arg Pro
                565                 570                 575

Asp Val Leu Val Leu Gln Gly Leu Thr Phe Thr Leu Arg Pro Gly Glu

            580                 585                 590

Val Thr Ala Leu Val Gly Pro Asn Gly Ser Gly Lys Ser Thr Val Ala
        595                 600                 605

Ala Leu Leu Gln Asn Leu Tyr Gln Pro Thr Gly Gly Gln Leu Leu Leu
    610                 615                 620

Asp Gly Lys Pro Leu Pro Gln Tyr Glu His Arg Tyr Leu His Arg Gln
625                 630                 635                 640

Val Ala Ala Val Gly Gln Glu Pro Gln Val Phe Gly Arg Ser Leu Gln
                645                 650                 655

Glu Asn Ile Ala Tyr Gly Leu Thr Gln Lys Pro Thr Met Glu Glu Ile
            660                 665                 670

Thr Ala Ala Ala Val Lys Ser Gly Ala His Ser Phe Ile Ser Gly Leu
        675                 680                 685

Pro Gln Gly Tyr Asp Thr Glu Val Asp Glu Ala Gly Ser Gln Leu Ser
    690                 695                 700

Gly Gly Gln Arg Gln Ala Val Ala Leu Ala Arg Ala Leu Ile Arg Lys
705                 710                 715                 720

Pro Cys Val Leu Ile Leu Asp Asp Ala Thr Ser Ala Leu Asp Ala Asn
                725                 730                 735

Ser Gln Leu Gln Val Glu Gln Leu Leu Tyr Glu Ser Pro Glu Arg Tyr
            740                 745                 750

Ser Arg Ser Val Leu Leu Ile Thr Gln His Leu Ser Leu Val Glu Gln
        755                 760                 765

Ala Asp His Ile Leu Phe Leu Glu Gly Gly Ala Ile Arg Glu Gly Gly
    770                 775                 780

Thr His Gln Gln Leu Met Glu Lys Lys Gly Cys Tyr Trp Ala Met Val
785                 790                 795                 800

Gln Ala Pro Ala Asp Ala Pro Glu
                805

<210>  49
<211>  3719
<212>  DNA
<213>  Homo sapiens

<220>
<221>  CDS
<222>  (97)..(2205)
<223>

<400>  349
ggaccagagc cggtagcgag gttgggagag acggagcgga cctcagcgct gaagcagaag      60

tccccggagc tgcggtctcc ccgccgcggc tgagcc atg cgg ctc cct gac ctg      114
                                        Met Arg Leu Pro Asp Leu
                                        1               5

aga ccc tgg acc tcc ctg ctg ctg gtg gac gcg gct tta ctg tgg ctg      162
Arg Pro Trp Thr Ser Leu Leu Leu Val Asp Ala Ala Leu Leu Trp Leu
            10                  15                  20

ctt cag ggc cct ctg ggg act ttg ctt cct caa ggg ctg cca gga cta      210
Leu Gln Gly Pro Leu Gly Thr Leu Leu Pro Gln Gly Leu Pro Gly Leu
        25                  30                  35

tgg ctg gag ggg acc ctg cgg ctg gga ggg ctg tgg ggg ctg cta aag      258
Trp Leu Glu Gly Thr Leu Arg Leu Gly Gly Leu Trp Gly Leu Leu Lys
    40                  45                  50

cta aga ggg ctg ctg gga ttt gtg ggg aca ctg ctg ctc ccg ctc tgt      306
Leu Arg Gly Leu Leu Gly Phe Val Gly Thr Leu Leu Leu Pro Leu Cys
55                  60                  65                  70

ctg gcc acc ccc ctg act gtc tcc ctg aga gcc ctg gtc gcg ggg gcc      354
Leu Ala Thr Pro Leu Thr Val Ser Leu Arg Ala Leu Val Ala Gly Ala
                75                  80                  85

tca cgt gct ccc cca gcc aga gtc gct tca gcc cct tgg agc tgg ctg      402
Ser Arg Ala Pro Pro Ala Arg Val Ala Ser Ala Pro Trp Ser Trp Leu
            90                  95                  100

ctg gtg ggg tac ggg gct gcg ggg ctc agc tgg tca ctg tgg gct gtt      450
Leu Val Gly Tyr Gly Ala Ala Gly Leu Ser Trp Ser Leu Trp Ala Val

        105                 110                 115

ctg agc cct cct gga gcc cag gag aag gag cag gac cag gtg aac aac 498
Leu Ser Pro Pro Gly Ala Gln Glu Lys Glu Gln Asp Gln Val Asn Asn
    120                 125                 130

aaa gtc ttg atg tgg agg ctg ctg aag ctc tcc agg ccg gac ctg cct 546
Lys Val Leu Met Trp Arg Leu Leu Lys Leu Ser Arg Pro Asp Leu Pro
135                 140                 145                 150

ctc ctc gtt gcc gcc ttc ttc ttc ctt gtc ctt gct gtt ttg ggt gag 594
Leu Leu Val Ala Ala Phe Phe Phe Leu Val Leu Ala Val Leu Gly Glu
                155                 160                 165

aca tta atc cct cac tat tct ggt cgt gtg att gac atc ctg gga ggt 642
Thr Leu Ile Pro His Tyr Ser Gly Arg Val Ile Asp Ile Leu Gly Gly
            170                 175                 180

gat ttt gac ccc cat gcc ttt gcc agt gcc atc ttc ttc atg tgc ctc 690
Asp Phe Asp Pro His Ala Phe Ala Ser Ala Ile Phe Phe Met Cys Leu
        185                 190                 195

ttc tcc ttt ggc agc tca ctg tct gca ggc tgc cga gga ggc tgc ttc 738
Phe Ser Phe Gly Ser Ser Leu Ser Ala Gly Cys Arg Gly Gly Cys Phe
    200                 205                 210

acc tac acc atg tct cga atc aac ttg cgg atc cgg gag cag ctt ttc 786
Thr Tyr Thr Met Ser Arg Ile Asn Leu Arg Ile Arg Glu Gln Leu Phe
215                 220                 225                 230

tcc tcc ctg ctg cgc cag gac ctc ggt ttc ttc cag gag act aag aca 834
Ser Ser Leu Leu Arg Gln Asp Leu Gly Phe Phe Gln Glu Thr Lys Thr
                235                 240                 245

ggg gag ctg aac tca cgg ctg agc tcg gat acc acc ctg atg agt aac 882
Gly Glu Leu Asn Ser Arg Leu Ser Ser Asp Thr Thr Leu Met Ser Asn
            250                 255                 260

tgg ctt cct tta aat gcc aat gtg ctc ttg cga agc ctg gtg aaa gtg 930
Trp Leu Pro Leu Asn Ala Asn Val Leu Leu Arg Ser Leu Val Lys Val
        265                 270                 275

gtg ggg ctg tat ggc ttc atg ctc agc ata tcg cct cga ctc acc ctc 978
Val Gly Leu Tyr Gly Phe Met Leu Ser Ile Ser Pro Arg Leu Thr Leu

280                    285                    290

ctt tct ctg ctg cac atg ccc ttc aca ata gca gcg gag aag gtg tac        1026
Leu Ser Leu Leu His Met Pro Phe Thr Ile Ala Ala Glu Lys Val Tyr
295                    300                    305                    310

aac acc cgc cat cag gaa gtg ctt cgg gag atc cag gat gca gtg gcc        1074
Asn Thr Arg His Gln Glu Val Leu Arg Glu Ile Gln Asp Ala Val Ala
                    315                    320                    325

agg gcg ggg cag gtg gtg cgg gaa gcc gtt gga ggg ctg cag acc gtt        1122
Arg Ala Gly Gln Val Val Arg Glu Ala Val Gly Gly Leu Gln Thr Val
                330                    335                    340

cgc agt ttt ggg gcc gag gag cat gaa gtc tgt cgc tat aaa gag gcc        1170
Arg Ser Phe Gly Ala Glu Glu His Glu Val Cys Arg Tyr Lys Glu Ala
            345                    350                    355

ctt gaa caa tgt cgg cag ctg tat tgg cgg aga gac ctg gaa cgc gcc        1218
Leu Glu Gln Cys Arg Gln Leu Tyr Trp Arg Arg Asp Leu Glu Arg Ala
        360                    365                    370

ttg tac ctg ctc ata agg agg gtg ctg cac ttg ggg gtg cag atg ctg        1266
Leu Tyr Leu Leu Ile Arg Arg Val Leu His Leu Gly Val Gln Met Leu
375                    380                    385                    390

atg ctg agc tgt ggg ctg cag cag atg cag gat ggg gag ctc acc cag        1314
Met Leu Ser Cys Gly Leu Gln Gln Met Gln Asp Gly Glu Leu Thr Gln
                    395                    400                    405

ggc agc ctg ctt tcc ttt atg atc tac cag gag agc gtg ggg agc tat        1362
Gly Ser Leu Leu Ser Phe Met Ile Tyr Gln Glu Ser Val Gly Ser Tyr
                410                    415                    420

gtg cag acc ctg gta tac ata tat ggg gat atg ctc agc aac gtg gga        1410
Val Gln Thr Leu Val Tyr Ile Tyr Gly Asp Met Leu Ser Asn Val Gly
            425                    430                    435

gct gca gag aag gtt ttc tcc tac atg gac cga cag cca aat ctg cct        1458
Ala Ala Glu Lys Val Phe Ser Tyr Met Asp Arg Gln Pro Asn Leu Pro
        440                    445                    450

tca cct ggc acg ctt gcc ccc acc act ctg cag ggg gtt gtg aaa ttc        1506
Ser Pro Gly Thr Leu Ala Pro Thr Thr Leu Gln Gly Val Val Lys Phe

455                 460                 465                 470

caa gac gtc tcc ttt gca tat ccc aat cgc cct gac agg cct gtg ctc      1554
Gln Asp Val Ser Phe Ala Tyr Pro Asn Arg Pro Asp Arg Pro Val Leu
                475                 480                 485

aag ggg ctg acg ttt acc cta cgt cct ggt gag gtg acg gcg ctg gtg      1602
Lys Gly Leu Thr Phe Thr Leu Arg Pro Gly Glu Val Thr Ala Leu Val
            490                 495                 500

gga ccc aat ggg tct ggg aag agc aca gtg gct gcc ctg ctg cag aat      1650
Gly Pro Asn Gly Ser Gly Lys Ser Thr Val Ala Ala Leu Leu Gln Asn
        505                 510                 515

ctg tac cag ccc aca ggg gga cag gtg ctg ctg gat gaa aag ccc atc      1698
Leu Tyr Gln Pro Thr Gly Gly Gln Val Leu Leu Asp Glu Lys Pro Ile
    520                 525                 530

tca cag tat gaa cac tgc tac ctg cac agc cag gtg gtt tca gtt ggg      1746
Ser Gln Tyr Glu His Cys Tyr Leu His Ser Gln Val Val Ser Val Gly
535                 540                 545                 550

cag gag cct gtg ctg ttc tcc ggt tct gtg agg aac aac att gct tat      1794
Gln Glu Pro Val Leu Phe Ser Gly Ser Val Arg Asn Asn Ile Ala Tyr
                555                 560                 565

ggg ctg cag agc tgc gaa gat gat aag gtg atg gcg gct gcc cag gct      1842
Gly Leu Gln Ser Cys Glu Asp Asp Lys Val Met Ala Ala Ala Gln Ala
            570                 575                 580

gcc cac gca gat gac ttc atc cag gaa atg gag cat gga ata tac aca      1890
Ala His Ala Asp Asp Phe Ile Gln Glu Met Glu His Gly Ile Tyr Thr
        585                 590                 595

gat gta ggg gag aag gga agc cag ctg gct gcg gga cag aaa caa cgt      1938
Asp Val Gly Glu Lys Gly Ser Gln Leu Ala Ala Gly Gln Lys Gln Arg
    600                 605                 610

ctg gcc att gcc cgg gcc ctt gta cga gac ccg cgg gtc ctc atc ctg      1986
Leu Ala Ile Ala Arg Ala Leu Val Arg Asp Pro Arg Val Leu Ile Leu
615                 620                 625                 630

gat gag gct act agt gcc cta gat gtg cag tgc gag cag gcc ctg cag      2034
Asp Glu Ala Thr Ser Ala Leu Asp Val Gln Cys Glu Gln Ala Leu Gln

                635                 640                 645

gac tgg aat tcc cgt ggg gat cgc aca gtg ctg gtg att gct cac agg 2082
Asp Trp Asn Ser Arg Gly Asp Arg Thr Val Leu Val Ile Ala His Arg
            650                 655                 660

ctg cag gca gtt cag cgc gcc cac cag atc ctg gtg ctc cag gag ggc 2130
Leu Gln Ala Val Gln Arg Ala His Gln Ile Leu Val Leu Gln Glu Gly
        665                 670                 675

aag ctg cag aag ctt gcc cag ctc cag gag gga cag gac ctc tat tcc 2178
Lys Leu Gln Lys Leu Ala Gln Leu Gln Glu Gly Gln Asp Leu Tyr Ser
    680                 685                 690

cgc ctg gtt cag cag cgg ctg atg gac tgaggcccca gggatactgg 2225
Arg Leu Val Gln Gln Arg Leu Met Asp
695                 700

gccctcttct caggggcgtc tccaggaccc agagctgttc ctgctttgag tttccctaga 2285

gctgtgcggc cagatagctg ttcctgagtt gcaagcacga tggagatttg gacactgtgt 2345

gcttttggtg gggtggagag gtggggtggg gtggggtgtg gtggggtggg aggctgtctg 2405

tgtccaggaa acttaattcc ctggtgacta gagctttgcc tggtgatgag gagtattttg 2465

tggcataata catatatttt aaaatatttt ccttcttacg tgaactgtat acattcatat 2525

agaaaattta gacaatataa aaaagtacaa agaagaaaag taaaagtacc cattgtttca 2585

cttcctggag ataaccatag ttgctatttt gctgcctgtc ccatcagtcg tttatctgtt 2645

gtttgagata gaaattaacc aaaaatgaca taaatattca tgagattgcc ttcctatatc 2705

cttccttgtt cctaccagtg tctgctattt tgaagaagct agggtctgga gggacagaga 2765

acagttccct gattaacagt attaatagcg acattggtaa cagctaccat ttatagagtt 2825

ttaatgggag taggagctat gctaagtgtt tttcatgtat tatcgttttt aatcattatc 2885

cccaacccta tgaggttggt tattatcccc attttacaga tgaggaaact gaagctcaaa 2945

gaggctcaat gactttccca aggtggtcgt agtggtggag ttggagtttg aacacaggcc 3005

tgaccctaga gtccacaccc tgacccaatc aattatattg catcttgggt ccataaaccc 3065

taatccataa tcccatcaag aaaagctctg ctgctcttag ctctaaataa ttcagaatct 3125

attctcttct ctccagtccc gttgttatag tcttcactca tagacttaag atgatcccat 3185

caccagagag gtttctctac cattagcttc cctcttccgg ccattcttca caaagtcatt 3245

tttctaaatt ctgtgtcaca tacgatgatg gcatttctgg aaattccttc aggtgctctc 3305

aagccctgct gcagagatcc ttttcagagc acacactgtt ccagcccatc tgtctcaccc 3365

tctcctgttg tatccagctc cacgacaaac tttctgcctt ccccaacacc tttgtgcctt 3425

tgcatatggt gttttcttgc ccattttctg ctcgactcgc ccctgatttt caagttcaag 3485

acttaactca gggttcaggt cttccaggag gccttactta tgtcgtcagt ctggggaact 3545

ctccatgtgc ttctatcact gtgcggttac ctctttcaca gcccttttaa agttctatct 3605

tccctttccc accttttttg accttccact agaccatgag cacctgggcg gaaagccata 3665

tatcttatta agctttatat ctgctacctg gccgagggct aattcatagt ggag 3719

<210> 50
<211> 703
<212> PRT
<213> Homo sapiens

<400> 50

Met Arg Leu Pro Asp Leu Arg Pro Trp Thr Ser Leu Leu Leu Val Asp
1               5                   10                  15

Ala Ala Leu Leu Trp Leu Leu Gln Gly Pro Leu Gly Thr Leu Leu Pro
            20                  25                  30

Gln Gly Leu Pro Gly Leu Trp Leu Glu Gly Thr Leu Arg Leu Gly Gly
        35                  40                  45

Leu Trp Gly Leu Leu Lys Leu Arg Gly Leu Leu Gly Phe Val Gly Thr
    50                  55                  60

Leu Leu Leu Pro Leu Cys Leu Ala Thr Pro Leu Thr Val Ser Leu Arg
65                  70                  75                  80

Ala Leu Val Ala Gly Ala Ser Arg Ala Pro Pro Ala Arg Val Ala Ser
                85                  90                  95

Ala Pro Trp Ser Trp Leu Leu Val Gly Tyr Gly Ala Ala Gly Leu Ser
            100                 105                 110

Trp Ser Leu Trp Ala Val Leu Ser Pro Pro Gly Ala Gln Glu Lys Glu
        115                 120                 125

Gln Asp Gln Val Asn Asn Lys Val Leu Met Trp Arg Leu Leu Lys Leu
    130                 135                 140

Ser Arg Pro Asp Leu Pro Leu Leu Val Ala Ala Phe Phe Phe Leu Val
145                 150                 155                 160

Leu Ala Val Leu Gly Glu Thr Leu Ile Pro His Tyr Ser Gly Arg Val
                165                 170                 175

Ile Asp Ile Leu Gly Gly Asp Phe Asp Pro His Ala Phe Ala Ser Ala
            180                 185                 190

Ile Phe Phe Met Cys Leu Phe Ser Phe Gly Ser Ser Leu Ser Ala Gly
        195                 200                 205

Cys Arg Gly Gly Cys Phe Thr Tyr Thr Met Ser Arg Ile Asn Leu Arg
    210                 215                 220

Ile Arg Glu Gln Leu Phe Ser Ser Leu Leu Arg Gln Asp Leu Gly Phe
225                 230                 235                 240

Phe Gln Glu Thr Lys Thr Gly Glu Leu Asn Ser Arg Leu Ser Ser Asp
                245                 250                 255

Thr Thr Leu Met Ser Asn Trp Leu Pro Leu Asn Ala Asn Val Leu Leu
            260                 265                 270

Arg Ser Leu Val Lys Val Val Gly Leu Tyr Gly Phe Met Leu Ser Ile
        275                 280                 285

Ser Pro Arg Leu Thr Leu Leu Ser Leu Leu His Met Pro Phe Thr Ile
    290                 295                 300

Ala Ala Glu Lys Val Tyr Asn Thr Arg His Gln Glu Val Leu Arg Glu
305                 310                 315                 320

Ile Gln Asp Ala Val Ala Arg Ala Gly Gln Val Val Arg Glu Ala Val
                325                 330                 335

Gly Gly Leu Gln Thr Val Arg Ser Phe Gly Ala Glu Glu His Glu Val
            340                 345                 350

Cys Arg Tyr Lys Glu Ala Leu Glu Gln Cys Arg Gln Leu Tyr Trp Arg
        355                 360                 365

Arg Asp Leu Glu Arg Ala Leu Tyr Leu Leu Ile Arg Arg Val Leu His
    370                 375                 380

Leu Gly Val Gln Met Leu Met Leu Ser Cys Gly Leu Gln Gln Met Gln
385                 390                 395                 400

Asp Gly Glu Leu Thr Gln Gly Ser Leu Leu Ser Phe Met Ile Tyr Gln
                405                 410                 415

Glu Ser Val Gly Ser Tyr Val Gln Thr Leu Val Tyr Ile Tyr Gly Asp
            420                 425                 430

Met Leu Ser Asn Val Gly Ala Ala Glu Lys Val Phe Ser Tyr Met Asp
        435                 440                 445

Arg Gln Pro Asn Leu Pro Ser Pro Gly Thr Leu Ala Pro Thr Thr Leu
    450                 455                 460

Gln Gly Val Val Lys Phe Gln Asp Val Ser Phe Ala Tyr Pro Asn Arg
465                 470                 475                 480

Pro Asp Arg Pro Val Leu Lys Gly Leu Thr Phe Thr Leu Arg Pro Gly
                485                 490                 495

Glu Val Thr Ala Leu Val Gly Pro Asn Gly Ser Gly Lys Ser Thr Val
            500                 505                 510

Ala Ala Leu Leu Gln Asn Leu Tyr Gln Pro Thr Gly Gly Gln Val Leu
        515                 520                 525

Leu Asp Glu Lys Pro Ile Ser Gln Tyr Glu His Cys Tyr Leu His Ser

530                 535                 540

Gln Val Val Ser Val Gly Gln Glu Pro Val Leu Phe Ser Gly Ser Val
545                 550                 555                 560

Arg Asn Asn Ile Ala Tyr Gly Leu Gln Ser Cys Glu Asp Asp Lys Val
                565                 570                 575

Met Ala Ala Ala Gln Ala Ala His Ala Asp Asp Phe Ile Gln Glu Met
            580                 585                 590

Glu His Gly Ile Tyr Thr Asp Val Gly Glu Lys Gly Ser Gln Leu Ala
        595                 600                 605

Ala Gly Gln Lys Gln Arg Leu Ala Ile Ala Arg Ala Leu Val Arg Asp
    610                 615                 620

Pro Arg Val Leu Ile Leu Asp Glu Ala Thr Ser Ala Leu Asp Val Gln
625                 630                 635                 640

Cys Glu Gln Ala Leu Gln Asp Trp Asn Ser Arg Gly Asp Arg Thr Val
                645                 650                 655

Leu Val Ile Ala His Arg Leu Gln Ala Val Gln Arg Ala His Gln Ile
            660                 665                 670

Leu Val Leu Gln Glu Gly Lys Leu Gln Lys Leu Ala Gln Leu Gln Glu
        675                 680                 685

Gly Gln Asp Leu Tyr Ser Arg Leu Val Gln Gln Arg Leu Met Asp
    690                 695                 700

<210>  51
<211>  264
<212>  DNA
<213>  human herpesvirus 1

<220>
<221>  CDS
<222>  (1)..(264)
<223>

<400>  51

atg tcg tgg gcc ctg gaa atg gcg gac acc ttc ctg gac acc atg cgg 48
Met Ser Trp Ala Leu Glu Met Ala Asp Thr Phe Leu Asp Thr Met Arg
1               5                   10                  15

gtt ggg ccc agg acg tac gcc gac gta cgc gat gag atc aat aaa agg 96
Val Gly Pro Arg Thr Tyr Ala Asp Val Arg Asp Glu Ile Asn Lys Arg
            20                  25                  30

ggg cgt gag gac cgg gag gcg gcc aga acc gcc gtg cac gac ccg gag 144
Gly Arg Glu Asp Arg Glu Ala Ala Arg Thr Ala Val His Asp Pro Glu
        35                  40                  45

cgt ccc ctg ctg cgc tct ccc ggg ctg ctg ccc gaa atc gcc ccc aac 192
Arg Pro Leu Leu Arg Ser Pro Gly Leu Leu Pro Glu Ile Ala Pro Asn
    50                  55                  60

gca tcc ttg ggt gtg gca cat cga aga acc ggc ggg acc gtg acc gac 240
Ala Ser Leu Gly Val Ala His Arg Arg Thr Gly Gly Thr Val Thr Asp
65                  70                  75                  80

agt ccc cgt aat ccg gta acc cgt 264
Ser Pro Arg Asn Pro Val Thr Arg
                85

<210> 52
<211> 88
<212> PRT
<213> human herpesvirus 1

<400> 52

Met Ser Trp Ala Leu Glu Met Ala Asp Thr Phe Leu Asp Thr Met Arg
1               5                   10                  15

Val Gly Pro Arg Thr Tyr Ala Asp Val Arg Asp Glu Ile Asn Lys Arg
            20                  25                  30

Gly Arg Glu Asp Arg Glu Ala Ala Arg Thr Ala Val His Asp Pro Glu
        35                  40                  45

Arg Pro Leu Leu Arg Ser Pro Gly Leu Leu Pro Glu Ile Ala Pro Asn
    50                  55                  60

Ala Ser Leu Gly Val Ala His Arg Arg Thr Gly Gly Thr Val Thr Asp
65                  70                  75                  80

Ser Pro Arg Asn Pro Val Thr Arg
                85

<210>  53
<211>  549
<212>  DNA
<213>  Human cytomegalovirus

<220>
<221>  CDS
<222>  (1)..(549)
<223>

<400>  53
atg gat ctc ttg att cgt ctc ggt ttt ctg ttg atg tgt gcg ttg ccg      48
Met Asp Leu Leu Ile Arg Leu Gly Phe Leu Leu Met Cys Ala Leu Pro
1               5                   10                  15

acc ccc ggt gag cgg tct tcg cgt gac ccg aaa acc ctt ctc tct ctg      96
Thr Pro Gly Glu Arg Ser Ser Arg Asp Pro Lys Thr Leu Leu Ser Leu
            20                  25                  30

tct ccg cga caa caa gct tgt gtt ccg aga acg aag tcg cac aga ccc      144
Ser Pro Arg Gln Gln Ala Cys Val Pro Arg Thr Lys Ser His Arg Pro
        35                  40                  45

gtt tgt tac aac gat aca ggg gac tgc aca gat gca gat gat agc tgg      192
Val Cys Tyr Asn Asp Thr Gly Asp Cys Thr Asp Ala Asp Asp Ser Trp
    50                  55                  60

aaa cag ctg ggt gag gac ttt gcg cac caa tgc ttg cag gcg gcg aaa      240
Lys Gln Leu Gly Glu Asp Phe Ala His Gln Cys Leu Gln Ala Ala Lys
65                  70                  75                  80

aag agg cct aaa acg cac aaa tcc cgt ccg aac gat agg aac ctt gag      288
Lys Arg Pro Lys Thr His Lys Ser Arg Pro Asn Asp Arg Asn Leu Glu
                85                  90                  95

ggt agg ctg acc tgt caa cga gtc cgt cgg cta ctg ccc tgt gat ttg      336
Gly Arg Leu Thr Cys Gln Arg Val Arg Arg Leu Leu Pro Cys Asp Leu

            100                 105                 110

gat att cat cct agc cac cgg ttg tta acg ctt atg aat aac tgc gtc 384
Asp Ile His Pro Ser His Arg Leu Leu Thr Leu Met Asn Asn Cys Val
        115                 120                 125

tgt gac ggg gcc gtt tgg aac gcg ttt cgc ttg ata gaa cga cac gga 432
Cys Asp Gly Ala Val Trp Asn Ala Phe Arg Leu Ile Glu Arg His Gly
    130                 135                 140

ttc ttc gct gtg act ttg tat tta tgt tgc ggg att act ctg ctg gtt 480
Phe Phe Ala Val Thr Leu Tyr Leu Cys Cys Gly Ile Thr Leu Leu Val
145                 150                 155                 160

gtt att cta gca ttg ctg tgc agc ata aca tac gaa tcg act gga cgt 528
Val Ile Leu Ala Leu Leu Cys Ser Ile Thr Tyr Glu Ser Thr Gly Arg
                165                 170                 175

ggg att cga cgt tgt ggc tcc 549
Gly Ile Arg Arg Cys Gly Ser
            180

<210> 54
<211> 183
<212> PRT
<213> Human cytomegalovirus

<400> 54

Met Asp Leu Leu Ile Arg Leu Gly Phe Leu Leu Met Cys Ala Leu Pro
1               5                   10                  15

Thr Pro Gly Glu Arg Ser Ser Arg Asp Pro Lys Thr Leu Leu Ser Leu
            20                  25                  30

Ser Pro Arg Gln Gln Ala Cys Val Pro Arg Thr Lys Ser His Arg Pro
        35                  40                  45

Val Cys Tyr Asn Asp Thr Gly Asp Cys Thr Asp Ala Asp Asp Ser Trp
    50                  55                  60

Lys Gln Leu Gly Glu Asp Phe Ala His Gln Cys Leu Gln Ala Ala Lys
65                  70                  75                  80

Lys Arg Pro Lys Thr His Lys Ser Arg Pro Asn Asp Arg Asn Leu Glu
                85                  90                  95

Gly Arg Leu Thr Cys Gln Arg Val Arg Arg Leu Leu Pro Cys Asp Leu
            100                 105                 110

Asp Ile His Pro Ser His Arg Leu Leu Thr Leu Met Asn Asn Cys Val
        115                 120                 125

Cys Asp Gly Ala Val Trp Asn Ala Phe Arg Leu Ile Glu Arg His Gly
    130                 135                 140

Phe Phe Ala Val Thr Leu Tyr Leu Cys Cys Gly Ile Thr Leu Leu Val
145                 150                 155                 160

Val Ile Leu Ala Leu Leu Cys Ser Ile Thr Tyr Glu Ser Thr Gly Arg
                165                 170                 175

Gly Ile Arg Arg Cys Gly Ser
            180